# EUROPEAN PATENT APPLICATION

(11) **EP 4 349 358 A1**
(43) Date of publication of application: **10.04.2024**
(21) Application number: 22816502.3
(22) Date of filing: 03.06.2022
(51) Int. Cl.: A61K 38/00, A61K 38/17, A61K 47/68, A61P 19/08, A61P 3/04, A61P 35/00, C12Q 1/6883, C12Q 1/6886, G01N 33/68, G01N 33/50

(54) **TM4SF19 INHIBITOR AND USES THEREOF**

(30) Priority: 03.06.2021 KR 20210072352; 07.12.2021 KR 20210174057
(71) Applicant: Medpacto, Inc., Seoul 06668 (KR); Kim, Seong Jin, Seoul 06667 (KR)
(72) Inventor: PARK, Su Jin, Namyangju-si Gyeonggi-do 12147 (KR); HEO, Jin Sun, Gwangju-si Gyeonggi-do 12778 (KR); HONG, Eun Ji, Seoul 06668 (KR); AN, Hae In, Seongnam-si Gyeonggi-do 13645 (KR); KIM, Min Woo, Seoul 02832 (KR); KIM, Seong Jin, Seoul 06667 (KR)
(74) Representative: Maiwald GmbH
(86) International application number: PCT/KR2022/007939
(87) International publication number: WO 2022/255843

(57) **Abstract**

The present invention relates to a composition for preventing or treating a bone disease, obesity or an obesity-mediated metabolic disease, cancer, or cancer metastasis, and a method of screening a drug for treating the diseases, which includes an inhibitor of transmembrane 4 L six family member 19 (TM4SF19) expression or activity.

## Description

### [Technical Field]

The present invention relates to a composition for preventing or treating a bone disease, obesity, an obesity-mediated metabolic disease, cancer, or cancer metastasis, which includes an inhibitor of the expression or activity of transmembrane 4 L six family member 19 (TM4SF19), and a method of screening a drug for treating the above-mentioned diseases.

### [Background Art]

Bone is active tissue that constantly changes throughout one's lifetime. Bone is visually divided into external cortical bone (compact bone) and internal trabecular bone (cancellous bone or spongy bone), wherein the cortical bone serves to protect and support the body due to a high physical strength, and the trabecular bone serves to absorb an impact or constantly maintain a calcium change.

Even after bone growth has stopped, a phenomenon in which old bone is destroyed and disappears (bone resorption) and new bone fills the place from which the old bone disappears (bone formation) is repeated throughout one's lifetime, which is called bone remodeling.

When the interaction between bone formation by osteoblasts and bone resorption by osteoclasts is balanced, bone homeostasis is maintained and the calcium concentration in the blood is constantly maintained. Bone-related diseases such as osteoporosis are caused by the imbalance in such bone metabolism.

The phenomenon of maintaining bone homeostasis by sequentially occurring bone resorption and bone formation through the interaction between osteoclasts and osteoblasts is called bone remodeling, and bone mass is determined by the balance in activity between osteoclasts and osteoblasts. The process of the bone remodeling is balanced by coupling the bone resorption caused by osteoclasts and subsequent bone formation caused by osteoblasts. When such efficient balance is disturbed, bone loss occurs.

Osteoclasts become multinucleated osteoclasts through differentiation, and bone resorption occurs by multinucleated osteoclasts. Dysfunction of osteoclasts causes osteopetrosis, and overactivation of osteoclasts after menopause increases bone resorption leading to osteoporosis and inflammatory arthritis after menopause. To date, bisphosphonates and anti-RANKL antibodies are drugs developed to target the overactivation of osteoclasts, but the overall inhibition of osteoclasts affects the balance between osteoclasts and osteoblasts, which greatly affects bone formation but has side effects. Therefore, there is a demand for the development of therapeutic agents that can more selectively inhibit osteoclasts.

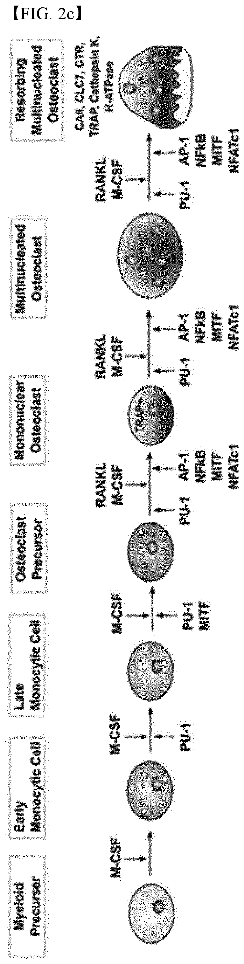

Osteoclasts, originating from hematopoietic stem cells, are multinucleated cells differentiating from monocyte/macrophage lineages through the activation caused by the stimulation of a monocyte/macrophage colony-stimulating factor (M-CSF) and the activation by a receptor activator of nuclear factor κB (RANK) ligand (RANKL). The differentiation of osteoclasts is performed by differentiation of an osteoclast precursor into TRAP-positive mononuclear osteoclasts, and maturation to multinucleated osteoclasts by cell-cell fusion [see FIG. 2C]. These mature and multinucleated osteoclasts cause bone resorption [Int J Mol Sci. 2020 Aug 8;21(16):5685.].

Obesity is a biological phenomenon caused by the interaction of a complex of genetic, metabolic, environmental, and behavioral factors, defined as abnormal or excessive accumulation of fat, and can have a bad impact on health. Particularly, obesity is known as an important risk factor for causing various adult diseases such as high blood pressure, type 2 diabetes, cancer, liver disease, hyperlipidemia, arteriosclerosis, etc. Obesity results from a chronic imbalance between caloric intake and energy expenditure, is accompanied by loss of metabolism, endocrine and immune function of adipose tissue, and causes metabolic abnormalities and decreased responsiveness (resistance) to insulin, a major fat storage signaling hormone, which causes fat accumulation in metabolic organs other than adipose tissue, resulting in several types of lipotoxicity. The most representative pathology of lipotoxicity is an inflammatory response, which may be characterized as chronic low-degrade inflammation. In obesity, before metabolic abnormalities and insulin resistance appear, the activation of macrophages occurs in adipose tissue and leads to a pre-inflammatory step. A such, obesity induces a low-intensity chronic inflammatory state in the body, causing various metabolic diseases. As described above, adipose tissue macrophages play an important role in the occurrence of chronic inflammation and metabolic complications in the body due to obesity.

Fat stored in adipocytes is used as an important energy source in the body. Obesity is caused by excessive differentiation of adipocytes and unbalanced oversupply of energy. In adipose tissue, there are various cells such as adipose-derived stem cells (ASCs), immune cells and endothelial cells, as well as adipocytes, which is called a stromal vascular fraction (SVF). Adipose tissue stores energy in the form of a lipid, but also acts to keep the body warm. It is divided into white adipose tissue (WAT), which is fat storing nutrients, and brown adipose tissue (BAT), which consumes nutrients and generates heat. In addition, adipose tissue also produces leptin, resistin, adiponectin, and tumor necrosis factor-α (TNFα). Adipocyte differentiation is very complicated due to the interaction of various hormones and various transcription factors, and is stimulated by stimuli such as insulin, insulin-like growth factor-1 or growth hormones. In this process, an increase in transcription factors such as the CCAAT enhancer-binding protein (C/EBP)) family and peroxisome proliferator-activated receptor (PPAR) gamma is observed. These transcription factors promote the differentiation of adipocytes together with an adipocyte regulator, and the expression levels of enzymes such as fatty acid-binding protein aP2 and fatty acid synthase are increased. On the other hand, it has been reported that excessive accumulation of triglycerides is involved in the progression of fatty liver [J. Clin. Invest., 98, 1575 1584 (1996).].

Currently, a fat absorption inhibitor such as Xenical (Roche, Switzerland) and an anorectic agent such as Meridia (Abbott, U.S.A.) are widely used as anti-obesity drugs, but these drugs have problems of side effects such as headaches, increased blood pressure, and diarrhea. Therefore, there is a need to develop an anti-obesity drug for a new target without side effects.

Cancer is one of the biggest diseases that threatens human health, caused by cells undergoing a series of mutations, proliferation in an unlimited, uncontrolled manner, and becoming immortal. In the case of cancer detected at an early stage, there are treatments such as surgery, radiation therapy, and chemotherapy, but the side effects are emerging as a big problem, and in the case of terminal or metastatic cancer, life ends with a limited life without special treatment.

Recently, although various biochemical mechanisms related to cancer have been identified and related therapeutic agents have been developed, fundamental methods for treating cancer have not been suggested yet. Accordingly, research to identify various cancer-related molecules in vivo and to develop drugs targeting them is being actively conducted, and efforts to enhance the cancer treatment effect by combining some of these drugs are also being attempted.

Therefore, efforts to additionally discover cancer-related target molecules are very important.

Meanwhile, the use of transmembrane 4 L six family member (TM4SF19) as a marker for obesity diagnosis has been disclosed in Korean Patent No. 10-1781200. However, so far, there has been no report on the effect of TM4SF19 in the treatment of bone-related diseases, the treatment of obesity or obesity-related metabolic diseases, and the treatment of cancer or inhibition of cancer metastasis.

### [Disclosure]

### [Technical Problem]

Therefore, to find related genes for the fundamental treatment of bone diseases, obesity, various metabolic diseases mediated by obesity, cancer treatment or the inhibition of cancer metastasis, the present inventors conducted research on the relevance of various genes with bone diseases, obesity or an obesity-mediated metabolic diseases, and cancer, and as a result, confirmed various functions of TM4SF19. Thus, the present invention was completed.

### [Technical Solution]

The present invention is directed to providing a pharmaceutical composition for preventing or treating a bone disease, which includes an inhibitor of TM4SF19 expression or activity as an active ingredient.

The present invention is also directed to providing a method of screening a drug for treating a bone disease, which includes: treating a specimen suspected of a bone disease with a candidate for treating a bone disease; and comparing the mRNA or protein expression level of a TM4SF19 gene with a control.

The present invention is also directed to providing a pharmaceutical composition for preventing or treating obesity or an obesity-mediated metabolic disease, which includes an inhibitor of TM4SF19 expression or activity as an active ingredient.

The present invention is also directed to providing a method of screening a drug for treating obesity or an obesity-mediated metabolic disease, which includes: treating a specimen suspected of obesity or an obesity-mediated metabolic disease with a candidate material for treating obesity or an obesity-mediated metabolic disease, and comparing the mRNA or protein expression level of a TM4SF19 gene with a control.

The present invention is also directed to providing a pharmaceutical composition for preventing or treating cancer, or inhibiting cancer metastasis, which includes: an inhibitor of TM4SF19 expression or activity as an active ingredient.

The present invention is also directed to providing a method of screening a drug for treating cancer or cancer metastasis, which includes: treating a specimen suspected of cancer or cancer metastasis with a candidate for preventing or treating cancer, or inhibiting cancer metastasis, and comparing the mRNA or protein expression level of a TM4SF19 gene with a control.

The present invention is also directed to providing a fusion protein for inhibiting TM4SF19 expression or activity.

The present invention is also directed to providing a method of preventing or treating a bone disease, which includes: administering a therapeutically effective amount of a composition for preventing or treating a bone disease including an inhibitor of TM4SF19 expression or activity into a subject.

The present invention is also directed to providing a method of preventing or treating obesity or an obesity-mediated metabolic disease, which includes administering a therapeutically effective amount of a composition for preventing or treating obesity or an obesity-mediated metabolic disease including an inhibitor of TM4SF19 expression or activity into a subject.

The present invention is also directed to providing a method of preventing or treating cancer or cancer metastasis, which include administering a therapeutically effective amount of a composition for preventing or treating cancer or inhibiting cancer metastasis including an inhibitor of TM4SF19 expression or activity into a subj ect.

### [Advantageous Effects]

An inhibitor of TM4SF19 expression or activity according to the present invention can be used in prevention or treatment of a bone disease, obesity, an obesity-mediated metabolic disease, cancer, or cancer metastasis.

In addition, by a method of screening a candidate for treating a bone disease, obesity, an obesity-mediated metabolic disease, cancer, or cancer metastasis, which inhibits TM4SF19, a candidate capable of treating a bone disease, obesity, an obesity-mediated metabolic disease, cancer, or cancer metastasis can be effectively selected.

### [Description of Drawings]

FIG. 1 shows the expression of a TM4SF19 gene over time after cells isolated from the mouse bone marrow are treated with M-CSF and receptor activator of nuclear factor kappa-B ligand (RANKL) to differentiate, confirmed by qPCR.

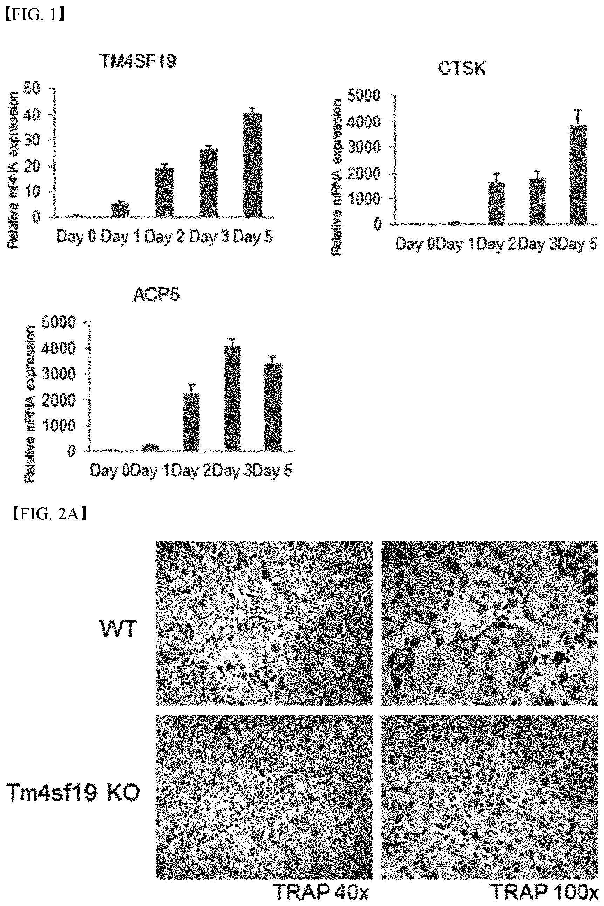

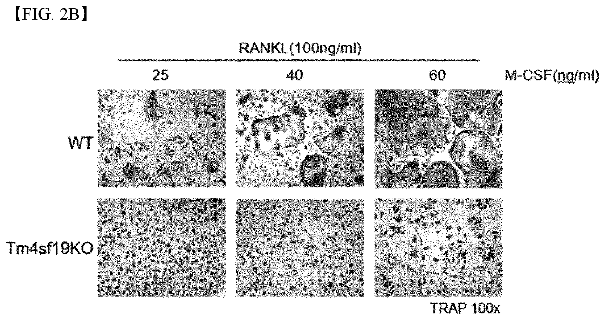

FIG. 2A is a TRAP staining result for bone marrow-derived cells of a wild-type mouse and a CRISPR-knock-out mouse, TM4SF19KO mouse, which have been treated with M-CSF (25 ng/ml) + RANKL (100 ng/ml) to differentiate. FIG. 2B shows a result of staining bone marrow-derived macrophages(BMMs) of TM4SF19 knock-out mice (TM4SF19KO) generated using CRISPR and wild-type (WT) mice with tartrate-resistant acid phosphatase (TRAP) staining, which is an osteoclast-related marker, after differentiation under conditions in which a RANKL concentration is fixed at 100 ng/ml and an M-CSF concentration is changed to 25, 40, or 60 ng/ml. FIG. 2C is a schematic diagram illustrating the process of osteoclast differentiation.
FIG. 3 shows the expression of osteoclast (OC) differentiation-related target genes, Ctsk, Acp5, c-Fos, and Nfatc1, confirmed by qPCR, at 4 days after treatment with M-CSF or M-CSF + RANKL (M-CSF: 25 ng/ml, RANKL: 100 ng/ml) to bone marrow cells of wild type (WT) and TM4SF19KO mice.
FIG. 4A shows whether an actin belt is formed in the osteoclast multinucleation, confirmed by F-actin staining, after differentiating the BMMs of wild-type and TM4SF19KO mice.
FIG. 4B shows the pit formation, confirmed by 1% toluidine blue staining, after plating BMMs on a Dentin disc and differentiating them.
FIG. 5A shows micro-CT analysis results for 8-week-old wild-type (WT) and TM4SF19KO female mice, which have been dissected without ovary removal (sham) or ovariectomized (OVX) and then have femurs fixed on day 31.
FIG. 5B shows 3D micro-CT parameters (bone microstructure parameters) including a bone volume (BV), a ratio of bone volume to tissue volume (% BV/TV), an average trabecular number (Tb.N), a trabecular separation (Tb.Sp), and a trabecular thickness (Tb.Th), obtained by analyzing micro-CT images using a 3D imaging program.
FIG. 6 is a TRAP staining result for the femurs of 8-week-old wild-type (WT) and TM4SF19KO mice, which have been fixed and decalcified.

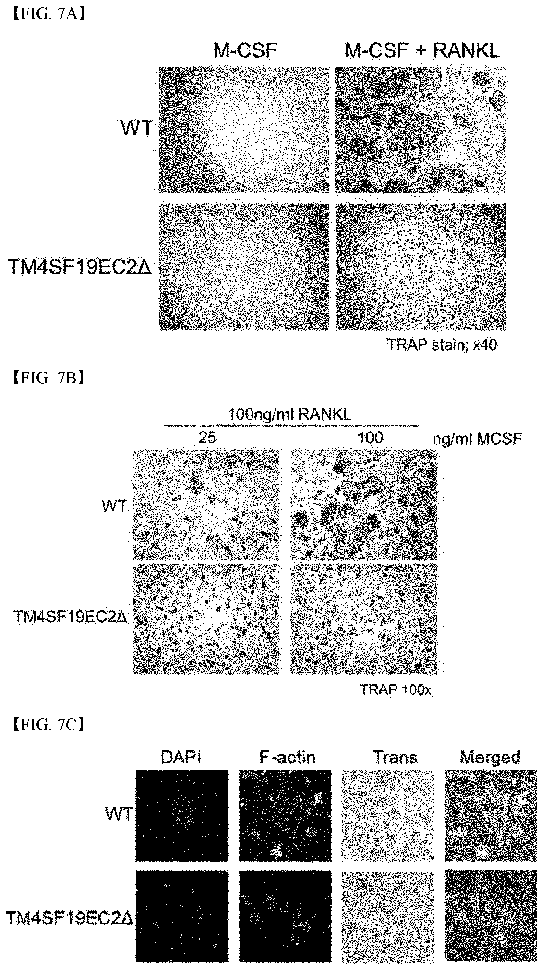

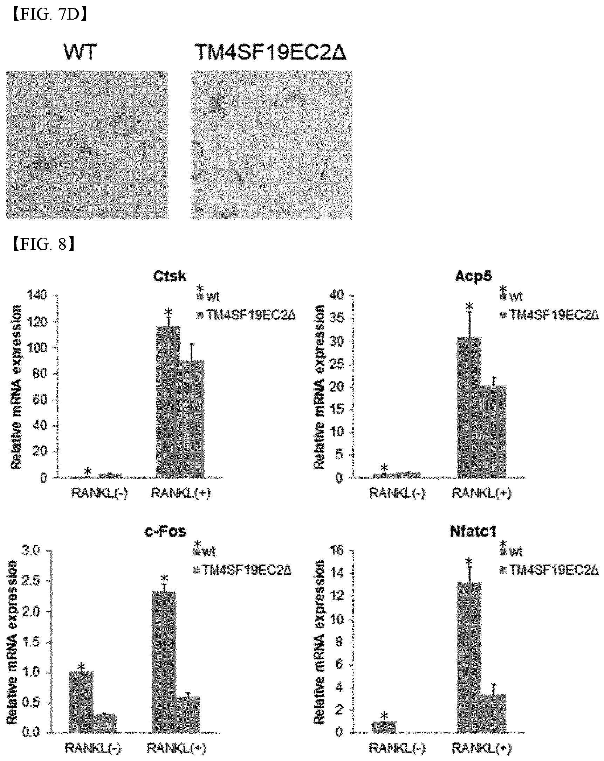

FIG. 7A shows a TRAP staining result which is obtained after cells isolated from the bone marrow of wild-type (WT) mice and TM4SF19EC2Δ mice in which TM4SF19 extracellular domain 2 (116-165) is knocked out by CRISPR, are treated with M-CSF (25 ng/ml) or M-CSF + RANKL (100 ng/ml), and differentiated. FIG. 7B shows a TRAP staining result after cells isolated from the bone marrow of wild-type (WT) mice and TM4SF19EC2Δ mice are treated by fixing the RANKL concentration to 100 ng/ml and the M-CSF concentration is changed to 25 ng/ml or 100 ng/ml and differentiated. FIG. 7C shows the formation of an actin belt during the differentiation of multinucleated osteoclasts, confirmed by F-actin staining, after differentiating the bone marrow of wild-type (WT) and TM4SF19EC2Δ mice. FIG. 7D shows pit formation, confirmed by 1% toluidine blue staining, after plating wild-type (WT) and TM4SF19EC2Δ BMMs on a Dentin disc and differentiating them.
FIG. 8 shows the expression of target genes (Ctsk, Acp5, c-Fos, and Nfatc1) associated with osteoclast differentiation in wild-type (WT) mice and TM4SF19EC2Δ mice, analyzed by qPCR.
FIG. 9A shows a micro-CT analysis result for 8-week-old wild-type (WT) and TM4SF19EC2Δ female mice, which have been dissected without ovary removal (sham) or ovariectomized (OVX), and then 31 days later, their femurs were fixed.
FIG. 9B shows 3D micro-CT parameters (bone microstructure parameters) including a bone volume (BV), a ratio of bone volume to tissue volume (% BV/TV), an average trabecular number (Tb.N), a trabecular separation (Tb.Sp), and a trabecular thickness (Tb.Th), obtained by analyzing micro-CT images using a 3D imaging program.
FIG. 10 is a micro-CT analysis result showing bone microstructure parameters for 8-week-old wild-type (WT), TM4SF19KO, and TM4SF19EC2Δ female mice, which have been dissected without ovary removal (sham) or ovariectomized (OVX), and then 31 days later, their femurs were fixed.
FIG. 11 is a set of micro-CT images for 8-week-old wild-type (WT), TM4SF19KO, and TM4SF19EC2Δ female mice, which have been dissected without ovary removal (sham) or ovariectomized (OVX), and then 31 days later, their femurs were fixed.

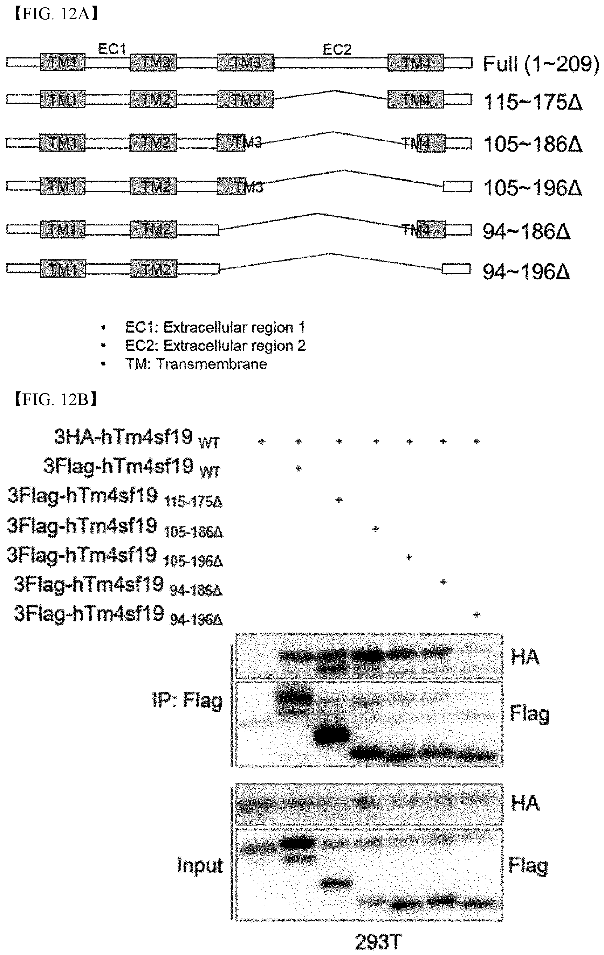

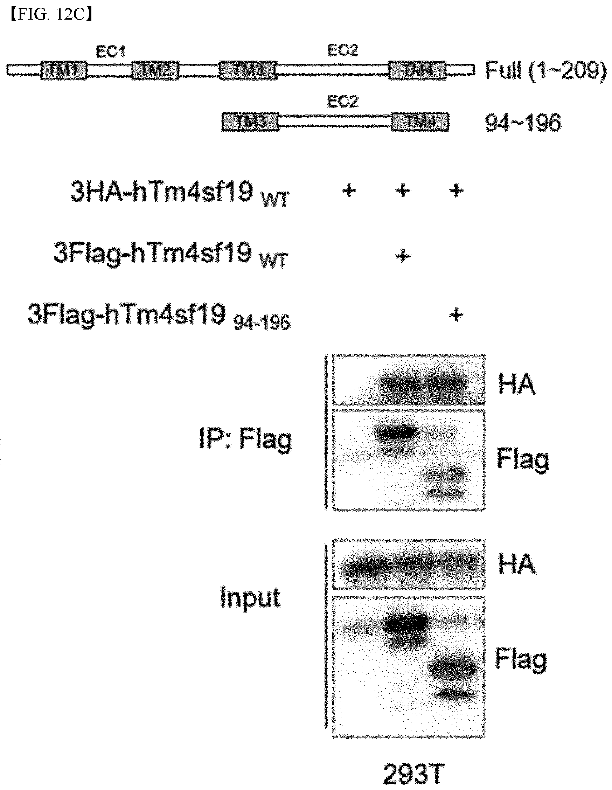

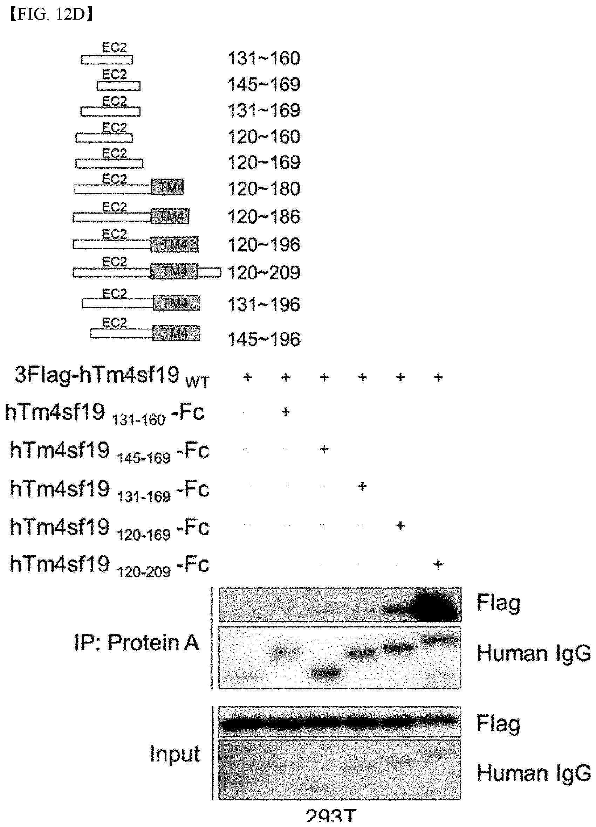

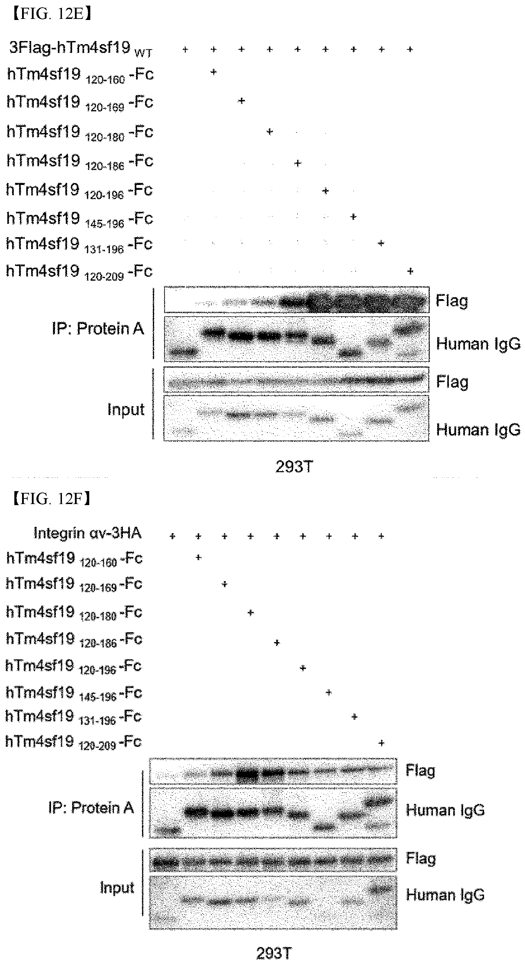

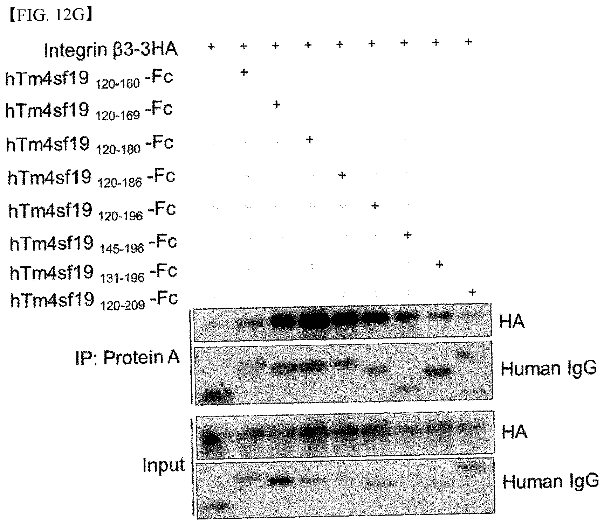

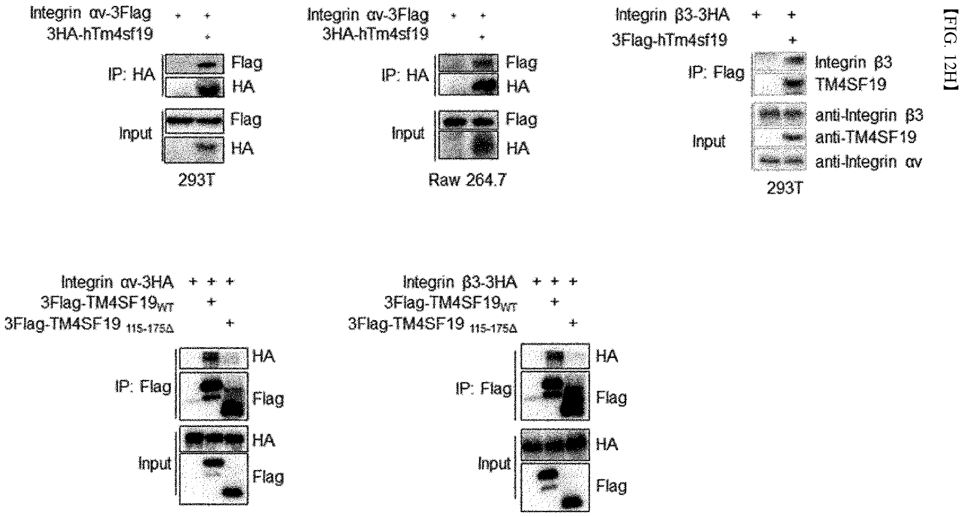

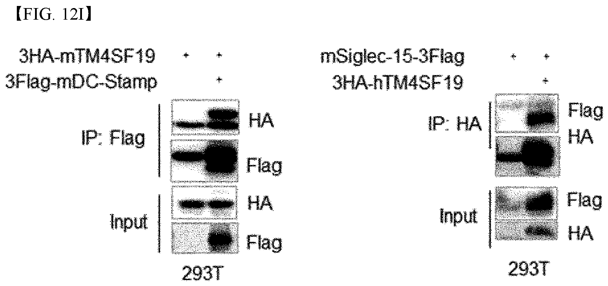

FIG. 12A is a schematic diagram illustrating the structures of hTm4sf19 variants (hTM4SF19_{115-175Δ}, hTm4sf19_{105-186Δ}, hTm4sf19_{105-196Δ}, hTm4sf19_{94-186Δ}, and hTm4sf19_{94-196Δ}) from which a partial sequence of TM4SF19 was deleted. FIG. 12B is a result showing that TM4SF19 binds to itself and is involved in intercellular interaction. It shows that, among the variants in which a partial sequence of TM4SF19 is deleted, the 94-196 deleted variant does not bind to TM4SF19 itself. FIG. 12C is a result showing that hTm4sf19 ₉₄₋₁₉₆ (top) in which other sequences, except for the sequence 94-196, are deleted from hTm4sf19 binds to wild-type (WT) and is involved in the interaction (bottom). FIGS. 12D and 12E are results showing the structures of hTm4sf19 131-160, hTm4sf19 145-169, hTm4sf19 131-169, hTm4sf19 120-160, hTm4sf19 120-169, hTm4sf19 120-180, hTm4sf19 120-186, hTm4sf19 120-196, hTm4sf19 120-209, hTm4sf19 131-196 and hTm4sf19 145-196, which are used as TM4SF19 fragments, and the interaction between TM4SF19 in which 3Flag is tagged at the N-terminus or each TM4SF19 fragment, and a hIgG1-Fc fusion protein, confirmed by immunoprecipitation. FIG. 12F is a result showing the interaction between integrin αv in which 3HA is tagged at the C-terminus and each TM4SF19 fragment fused with hIgG1 confirmed by immunoprecipitation. FIG. 12G is a result showing the interaction between integrin β3 in which 3HA is tagged at the C-terminus and each TM4SF19 fragment fused with hIgG1, confirmed by immunoprecipitation. FIG. 12H is a result of confirming the interaction after overexpressing integrin αv or integrin β3, which is a protein that regulates osteoclast functions and is involved in cytoskeletal rearrangement during multinucleated osteoclastogenesis, and TM4SF19 in 293T cells or osteoclast precursor Raw 264.7 cells. It also shows the interaction between integrin αv or integrin β3 in which 3HA is tagged at the C-terminus, and TM4SF19 or a TM4SF19 EC2 (115-175)-deleted variant in which 3Flag is tagged at the N-terminus. FIG. 12I shows the interaction between TM4SF19, and DC-Stamp or siglec-15, which are membrane proteins involved in the regulation of osteoclast functions and cytoskeletal rearrangement during multinucleated osteoclast formation, confirmed by immunoprecipitation.
FIG. 13 is a result of confirming cell proliferation, colony formation, and cell migration after overexpressing TM4SF19 in murine breast cancer cells E0771 [LPCX: retroviral control vector].
FIG. 14 is a result confirming the inhibition of lung metastasis after injecting murine breast cancer cells E0771 into the tail vein of wild-type (WT), TM4SF19KO mice, and TM4SF19EC2Δ mice.
FIG. 15 shows the expression of target genes (CDH2, SNAI1 and SNAI2) involved in cell migration, confirmed by qPCR (FIG. 15A), and the expression of metastasis-associated proteins (vimentin, slug, bnail, E-cadherin, β-actin), confirmed by western blotting (FIG. 15B), after cells are isolated from the bone marrow of wild-type (WT) mice and TM4SF19KO mice and differentiate into bone marrow-derived macrophages, and co-cultured with breast cancer cells to allow cell migration [BMDM: bone marrow-derived macrophages].
FIG. 16 shows the expression of genes (C/EBPα, PPARγ), confirmed by qPCR (top), and the expression of proteins (TM4SF19, PPARγ, and FABP4), confirmed by western blotting (bottom), after differentiation of human adipose-derived mesenchymal stem cells (hADMSCs).
FIG. 17 is a result confirming the increase in weight of 6-week-old wild-type (WT) mice and TM4SF19KO mice, which have been fed a normal diet, while being fed a high-fat diet (60% fat) for 16 weeks.
FIG. 18 shows (A) insulin resistance, confirmed by HOMA-IR, (B) liver phenotypes obtained by fixing liver tissue and stained with H&E, and (C) triglyceride levels in the liver after feeding 6-week-old wild-type (WT) mice and TM4SF19KO mice, which had been fed a normal diet, a high-fat diet for 12 weeks.

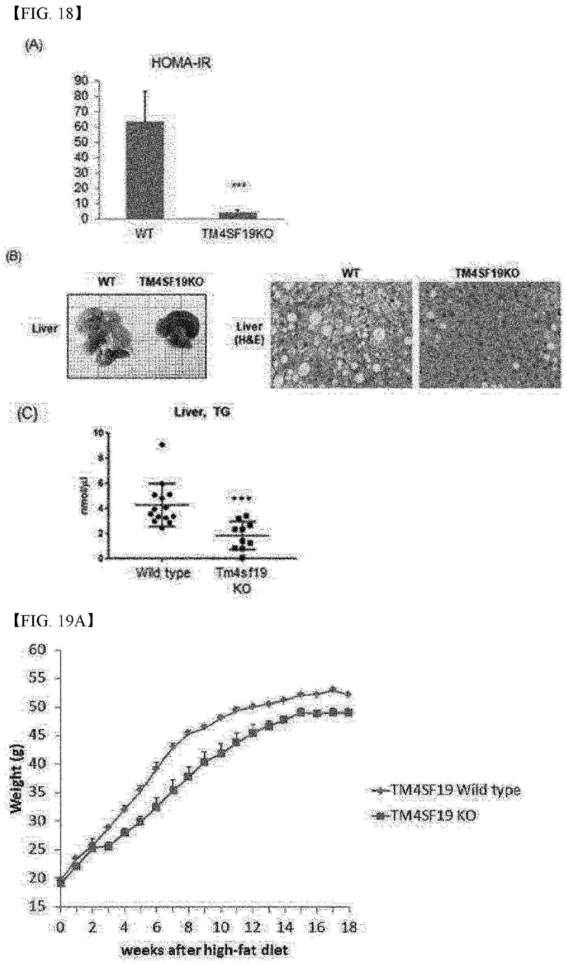

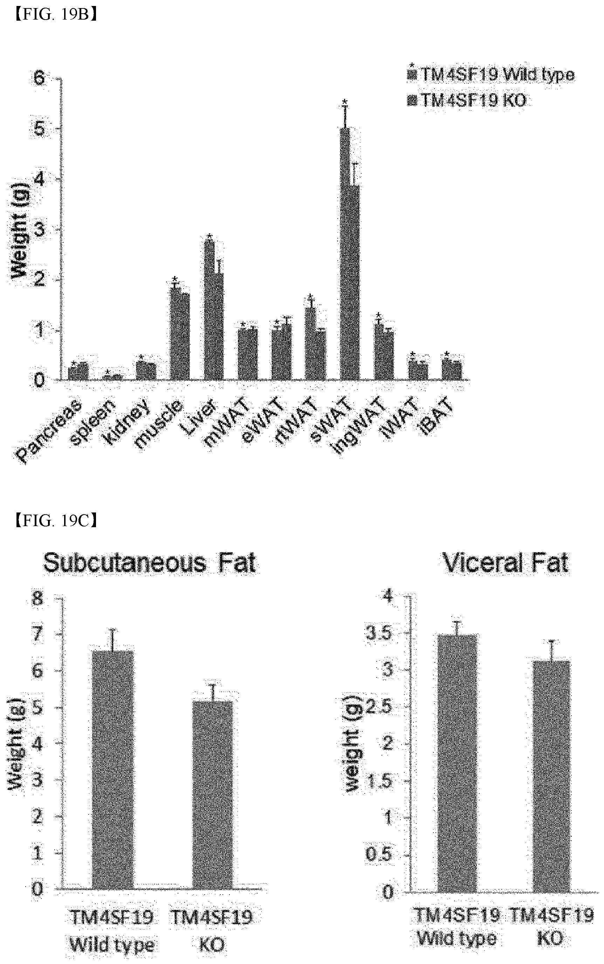

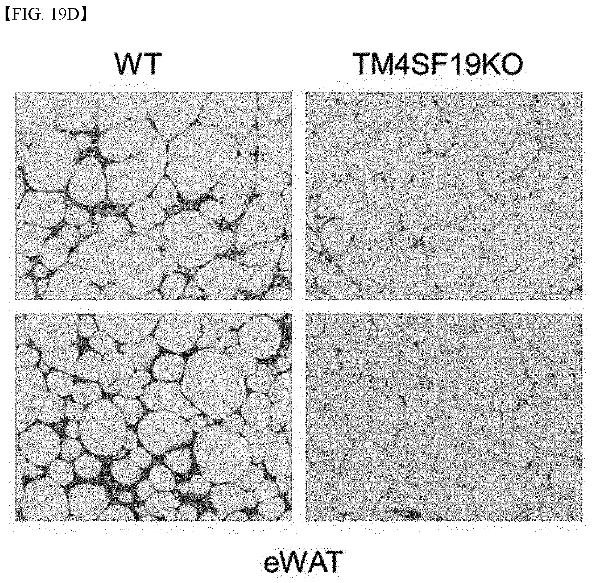

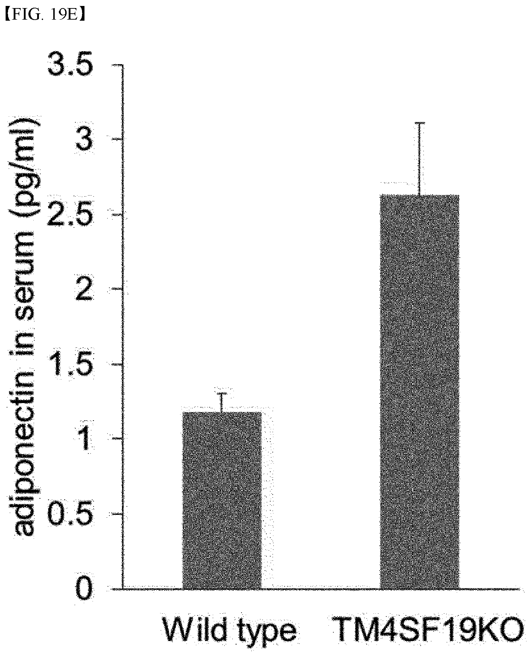

FIG. 19 shows (a) the increase in weight, (b) the weight of each tissue, and (c) the weights of subcutaneous fat and visceral fat, measured while 6-week-old wild-type (WT) mice and TM4SF19KO mice, which had been fed a normal diet, are fed a high-fat diet for 18 weeks, (d) fat phenotypes and adipose tissue macrophages around fat confirmed by H&E staining of epididymal white adipose tissue (eWAT), and (e) the secretion of the anti-obesity cytokine adiponectin in serum [rtWAT: retroperitoenal white adipose tissue, sWAT: subcutaneous white adipose tissue, ingWAT: inguinal white adipose tissue, iWAT: interscapular white adipose tissue, iBAT: interscapular brown adipose tissue].
FIG. 20 shows the expression of adipogenic differentiation markers (top), macrophage/M1-like macrophage markers (bottom) in epididymal white adipose tissue (eWAT) and subcutaneous white adipose tissue (sWAT), confirmed after 6-week-old wild-type (WT) mice and TM4SF19KO mice, which have been fed a normal diet, are fed a high-fat diet for 12 weeks.
FIG. 21 shows % of macrophages (A) and % of dendritic cells (B), confirmed by FACS analysis for epididymal white adipose tissue (eWAT) after 6-week-old wild-type (WT) mice and TM4SF19KO mice, which have been fed a normal diet, are fed a high-fat diet for 12 weeks.
FIG. 22 shows the result of confirming the expression of macrophage marker genes MCP1 and F4/80, M1-like macrophage markers IL6 and TNFα, and M2-like macrophage marker IL10 in stromal vascular fractions (SVFs) derived from epididymal white adipose tissue after feeding wild-type (WT) and TM4SF19KO mice a high-fat diet for 12 weeks.
FIG. 23 shows the role of TM4SF19 in inflammation, insulin resistance and fatty liver in eWAT.
FIG. 24A is a result confirming the conversion of eWAT to a beige or brown adipocyte phenotype of a TM4SF19KO mouse in a high-fat diet-induced obese mouse model fed a high-fat diet for 12 weeks after starting a high-fat diet for 6-week-old mice fed a normal diet.
FIG. 24B is a result confirming Ucp1 expression in white fat of a TM4SF19KO mouse in a high-fat diet-induced obese mouse model.
FIG. 24C shows a process of in vitro for brown fat differentiation model.
FIG. 24D is a result confirming that Ucp1 is increased in TM4SF19KO in an in vitro model for brown fat differentiation.
FIG. 24E is a result confirming that TM4SF19 is involved in changing white adipocytes into beige adipocytes.
FIG. 25A is a schematic diagram of cell types in each fraction after white fat is digested with collagen type I and divided into adipose tissue, an infranatant and an SVF fraction through centrifugation. It is a schematic diagram of the cell types in each fraction.
FIG. 25B is a result confirming the expression of TM4SF19 after epididymal white adipose and subcutaneous white fat of mice fed a high-fat diet (60% fat) for 24 weeks are divided into adipocytes and an SVF.
FIG. 26 shows that thermogenic gene expression and beige adipose marker gene expression after an SVF is extracted from subcutaneous white fat and isopropanol is used to induce differentiation into brown fat in wild-type (WT) and TM4SF19KO mice.
FIG. 27 is a result of the contact co-culture (right) of adipocytes together with macrophages after differentiation using an in vitro model, and then identifying marker genes for macrophage, inflammation, and adiponectin (left) [Control: After macrophages and adipocytes are grown, RNA is extracted, combined and used].
FIG. 28 is a result of inducing an inflammatory change after an SVF is extracted from wild-type (WT) mice to allow adipogenesis, differentiates into WT bone marrow macrophages and then is subjected to separate culture or contact co-culture [Control (ctrl): After macrophages and adipocytes are separately cultured, RNA is extracted, combined and used].
FIG. 29 is a result of inducing inflammatory changes after an SVF is extracted from wild-type (WT) mice and TM4SF19KO mice to allow adipogenesis, differentiates into WT bone marrow macrophages and then is subjected to contact co-culture.
FIG. 30 is a result of inducing inflammatory changes after an SVF is extracted from wild-type (WT) mice and TM4SF19KO mice to allow adipogenesis, differentiates into macrophages and then is subjected to separate culture or contact co-culture [Control (ctrl): After macrophages and adipocytes are separately cultured, RNA is extracted, combined and used].
FIG. 31 is a result of confirming the process of manufacturing an in vitro insulin-resistant model (top), and the adipogenic differentiation marker C/EBPα and the insulin resistance in vitro model marker C/EBPβ (middle) and an increase in TM4SF19 (bottom) after adipocytes are differentiated for 12 days and then treated with TNFα for 24 hours.
FIG. 32 shows *in vitro* insulin-resistance models which are decreased in TM4SF19KO, compared to a wild type.
FIG. 33 is a result confirming a marker involved in M1 polarization after extraction of bone marrow from wild-type (WT) mice and differentiation into macrophages.
FIG. 34 is a result confirming M1 polarization after bone marrow is extracted from wild-type (WT) mice and TM4SF19KO mice and differentiation into macrophages, and a marker involved in M1 polarization after bone marrow is extracted from wild-type mice and TM4SF19KO mice and differentiated into macrophages (bottom).
FIG. 35 is a result showing that human TM4SF19 (hTM4SF19), human EC2Δ (hTM4SF19 115-175Δ), mouse TM4SF19 (mTM4SF19), and mouse EC2Δ (mTM4SF19 116-165Δ) are transiently overexpressed in 293T cells by performing western blotting using the previously-manufactured polyclonal mouse TM4SF19 antibody to confirm expression.
FIG. 36 shows the results of extracting bone marrow from wild-type mice and differentiated into osteoclasts, treating with a TM4SF19 antibody, and staining with TRAP.
FIG. 37 shows the results of Coomassie staining (left, right) and Western blotting (middle) on human TM4SF19₁₂₀₋₁₆₉-Fc and mouse TM4SF19₁₁₆₋₁₆₅-Fc after concentrating media.
FIG. 38A shows the inhibition of multinucleated osteoclastogenesis induced by osteoclast differentiation of mM4SF19₁₁₆₋₁₆₅-Fc, confirmed by TRAP staining [E1; Sample eluted with elution buffer (20 mM glycine). E1-1; Sample subjected to ultrafiltration/diafiltration (UF/DF) using buffer A (50 mM phosphate, 50 mM NaCl, pH 7.0) (Fc may be more stable)].
FIG. 38B shows the inhibition of multinucleated osteoclast formation by treatment of hTM4SF19₁₂₀₋₁₆₉-Fc, confirmed by TRAP staining.
FIG. 38C shows that hTM4SF19₁₂₀₋₁₆₉-Fc and mTM4SF19₁₁₆₋₁₆₅-Fc inhibit multinucleated osteoclast formation by treatment of hIgG1-Fc, hTM4SF19₁₂₀₋₁₆₉-Fc and mTM4SF19₁₁₆₋₁₆₅-Fc, confirmed by TRAP staining.
FIG. 38D shows whether an actin belt is formed or not during multinucleated osteoclast formation of BMMs of wild-type mice and treatment with 10 µg/ml each of IgG-Fc and mTM4SF19-Fc, confirmed by F-actin staining.
FIG. 38E shows that bone resorption is blocked by mTM4SF19-Fc, confirmed by toluidine blue staining.
FIG. 39A is a set of micro-CT images showing the bone loss inhibitory effect by mouse TM4SF19-Fc.
FIG. 39B shows 3D micro-CT parameters including a bone volume (BV), a ratio of bone volume to tissue volume (% BV/TV), an average trabecular number (Tb.N) and a trabecular separation (Tb.Sp), which are obtained by analyzing the micro-CT images of FIG. 39A using a 3D imaging program.
FIG. 39C shows the histopathological analysis result for mouse femur tissue through H&E staining.
FIG. 39D shows that bone loss is remarkably inhibited after the tail vein injection of hIgG1-Fc, mouse TM4SF19-Fc (116-165), and human TM4SF19-Fc (131-169) after an ovariectomy in 8-week-old female mice.
FIG. 40A is a set of graphs showing the mRNA expression of Ctsk and Acp5 in differentiated cells under conditions of M-CSF or M-CSF and RANKL treatment and treatment with 10 µg/ml of mouse TM4SF19-Fc.
FIG. 40B shows the result of confirming the expression of osteoclast differentiation marker proteins after the treatment of 5 µg/ml of mTM4SF19-Fc while differentiating under a condition in which cells extracted from WT mouse bone marrow are treated with M-CSF or M-CSF and RANKL (M-CSF 25 ng/ml, RANKL 100 ng/ml).
FIG. 41 shows surface binding of mTM4SF19-Fc before and after the differentiation of Raw264.7 cells, confirmed by FACS analysis.

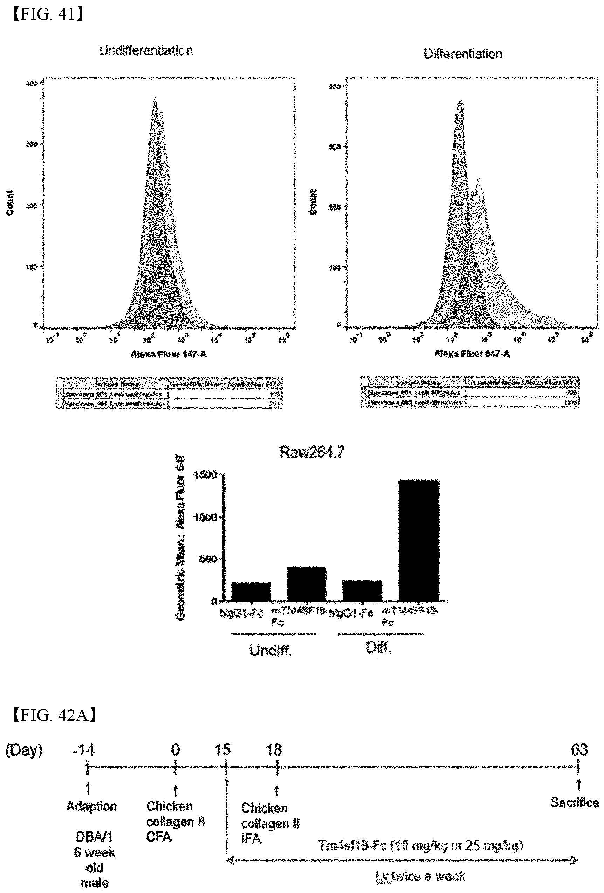

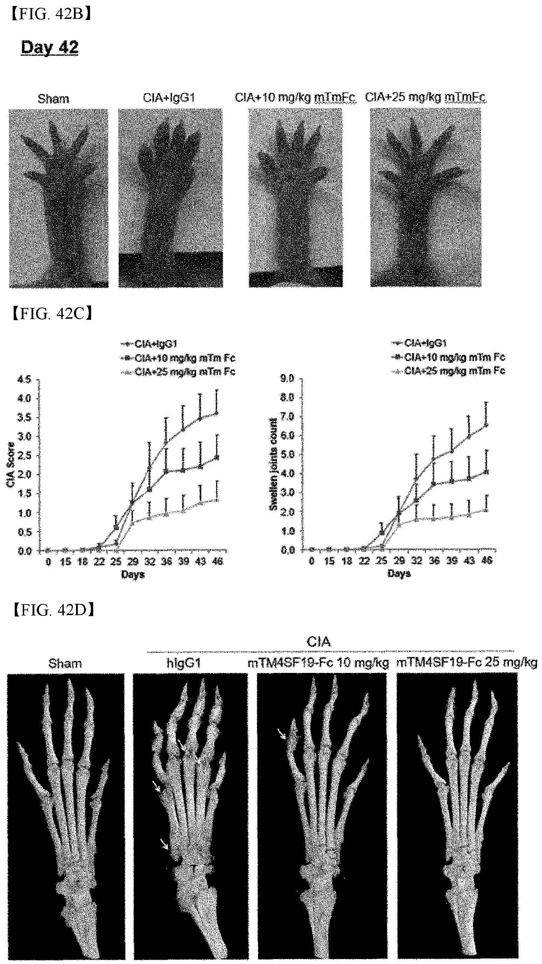

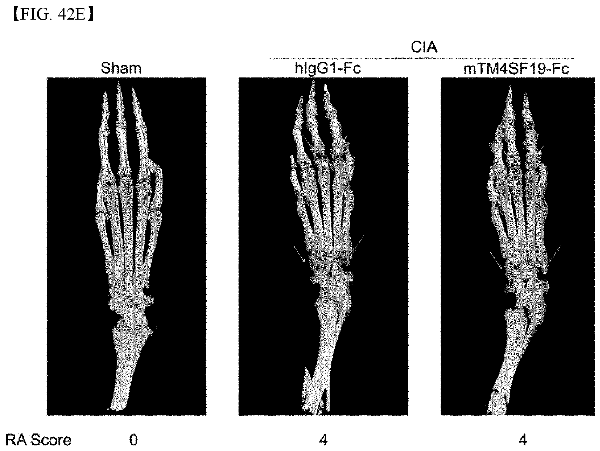

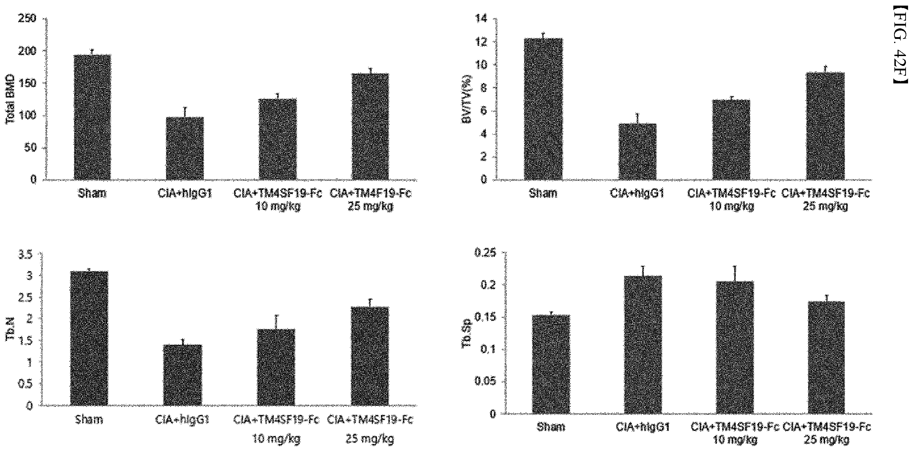

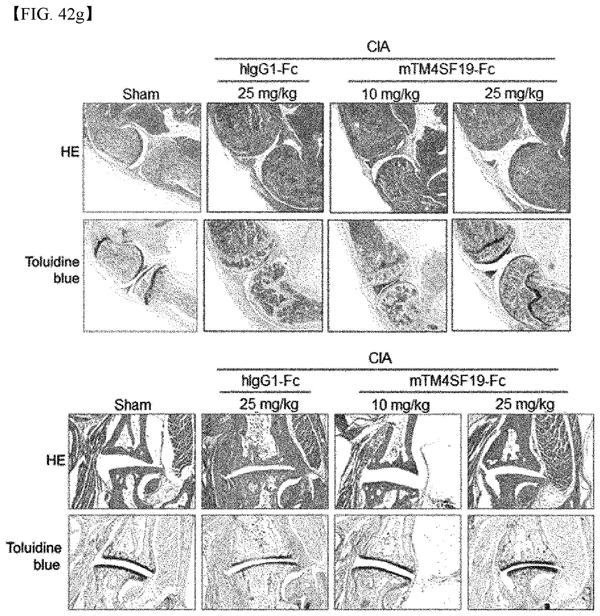

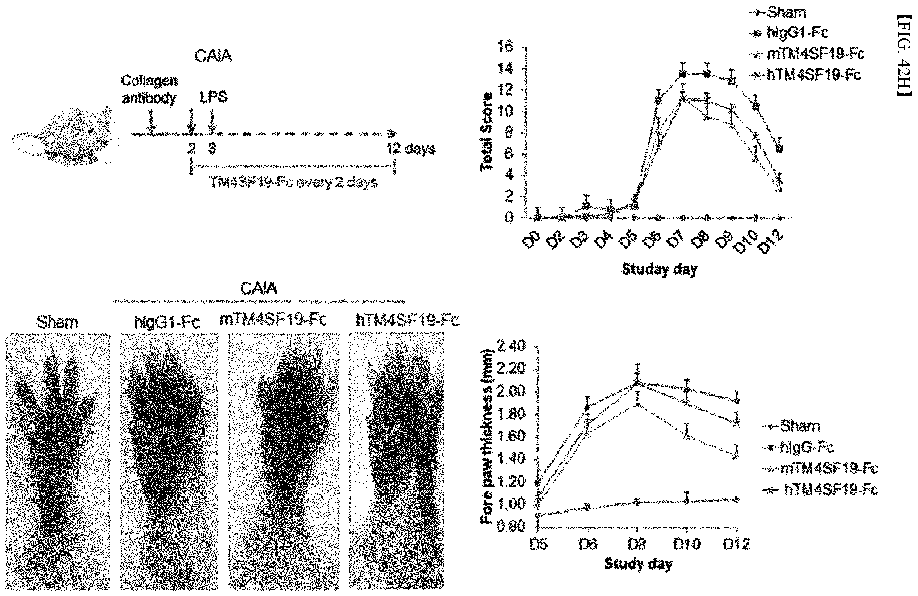

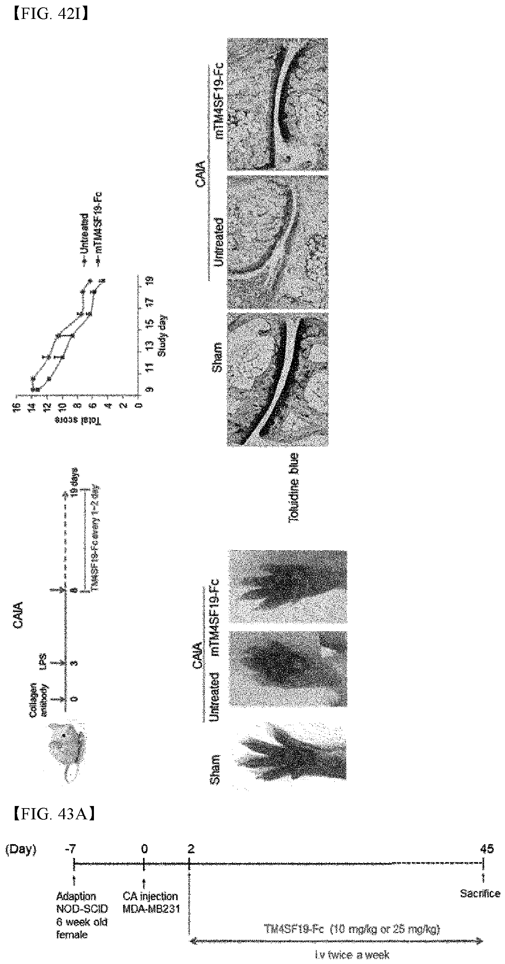

FIG. 42A schematically shows an experimental schedule for confirming the prevention and treatment of rheumatoid arthritis using TM4SF19-Fc. FIG. 42B shows a result of confirming the joint state of mice 42 days after the induction of collagen induced arthritis (CIA). FIG. 42C shows a result of analyzing CIA scores and the number of swollen joints, which was investigated during the experimental period. FIG. 42D is a set of micro-CT images showing that inflammation and bone damage occurring in the paw of collagen-induced arthritis mouse models are dose-dependently inhibited by mTM4SF19-Fc treatment. FIG. 42E is a result showing that inflammation and bone damage occurring in the paw of collagen-induced arthritis mouse models are dose-dependently inhibited by mTM4SF19-Fc treatment. FIG. 42F shows 3D micro-CT parameters (bone microstructure parameters) including a bone volume (BV), a ratio of bone volume to tissue volume (% BV/TV), an average trabecular number (Tb.N), and a trabecular separation (Tb.Sp) by analyzing the micro-CT images of the femurs of the collagen-induced arthritis mice using a 3D imaging program. FIG. 42G is a result of confirming cartilage damage in collagen-induced arthritis mice using toluidine blue. Compared to a hIgG1-Fc treated group, it is shown that the cartilage damage is inhibited in the mTM4SF19-Fc treated group. FIG. 42H shows the semi-therapeutic effects of mTM4SF19-Fc and hTM4SF19-Fc (120-169) in LPS-injected arthritis-induced mouse models three days after collagen antibody treatment, assessed by arthritis score. Compared to hIgG 1-Fc, it was confirmed that the arthritis disease score was lowered by TM4SF19-Fc treatment and a paw thickness was reduced. FIG. 42I shows the therapeutic effect of mTM4SF19-Fc in arthritis-induced mouse models, which are subjected to LPS injection three days after collagen antibody treatment, 8 days after the LPS injection at which the arthritis disease score is the maximum. Compared to an untreated group, TM4SF19-Fc treatment leads to decreases in arthritis disease score and paw thickness, and inhibited cartilage damage, confirmed by toluidine blue.
FIG. 43A schematically shows an experimental schedule for confirming a bone metastasis inhibitory effect on breast cancer using mTM4SF19-Fc. FIG. 43B shows a result of confirming a cancer progression status through bioluminescence image analysis. FIG. 43C shows micro-CT scans of the joint of mice with bone metastasis of breast cancer. FIG. 43D shows the result of histological analysis for the joint of mice in which breast cancer has metastasized to the bone, through H&E staining.
FIG. 44A schematically shows an experimental schedule for confirming a bone metastasis inhibitory effect on breast cancer using hTM4SF19-Fc (145-169). FIG. 44B shows the result of confirming the cancer progression status through bioluminescence imaging analysis. FIG. 44C shows the result of luminescence analysis on the joint of mice in which the metastasis of breast cancer to the bone occurs. FIG. 44D shows micro-CT scans of the joint of the mice in which the metastasis of breast cancer to the bone occurs.
FIG. 45 is a result confirming that lung metastasis is remarkably inhibited by mTM4SF19-Fc (116-165) and hTM4SF19-Fc (145-169) after lung metastasis is caused by injecting murine E0771 breast cancer cells into the tail vein of wild-type mice.
FIG. 46A shows that high-fat diet-induced obesity is inhibited by mTM4SF19-Fc treatment.
FIG. 46B shows that mTM4SF 19-Fc treatment induces the effect of reducing the weight of white fat in an organ and inhibiting fatty liver by a high-fat diet, confirmed by an organ weight and a ratio of an organ weight to a body weight.
FIG. 47 shows the effect of reducing fatty liver and reducing triglycerides in plasma according to mTM4SF19-Fc treatment, confirmed by H&E staining and Masson & trichrome staining.
FIG. 48 shows that mTM4SF19-Fc (116-165) treatment dose-dependently inhibits adipocyte differentiation, confirmed by treating lipid droplets through oil-red-O staining and by the expression of genes of adipogenesis markers C/EBPα and PPAR-γ.
FIG. 49 shows that TM4SF19-Fc inhibits cancer cell migration induced by osteoclast differentiation, confirmed by staining with 0.05% crystal violet solution.
FIG. 50A shows the inhibition of cancer cell migration by osteoclast differentiation in TM4SF19KO, confirmed by staining with a 0.05% crystal violet solution.
FIG. 50B shows the inhibition of cancer cell migration by osteoclast differentiation in TM4SF19EC2Δ, confirmed by staining with a 0.05% crystal violet solution.
FIG. 51 is a result of confirming cell growth after stably overexpressing 3Flag-hTM4SF19 in a MG63 osteosarcoma cell line by MTT analysis, and confirming colony formation and migration.
FIG. 52 is a result of confirming cell growth after stably overexpressing 3Flag-hTM4SF19 in a HOS osteosarcoma cell line by MTT analysis, and confirming colony formation and migration.
FIG. 53 is a result of confirming cell growth using different TM4SF19 clones (#4 and #6) which have been knocked out by CRISPR in a 143b osteosarcoma cell line through MTT analysis, and confirming colonization ability by colony formation.
FIG. 54 is a result of confirming cell growth by treating 143b osteosarcoma cells with hTM4SF19-Fc (aa 120-169) by MTT analysis (FIG. 54A) and the number of cells (FIG. 54C), confirming colonization ability by colony formation (FIG. 54B), and confirming cell migration (FIG. 54D).
FIG. 55 shows that U2OS and MG63 osteosarcoma colony formation is inhibited by treatment with human TM4SF19-Fc (aa 120-169) (10 µg/ml).
FIG. 56 shows that the cell migration ability of HOS osteosarcoma cells is inhibited by treatment with 10 µg/ml of hTM4SF19-Fc (aa 120-169) and hTM4SF19-Fc (aa 145-169).
FIG. 57A shows the inhibition of pancreatic cancer cell colony formation by human TM4SF19-Fc (aa 120-169).
FIG. 57B shows the inhibition of pancreatic cancer cell growth by human TM4SF19-Fc (aa 120-169), confirmed by a cell count.
FIGS. 58A to 58D show the information on the sequences of TM4SF19 fragments and TM4SF19-Fc fusion proteins used in examples of the present invention [In each drawing, the corresponding target sequence is shown in bold and represented by SEQ ID NOs: 8, 12, 15 or 18; an enzymatic site is a site between the target sequence and the immunoglobulin Fc region; and the immunoglobulin Fc region is the highlighted portion and represented by SEQ ID NO:9].

### [Modes of the Invention]

Hereinafter, the present invention will be described in detail.

Meanwhile, embodiments of the present invention may be modified in a variety of different forms, and it should not be construed that the scope of the present invention is not limited to the following embodiments. In addition, the embodiments of the present invention are provided to more completely explain the present invention to those of ordinary skill in the art. Further, throughout the specification, when one part "includes" a component, it means that it may also include other components rather than excluding components unless particularly stated otherwise.

The present invention relates to a pharmaceutical composition for preventing or treating a bone disease, which includes an inhibitor of TM4SF19 expression or activity as an active ingredient.

The present invention also relates to a pharmaceutical composition for preventing or treating obesity or an obesity-mediated metabolic disease, which includes an inhibitor of TM4SF19 expression or activity as an active ingredient.

The present invention also relates to a pharmaceutical composition for preventing or treating cancer, or inhibiting cancer metastasis, which includes: an inhibitor of TM4SF19 expression or activity as an active ingredient.

The term "TM4SF19" used herein refers to transmembrane 4 L six family member 19 (TM4SF19, OCTM4), belonging to the transmembrane 4L 6 superfamily. Such a superfamily member is known to be involved in various cellular processes, including cell proliferation, motility and adhesion through interaction with integrins, and to be associated with diseases such as liver fibrosis and cancer. However, TM4SF19 has not been properly studied in relation to its function.

In the case of a TM4SF19 protein, the same protein may be expressed not only in a human (Homo sapiens) or mouse (Mus musculus), but in other mammals such as a monkey, a cow, a horse, a dog, or a cat.

Human-derived TM4SF19 may be translated into a peptide or protein including respective amino acid sequences represented by NP_612470.2, NP_001191826.1, and NP_001191827.1 from respective mRNAs of GenBank Accession Nos. NM_138461.4, NM_001204897.2, and NM_001204898.2. That is, human-derived TM4SF19 includes three types of transcript variants (GenBank Accession No. NM_138461.4 (transcript variant 1), NM_001204897.2(transcript variant 2), NM_001204898.2 (transcript variant 3)), and three types of isoforms (GenBank Accession No. NP_612470.2 (isoform 1), NP_001191826.1 (isoform 2), NP_001191827.1(isoform 3)). The TM4SF19 used in the embodiment of the present invention is NP_612470.2 (isoform 1) obtained from mRNA including GenBank Accession No. NM_138461.4 (transcript variant 1). Isoform 1 has a high homology with mouse TM4SF19.

Mouse TM4SF19 is GenBank Accession No. NP_001153874.1, represented by SEQ ID NO: 7.
<GenBank Accession No. NP_612470.2 (isoform 1) [SEQ ID NO: 1]> 
MVSSPCTQAS SRTCSRILGL SLGTAALFAA GANVALLLPN
WDVTYLLRGL LGRHAMLGTG 60
 
LWGGGLMVLT AAILISLMGW RYGCFSKSGL CRSVLTALLS
GGLALLGALI CFVTSGVALK 120
DGPFCMFDVS SFNQTQAWKY GYPFKDLHSR
NYLYDRSLWN SVCLEPSAAV VWHVSLFSAL 180
LCISLLQLLL VVVHVINSLL GLFCSLCEK
<GenBank Accession No. NP_001191826.1 (isoform 2) [SEQ ID NO: 2]> 
MVSSPCTQAS SRTCSRILGL SLGTAALFAA GANVALLLPN
WDVTYLLRGL LGRHAMLGTG 60
LWGGGLMVLT AAILISLMGW RYGCFSKSGL CRSVLTALLS
GGLALLGALI CFVTSGVALK 120
DGPFCMFDVS SFNQTQAWKY GYPFKDLHRI ICMTVRSGTP
SAWSPLQLLS GTCPSSPPFC 180
ASACSSFSWW SFMSSTASWA FSAASARSDR
QNLHLQAWVF SSSAVLNPFY KEWVRIINKL 240
PL
<GenBank Accession No.NP_001191827.1(isoform 3) [SEQ ID NO: 3]> 
MVSSPCTQAS SRTCSRILGL SLGTAALFAA GANVALLLPN
WDVTYLLRGL LGRHAMLGTG 60
LWGGGLMVLT ALLSGGLALL GALICFVTSG VALKDGPFCM
FDVSSFNQTQ AWKYGYPFKD 120
 
LHSRNYLYDR SLWNSVCLEP SAAVVWHVSL FSALLCISLL
QLLLVVVHVI NSLLGLFCSL 180
CEK
<GenBank Accession No. NM_138461.4 (transcript variant 1) [SEQ ID NO: 4]> 
1 aaagagtcct ggaaagacaa ccttcaggtc cagccctgga gctggaggag

tggagcccca

61 ctctgaagac gcagcctttc tccaggttct gtctctccca ttctgattct tgacaccaga

121 tgcaggatgg tgtcctctcc ctgcacgcag gcaagctcac ggacttgctc

ccgtatcctg

181 ggactgagcc ttgggactgc agccctgttt gctgctgggg ccaacgtggc

actcctcctt

241 cctaactggg atgtcaccta cctgttgagg ggcctccttg gcaggcatgc

catgctggga

301 actgggctct ggggaggagg cctcatggta ctcactgcag ctatcctcat

ctccttgatg

361 ggctggagat acggctgctt cagtaagagt gggctctgtc gaagcgtgct

tactgctctg

421 ttgtcaggtg gcctggcttt acttggagcc ctgatttgct ttgtcacttc tggagttgct

481 ctgaaagatg gtcctttttg catgtttgat gtttcatcct tcaatcagac acaagcttgg

541 aaatatggtt acccattcaa agacctgcat agtaggaatt atctgtatga ccgttcgctc

601 tggaactccg tctgcctgga gccctctgca gctgttgtct ggcacgtgtc cctcttctcc

661 gcccttctgt gcatcagcct gctccagctt ctcctggtgg tcgttcatgt catcaacagc
 
721 ctcctgggcc ttttctgcag cctctgcgag aagtgacagg cagaaccttc
acttgcaagc
781 atgggtgttt tcatcatcgg ctgtcttgaa tcctttctac aaggagtggg tacgaattat
841 aaacaaactt cccctttagg tatccctgga gtaataatga caacaaaatt
cactgcaggt
901 cggtggaatg atagaatgca ttttaaatca cattgtaaac ttccaggtga tccatggata
961 ggataaataa ctaagttatt ataattgttt aggaatttat agtccataaa atatcctcca
1021 gccagg
<GenBank Accession No. NM_001204897.2(transcript variant 2) [SEQ ID NO: 5]> 
1 aaagagtcct ggaaagacaa ccttcaggtc cagccctgga gctggaggag
tggagcccca
61 ctctgaagac gcagcctttc tccaggttct gtctctccca ttctgattct tgacaccaga
121 tgcaggatgg tgtcctctcc ctgcacgcag gcaagctcac ggacttgctc
ccgtatcctg
181 ggactgagcc ttgggactgc agccctgttt gctgctgggg ccaacgtggc
actcctcctt
241 cctaactggg atgtcaccta cctgttgagg ggcctccttg gcaggcatgc
catgctggga
301 actgggctct ggggaggagg cctcatggta ctcactgcag ctatcctcat
ctccttgatg
 
361 ggctggagat acggctgctt cagtaagagt gggctctgtc gaagcgtgct
tactgctctg
421 ttgtcaggtg gcctggcttt acttggagcc ctgatttgct ttgtcacttc tggagttgct
481 ctgaaagatg gtcctttttg catgtttgat gtttcatcct tcaatcagac acaagcttgg
541 aaatatggtt acccattcaa agacctgcat agaattatct gtatgaccgt tcgctctgga
601 actccgtctg cctggagccc tctgcagctg ttgtctggca cgtgtccctc
ttctccgccc
661 ttctgtgcat cagcctgctc cagcttctcc tggtggtcgt tcatgtcatc aacagcctcc
721 tgggcctttt ctgcagcctc tgcgagaagt gacaggcaga accttcactt
gcaagcatgg
781 gtgttttcat catcggctgt cttgaatcct ttctacaagg agtgggtacg aattataaac
841 aaacttcccc tttaggtatc cctggagtaa taatgacaac aaaattcact
gcaggtcggt
901 ggaatgatag aatgcatttt aaatcacatt gtaaacttcc aggtgatcca tggataggat
961 aaataactaa gttattataa ttgtttagga atttatagtc cataaaatat cctccagcca
1021 gg
<GenBank Accession No. NM_001204898.2(transcript variant 3) [SEQ ID NO: 6]> 
1 aaagagtcct ggaaagacaa ccttcaggtc cagccctgga gctggaggag

tggagcccca

61 ctctgaagac gcagcctttc tccaggttct gtctctccca ttctgattct tgacaccaga
 
121 tgcaggatgg tgtcctctcc ctgcacgcag gcaagctcac ggacttgctc
ccgtatcctg
181 ggactgagcc ttgggactgc agccctgttt gctgctgggg ccaacgtggc
actcctcctt
241 cctaactggg atgtcaccta cctgttgagg ggcctccttg gcaggcatgc
catgctggga
301 actgggctct ggggaggagg cctcatggtg cttactgctc tgttgtcagg
tggcctggct
361 ttacttggag ccctgatttg ctttgtcact tctggagttg ctctgaaaga tggtcctttt
421 tgcatgtttg atgtttcatc cttcaatcag acacaagctt ggaaatatgg ttacccattc
481 aaagacctgc atagtaggaa ttatctgtat gaccgttcgc tctggaactc
cgtctgcctg
541 gagccctctg cagctgttgt ctggcacgtg tccctcttct ccgcccttct gtgcatcagc
601 ctgctccagc ttctcctggt ggtcgttcat gtcatcaaca gcctcctggg ccttttctgc
661 agcctctgcg agaagtgaca ggcagaacct tcacttgcaa gcatgggtgt
tttcatcatc
721 ggctgtcttg aatcctttct acaaggagtg ggtacgaatt ataaacaaac ttccccttta
781 ggtatccctg gagtaataat gacaacaaaa ttcactgcag gtcggtggaa
tgatagaatg
841 cattttaaat cacattgtaa acttccaggt gatccatgga taggataaat aactaagtta
901 ttataattgt ttaggaattt atagtccata aaatatcctc cagccagg
<GenBank Accession No. NP_001153 874.1[SEQ ID NO: 7]>

MLSFSRVVNC SRTCSRFLGL SLGTASLCAA GANIALLFPN
WDVTYLMRGL IGKHAMLGSG
LWGGGLMVLL AATLISMTGS FSKSAPCLQV LIALLSSGLA
LLGAVICFVT SGVALKDGPF
CMFDVSSFNQ TQAWKFGYPF KDLHNRNYLY
DRSLWTSVCL EPSKAVVWHV AFFSILLCIS
LLQLLLVAIH LVNSILGLFC SFCEKH

The inhibitor of TM4SF19 expression or activity of the present invention refers to a material that reduces the expression of the TM4SF19 gene or the activity of the TM4SF19 protein. In one embodiment, the inhibitor for inhibiting the expression of the TM4SF19 gene of the present invention refers to a material that directly acts on the TM4SF19 gene or indirectly acts on an up-regulator of the TM4SF19 gene to reduce the expression of the TM4SF19 gene at the transcription level, increase the degradation of the expressed TM4SF19 gene, or inhibit its activity, thereby reducing the expression level of the TM4SF19 gene or its activity. Specifically, the inhibitor for inhibiting the expression of the TM4SF19 gene may include one or more selected from the group consisting of an antisense nucleotide, small hairpin RNA (shRNA), small interfering RNA (siRNA), microRNA (miRNA) and a ribozyme, which complementarily bind to mRNA of the TM4SF19 gene, but the present invention is not limited thereto.

The term "antisense nucleotide" used herein refers to a DNA or RNA sequence complementary to a specific gene and capable of binding to TM4SF19 mRNA. Since the antisense nucleotides are long chains of monomer units, they can be easily synthesized for a target gene sequence.

The term "small interfering RNA (siRNA)" refers to short double-stranded RNA capable of inducing RNA interference (iRNA) through the cleavage of specific mRNA. siRNA includes a sense RNA strand having a sequence homologous to the mRNA of a target gene and an antisense RNA strand having a sequence complementary thereto. Since siRNA can inhibit the expression of the target gene, it is used for a gene knockdown method or gene therapy.

The term "short hairpin RNA (shRNA)" used herein is single-stranded RNA and is divided into a stem part forming a double-stranded part by a hydrogen bond and a loop part formed in a ring shape, and is processed by a protein such as a dicer to be changed into siRNA, and thus can play the same function as siRNA.

The term "microRNA (miRNA)" used herein refers to a 21 to 23-nt non-coding RNA which regulates post-transcriptional gene expression by promoting the degradation of target RNA or inhibiting the translation thereof.

The term "ribozyme" used herein refers to an RNA molecule that recognizes a specific base sequence and has an enzyme-like function that cleaves it by itself. The ribozyme is composed of a region binding to a complimentary base sequence of a target messenger RNA strand with specificity and a region cleaving target RNA.

In addition, in one embodiment, an inhibitor for inhibiting the activity of TM4SF19 protein according to the present invention may be one or more selected from the group consisting of a compound, a peptide, a peptide mimetic, an aptamer, a fusion protein and an antibody, which specifically bind to the TM4SF19 protein, but the present invention is not limited thereto.

The term "compound" used herein includes all compounds that specifically bind to the TM4SF19 protein to inhibit its activity.

The term "peptide" used herein has an advantage of high binding strength to a target material, and does not undergo denaturation even during heat/chemical treatment. In addition, because of its small molecular size, it can be used as a fusion protein by attachment to another protein. Specifically, since it can be used by attachment to a polymer protein chain, it can be used as a diagnostic kit and a drug delivery material.

The term "peptide mimetic" used herein inhibits a binding domain of the TM4SF19 protein to inhibit the activity of the TM4SF19 protein. The peptide mimetic may be a peptide or a non-peptide, and consist of an amino acid bound by a non-peptide bond such as a psi bond. In addition, the peptide mimetic may be a "conformationally constrained" peptide, cyclic mimetic, or a cyclic mimetic including at least one exocyclic domain, a binding part (binding amino acid) and an active part. The peptide mimetic is a novel small molecule which can be similarly structured to the characteristics of the secondary structure of the TM4SF19 protein, imitate an inhibitory property of a large molecule such as an antibody or a soluble receptor, and act to have an equivalent effect to that of a natural antagonist.

The term "aptamer" used herein refers to a single-stranded nucleic acid (DNA, RNA or modified nucleic acid) having a stable tertiary structure on its own and capable of binding to a target molecule with high affinity and specificity.

The term "fusion protein" used herein is a novel protein made in a form in which two or more different proteins or the same type of protein is bound, and is also called a chimeric protein. Two or more heterologous proteins are bound in a part-to-part, part-to-whole, or whole-to-whole manner. In many cases, if necessary, the sequences of one gene and another gene are codon-aligned and joined to form a hybrid gene, which is expressed to produce a protein.

In one embodiment, the fusion protein according to the present invention may include a TM4SF19 fragment specifically binding to the TM4SF19 protein.

In addition to the fragment, the immunoglobulin Fc domain may be further included.

The fragment may be all or a part of an extracellular loop 2 (EC2)-derived fragment of TM4SF19. The amino acid sequence region corresponding to the entire EC2 may be the region of amino acids 120-169 of a human TM4SF19 protein or the region of amino acids 116-165 of a mouse TM4SF19 protein. The amino acid sequence region corresponding to a part of EC2 may include the region of amino acids 145-169 or 131-169 of the human TM4SF19 protein.

The preferable fusion protein according to the present invention may include an amino acid sequence represented by SEQ ID NO: 10, SEQ ID NO: 13, SEQ ID NO: 16 or SEQ ID NO: 19.

As an embodiment, the present invention may include a fusion protein in which the immunoglobulin Fc domain binds to a TM4SF19 fragment, for example, a fusion protein in which the immunoglobulin Fc domain binds to all or a part of the extracellular loop region of TM4SF19; or a fusion protein in which an immunoglobulin Fc domain binds to all or a part of the extracellular loop region of TM4SF19 and all or a part of a membrane protein. Here, the fusion protein in which the immunoglobulin Fc domain binds to the TM4SF19 fragment includes those in which an immunoglobulin Fc domain is indirectly bound to the TM4SF19 fragment in addition to that in which the immunoglobulin Fc domain directly binds to the TM4SF19 fragment. When the immunoglobulin Fc domain indirectly binds to the TM4SF19 fragment, there may be additional 1 to 10 amino acids, for example, 1 to 9, 1 to 8, 1 to 7, 1 to 6, 1 to 5, 1 to 4, 1 to 3, 1 to 2, specifically, 1 amino acid, corresponding to a linker, spacer or enzyme site, between the TM4SF19 fragment and the immunoglobulin Fc domain.

Available TM4SF19 fragments in the present invention include, but not limited to, various examples (hTm4sf19 94-196, hTm4sf19 131-160, hTm4sf19 145-169, hTm4sf19 131-169, hTm4sf19 120-160, hTm4sf19 120-169, hTm4sf19 120-180, hTm4sf19 120-186, hTm4sf19 120-196, hTm4sf19 120-209, hTm4sf19 131-196 and hTm4sf19 145-196). Meanwhile, for the stability of the TM4SF19 protein, the immunoglobulin Fc domain is used as an example, but a fusion partner capable of binding to the TM4SF19 protein is not limited to the immunoglobulin Fc domain.

Although not limited thereto, the present invention relates to a fusion protein for inhibiting TM4SF19 expression or activity, which includes a fragment derived from the extracellular loop 2 of the TM4SF19 protein and the immunoglobulin Fc domain.

The extracellular loop 2 (EC2)-derived fragment of the TM4SF19 protein may be an amino acid sequence region corresponding to all or a part of EC2.

The amino acid sequence region corresponding to entire EC2 may be the region of amino acids 120-169 of the human TM4SF19 protein or the region of amino acids 116-165 of the mouse TM4SF19 protein.

The amino acid sequence region corresponding to a part of EC2 may include the region of amino acids 145-169 of the human TM4SF19 protein.

The amino acid sequence region corresponding to a part of EC2 may include the region of amino acids 131-169 of the human TM4SF19 protein.

The preferable fusion protein according to the present invention may include an amino acid sequence represented by SEQ ID NO: 10, SEQ ID NO: 13, SEQ ID NO: 16, or SEQ ID NO: 19.

An enzyme site may be included between the fragment derived from the extracellular loop 2 (EC2) of the TM4SF19 protein and the immunoglobulin Fc domain.

To produce a fusion protein, the extracellular loop 2 site of the human TM4SF19 used as the TM4SF19 fragment is merely exemplary, and the present invention is not limited thereto.

The term "Fc domain" used herein refers to a protein that includes a heavy chain constant domain 2 (CH2) and a heavy chain constant domain 3 (CH3) of an immunoglobulin, and does not include heavy and light chain variable domains and a light chain constant domain 1 (CL1) of the immunoglobulin. The Fc domain may further include a hinge of the heavy chain constant domain. Although not limited, the "Fc domain" may be an IgG-, IgA-, IgD-, IgE- or IgM-derived Fc domain. In the following embodiment, human IgG1-Fc used to produce a fusion protein is merely exemplary, and the present invention is not limited thereto.

In addition, the "Fc domain" may include an "Fc domain" or "Fc domain variant" of a modified immunoglobulin, which is prepared by substituting some amino acids of the Fc region or combining different types of Fc domains. Preferably, it means an Fc domain in which, compared to a wild-type Fc domain, antibody-dependent cellular cytolysis (ADCC) or complement-dependent cytolysis (CDC) is attenuated by modifying the binding force with an FC receptor and/or the binding force with a complement. Here, the Fc domain of the modified immunoglobulin may be selected from the group consisting of IgG1, IgG2, IgG3, IgD, IgG4, and a combination thereof.

The term "antibody" used herein refers to a specific immunoglobulin directed to an antigenic site. The "antibody" includes a monoclonal antibody, a polyclonal antibody, a bispecific antibody, a multispecific antibody, a chimeric antibody, a humanized antibody, and a human antibody, and also includes antibodies known in the art or commercially available, in addition to new antibodies. As long as it specifically binds to TM4SF19, the antibody includes not only a full-length form including two heavy chains and two light chains, but also a functional fragment of the antibody molecule. The functional fragment of the antibody molecule refers to a fragment having at least an antigen-binding function, and may include Fab, F(ab'), and F(ab')2, Fv, but the present invention is not limited thereto.

The present invention relates to an anti-TM4SF19 antibody specific for all or a part of the extracellular loop 2 (EL2) region of human TM4SF19. In an exemplary embodiment, the present invention relates to an anti-TM4SF19 antibody specific for the entire region or a part of the extracellular loop 2 of human TM4SF19, represented by SEQ ID NOs: 115 to 175, of human TM4SF19 of GenBank Accession No. NP_612470.2. In another exemplary embodiment, the present invention relates to an anti-TM4SF19 antibody specific for the entire region or a part of the extracellular loop 2, represented by SEQ ID NOs: 120 to 169, of human TM4SF19 of GenBank Accession No. 138461.4. A target site of the extracellular loop 2 of human TM4SF19 used for antibody production is merely exemplary, and the present invention is not limited thereto.

According to an embodiment of the present invention, the TM4SF19 protein was found to be involved in cell-to cell interactions through self-binding.

Specifically, the present inventors produced fusion proteins by fusing antibodies against the TM4SF19 protein capable of inhibiting the self-binding of the TM4SF19 protein, and TM4SF19 fragments (e.g., an extracellular loop region, an extracellular loop region and a membrane protein) and Fc by targeting the extracellular loop region of the TM4SF19 protein, and evaluated the efficacy of the inhibitor against the activity of the TM4SF19 protein.

The term "bone disease" used herein may be one or more selected from the group consisting of a metabolic bone disease, an orthopedic bone disease, aplastic bone disease, degenerative bone disease, degenerative arthritis, rheumatoid arthritis, psoriatic arthritis, psoriatic spondylitis, age-related bone loss, osteoporosis, osteogenesis imperfecta, osteomalacia, sarcopenia, fractures, bone defect and hip arthrosis, rickets, Paget's bone disease, periodontal disease, and bone damage caused by the bone metastasis of cancer cells.

The term "obesity" used herein refers to a condition with abnormal or excessive fat accumulation.

The term "obesity-mediated metabolic disease" used herein includes diabetes, high blood pressure, hyperlipidemia, non-alcoholic steatohepatitis and specific cancers, and more broadly, includes high blood pressure, diabetes, insulin-resistant syndrome, metabolic syndrome, obesity-related gastroesophageal reflux disease, arteriosclerosis, hyperlipidemia, hypertriglyceridemia, hypercholesterolemia, lipodystrophy, non-alcoholic steatohepatitis, cardiovascular disease, and polycystic ovary syndrome. When the composition of the present invention is used, the treatment of the above diseases may also be performed at the same time. In addition, targets for treatment for these obesity-related diseases include one that has a desire to lose weight.

The term "cancer" used herein encompasses cell-mediated diseases having an aggressive characteristic of cells dividing and growing by ignoring normal growth limits, an invasive characteristic of invading into surrounding tissue, and a metastatic characteristic of spreading to other parts of the body.

While there is no limitation to the type of cancer used in the present invention, the cancer may include one or more selected from the group consisting of colorectal cancer, stomach cancer, colon cancer, breast cancer, lung cancer, non-small cell lung cancer, bone cancer, pancreatic cancer, skin cancer, head or neck cancer, melanoma, uterine cancer, ovarian cancer, small intestine cancer, rectal cancer, perianal cancer, fallopian tube carcinoma, endometrial carcinoma, cervical carcinoma, vaginal carcinoma, vulvar carcinoma, esophageal cancer, lymphatic adenocarcinoma, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumors, spinal cord tumors, brainstem glioma, and pituitary adenomas.

The present invention also includes the use of TM4SF19 associated with the inhibition of "cancer" as well as "cancer metastasis."

Meanwhile, the term "prevention" used herein refers to all actions of preventing a desired symptom or disease, or delaying the occurrence or expression thereof by administering the composition of the present invention.

The term "treatment" used herein refers to all actions involved in alleviating or eliminating a desired symptom or disease by administration of the composition of the present invention.

Meanwhile, the composition of the present invention may further include a pharmaceutically acceptable carrier, and may be formulated with a carrier.

The term "pharmaceutically acceptable carrier" used herein refers to a carrier or diluent that does not irritate the organism and does not impair the biological activity and properties of the administered compound. As pharmaceutical carriers available for the composition formulated in a liquid, which are suitable for sterilization and the living body, saline, sterilized water, Ringer's solution, buffered saline, an albumin injectable solution, a dextrose solution, glycerol, ethanol, or a mixture of one or more thereof may be used. As needed, other conventional additives such as an antioxidant, a buffer and a bacteriostat may be added. In addition, the pharmaceutically acceptable carriers may be formulated into an injectable formulation such as an aqueous solution, a suspension or an emulsion, pills, capsules, granules or tablets by further addition of a diluent, a dispersant, a surfactant, a binder and a lubricant.

The composition including the inhibitor of TM4SF19 expression or activity and the pharmaceutically acceptable carrier according to the present invention can be formulated in any dosage form including it as an active ingredient, may be prepared in an oral or parenteral formulation, and may be formulated in a unit dosage form for ease of administration and dosage uniformity. The pharmaceutical formulation of the present invention includes a suitable form for oral, rectal, nasal, topical (including cheek and sublingual administration), subcutaneous, vaginal or parenteral (including intramuscular, subcutaneous and intravenous administration), or for administration by inhalation or insufflation. Formulations for oral administration including the composition of the present invention as an active ingredient may be prepared, for example, in tablets, troches, lozenges, an aqueous or oily suspension, powder or granules, an emulsion, hard or soft capsules, a syrup, or an elixir.

Formulations for parenteral administration including the composition of the present invention as an active ingredient may be prepared in an injectable form for subcutaneous injection, intravenous injection or intramuscular injection, suppository-injectable type, or a spray such as an inhalable aerosol using a respirator. In order to formulate an injectable formulation, the composition of the present invention may be prepared as a solution or suspension by mixing with a stabilizer or buffer in water, and may be formulated for unit administration in ampoules or vials.

The composition of the present invention is administered in a pharmaceutically effective amount, that is, a therapeutically effective amount. In the present invention, the "therapeutically effective amount" refers to an amount sufficient to treat a disease, and the effective dose level may be determined by the type of a patient's disease, severity, the activity of a drug, the sensitivity to a drug, administration time, an administration route, and an excretion rate, treatment duration, concomitant medications, and other factors well known in the medical field. The composition of the present invention may be administered as an individual therapeutic agent or in combination with other therapeutic agents, and may be sequentially or simultaneously administered with a conventional therapeutic agent, or may be administered at a single or multiple doses. That is, the total effective amount of the composition of the present invention may be administered into a patient with a single dose, or with multiple doses by a fractionated treatment protocol administered for a long period of time. In consideration of all of the above factors, it is important to administer an amount that can obtain the maximum effect with a minimum amount without side effects, which can be easily determined by those of ordinary skill in the art.

A dose of the pharmaceutical composition of the present invention may vary according to a patient's weight, age and sex, a health condition, a diet, administration time, an administration method, an excretion rate and the severity of a disease. In parenteral administration, a daily dose is preferably 0.01 µg to 100 mg, and more preferably 1 µg to 50 mg per kg of a weight. However, since the dose may be increased or decreased depending on an administration route, the severity of obesity, sex, a body weight or age, the above-mentioned dose does not limit the scope of the present invention in any way.

The composition of the present invention can be used alone or in combination with methods using surgery, radiation therapy, hormone therapy, chemotherapy, and a biological reaction modifier.

In another aspect, the present invention relates to a method of preventing or treating a bone disease, which includes administering a therapeutically effective amount of a composition for preventing or treating a bone disease, including a material for inhibiting TM4SF19 expression or activity into a subject.

In still another aspect, the present invention relates to a method of preventing or treating obesity or an obesity-mediated metabolic disease, which includes administering a therapeutically effective amount of a composition for preventing or treating obesity or an obesity-mediated metabolic disease, including an inhibitor of TM4SF19 expression or activity as an active ingredient into a subject.

In yet another aspect, the present invention relates to a method of preventing or treating cancer or cancer metastasis, which includes administering a therapeutically effective amount of a composition for preventing or treating cancer or inhibiting cancer metastasis, which includes an inhibitor of TM4SF19 expression or activity as an active ingredient, into a subject.

The term "administration" used herein refers to introduction of a predetermined material into an individual by an appropriate method, and the administration route of the preventive or therapeutic composition according to the present invention may be orally or parenterally administered via a general route that can reach desired tissue. In addition, the composition for preventing or treating a cartilage-related disease according to the present invention may be administered by any device capable of moving the active ingredient to a target cell.

The term "subject" used herein includes a mammal such as a mouse, a rat, a rabbit, a cow, a horse, a pig, goat, a camel, an antelope or a dog, or a human, which has a related disease alleviating a symptom by the administration of a pharmaceutical composition according to the present invention.

The administration route of the composition of the present invention may be administered by a conventional method via various oral or parenteral routes that can reach desired tissue, specifically, oral, rectal, local, intravenous, intraperitoneal, intramuscular, intraarterial, transdermal, intranasal, inhalational, intraocular or intradermal routes.

The prevention or treatment method of the present invention includes administrating the composition of the present invention at a therapeutically effective amount. The therapeutically effective amount refers to an amount that increases the effect of reducing a weight or size of adipocytes. It is apparent to those of ordinary skill in the art that a suitable total daily amount can be determined by a physician within the scope of correct medical judgement. Preferably, a specific therapeutic effective amount for a particular patient depends on the type and extent of a reaction to be achieved, whether or not another agent is used when needed, a patient's age, weight, general health condition, sex and diet, administration time, administration route and a secretion rate of the composition, a treatment duration, and various factors and other factors well known in the medical field. Accordingly, the effective amount of the pharmaceutical composition suitable for the purpose of the present invention is preferably determined in consideration of the above-described contents. In addition, in some cases, by co-administration of the composition of the present invention with a therapeutic agent for a known related disease, a therapeutic effect may be improved.

In yet another aspect, the present invention relates to a method of screening a drug for treating a bone disease, which includes: treating a specimen suspected of a bone disease with a candidate for treating a bone disease; and
comparing the mRNA or protein expression level of a TM4SF19 gene with a control.

In yet another aspect, the present invention relates to a method of screening a drug for treating obesity or an obesity-mediated metabolic disease, which includes: treating a specimen suspected of obesity or an obesity-mediated metabolic disease with a candidate for treating obesity or an obesity-mediated metabolic disease; and
comparing the mRNA or protein expression level of a TM4SF19 gene with a control.

In yet another aspect, the present invention relates to a method of screening a drug for treating cancer or cancer metastasis, which includes:
treating a specimen suspected of cancer or cancer metastasis with a candidate for preventing or treating cancer or inhibiting cancer metastasis; and
comparing the mRNA or protein expression level of a TM4SF19 gene with a control.

The term "specimen" used herein refers to an individual or sample used to screen a candidate for treating a related disease, and includes mammals including a dog, a cow, a pig, a rabbit, a chicken, a mouse, and a human without limitation, and includes a sample such as whole blood, serum, blood, plasma, saliva, urine, sputum, lymphatic fluid, cells or tissue, isolated from the individual.

The term "control" used herein refers to a specimen which is not treated with a candidate. In addition, the term "candidate" used herein refers to a drug for testing a change in the expression or activity of TM4SF19, and a target for measuring an ability of preventing or treating a related disease by directly or indirectly changing the expression level or activity of TM4SF19, and includes any molecules, for example, a protein, an oligopeptide, a small organic molecule, a polysaccharide, a polynucleotide, and a wide range of compounds. These candidates also include a natural material as well as a synthetic material.

The term "therapeutic drug" used herein refers to a material for preventing or treating a related disease.

The screening method of the present invention may be performed by determining a material that reduces the mRNA or protein expression of the TM4SF19 gene as a therapeutic agent for a related disease, compared with a control, by treating a related disease-suspected subject with a candidate for treatment and comparing the mRNA or protein expression level of the TM4SF19 gene with the control that is not treated with a candidate. Analysis methods for measuring an mRNA level include reverse transcriptase polymerase reaction, competitive reverse transcriptase polymerase reaction, real-time reverse transcriptase polymerase reaction, RNase protection assay, Northern blotting, a DNA chip, but the present invention is not limited thereto.

Analysis methods for measuring a protein level include Western blotting, ELISA, radioimmunoassay, radial immunodiffusion, Ouchterlony immunodiffusion, rocket immunoelectrophoresis, immunohistochemistry, immunoprecipitation assay, complement fixation assay, FACS, and protein chip assay, but the present invention is not limited thereto.

In addition, according to one embodiment of the present invention, it was confirmed that TM4SF19 expression was increased during osteoclast differentiation. In the case of TM4SF19-deficient models (TM4SF19 knock-out mouse, TM4SF19KO and TM4SF19 extracellular domain 2 (116-165) knock-out mouse, TM4SF19EC2^{△}), it was confirmed that mononuclear osteoclasts are formed in osteoclast differentiation, whereas multinucleated osteoclast formation is inhibited. Osteoclasts that are not differentiated into multinucleated osteoclasts have phenotypes as osteoclasts and retain a low level of bone resorption activity. That is, the TM4SF19 inhibitor of the present invention, which can selectively inhibit multinucleated osteoclast formation, can more selectively control bone resorption, not completely abrogating the bone resorption function, significantly lowers side effects compared to a therapeutic agent that inhibits formation of mononuclear osteoclasts such as an anti-RANKL antibody. In addition, it was confirmed that the TM4SF19-deficient model prevents bone loss caused by an ovariectomy. In addition, as a result of treating an anti-mouse TM4SF19 polyclonal antibody and mouse TM4SF19 EC2 (116-165 of GenBank Accession No. NP_001153874.1)-Fc fusion protein, human TM4SF19 EC2 (120-169 of GenBank Accession No. NP_612470.2 (isoform 1))-Fc fusion protein in osteoclast differentiation, it was confirmed that these inhibitors inhibited multinucleated osteoclast formation during the osteoclast differentiation. Accordingly, a material for inhibiting TM4SF19 expression or a material for inhibiting TM4SF19 activity are effective in prevention or treatment of a bone disease.

In addition, according to one embodiment of the present invention, TM4SF19 expression was increased during adipocyte differentiation of human adipose-derived mesenchymal stem cells (hADMSCs). It was confirmed that a weight increase is inhibited in a high-fat diet-induced model in TM4SF19 knock-out mice, and the amount of adipose tissue-related macrophages due to obesity is reduced, confirming that TM4SF19 acts on adipocyte differentiation and an inflammatory response including macrophages. As a result, it was confirmed that a TM4SF19KO mouse lowers insulin resistance and inhibits fatty liver in the high-fat diet-induced obese mouse model. Therefore, it can be seen that substances that inhibit TM4SF19 expression or activity are effective in preventing or treating obesity and obesity-mediated metabolic diseases.

In addition, according to one embodiment of the present invention, it was confirmed that TM4SF19 overexpression increases mouse-derived breast cancer and proliferation, migration and colony formation of human osteosarcoma cells, and TM4SF19 deficiency is effective in inhibition of lung metastasis of breast cancer cell, inhibition of the cell proliferation, migration and colony formation of osteosarcoma, and inhibition of bone metastasis of breast cancer cells, and a material inhibiting TM4SF19 expression or activity is effective in prevention and treatment of cancer or cancer metastasis.

In addition, according to one embodiment of the present invention, it was confirmed that the treatment of a human TM4SF19 EC2 (120-169 of GenBank Accession No. NP_612470.2 (isoform 1))-Fc fusion protein can inhibit the growth and colonization ability of pancreatic cancer cells, and inhibit the cell proliferation, migration and colony formation of osteosarcoma cells. Therefore, it can be seen that the material for inhibiting TM4SF19 expression or TM4SF19 activity is effective in prevention and treatment of cancer or cancer metastasis.

Hereinafter, the advantages and features of the present invention and the methods of accomplishing the same may be clearly understood with reference to the detailed description of exemplary embodiments and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art.

### [Examples]

### Example 1: Role of TM4SF19 in osteoclast differentiation

FIG. 1 shows the mRNA expression of TM4SF19 over time, confirmed by qPCR, after cells isolated from mouse bone marrow are treated with M-CSF and receptor activator of nuclear factor kappa-B ligand (RANKL) to induce osteoclast (OC) differentiation. As shown in FIG. 1, it was confirmed that TM4SF19 expression increased according to the progression of osteoclast differentiation. The expression of ACP5 and CTSK, which are osteoclast differentiation-related genes, increased according to the progression of osteoclast differentiation.

### Example 2: Confirmation of role of TM4SF19 using TM4SF19 knock-out mice

### (1) Confirmation of TM4SF19 knock-out and inhibition of multinucleated osteoclast formation

Cells were isolated from the bone marrow of CRISPR-induced TM4SF19 knock-out mice TM4SF19KO and wild-type (WT) mice and treated with M-CSF (25 ng/ml) + RANKL (100 ng/ml) to induce differentiation, followed by staining with tartrate-resistant acid phosphatase (TRAP), which is an osteoclast-related marker.

As shown in FIG. 2A, when TM4SF19KO mouse bone marrow cells were induced to differentiate into osteoclasts, they differentiated into mononuclear osteoclasts, not into multinucleated osteoclasts.

In the same manner as described above, cells were isolated from the bone marrow of CRISPR-induced TM4SF19 knock-out mice TM4SF19KO and wild-type (WT) mice, a RANKL concentration was fixed at 100 ng/ml, and the cells were induced to differentiate at various M-CSF concentrations of 25, 40, and 60 ng/ml, followed by staining with TRAP.

As shown in FIG. 2B, it was confirmed that, TM4SF19KO mouse bone marrow cells failed to form multinucleated osteoclast even by the treatment with a high concentration of M-CSF.

FIG. 2C is a schematic diagram illustrating the process of osteoclast differentiation. As shown in FIG. 2C, an osteoclast precursor was first differentiated into TRAP-positive mononuclear osteoclasts and then maturated to giant multinucleated cells through cell-to-cell fusion and incomplete cytokinesis. Multinucleated osteoclasts have high bone resorption activity, whereas osteoclasts that do not differentiate into multinucleated osteoclasts have their own phenotype and express osteoclast-related markers such as TRAP and cathepsin K, but maintain a low level of bone resorption activity.

Accordingly, the fact that TM4SF19 knock-out prevents the multinucleated osteoclasts formation suggest that the prevention or treatment of a bone loss-related disease can be attempted through the inhibition of TM4SF19. Particularly, a TM4SF19 inhibitor can selectively inhibit multinucleated osteoclast formation, and more selectively regulate bone resorption rather than completely abrogating the bone resorption function of osteoclasts, thereby significantly reducing side effects compared to therapeutic agents that inhibit the formation of mononuclear osteoclasts such as an anti-RANKL antibody.

### (2) Confirmation of inhibition of expression of gene involved in osteoclast differentiation in TM4SF19KO mice

Four days after cells isolated from the bone marrow of a CRISPR-mediated TM4SF19 knock-out mouse TM4SF19KO and a wild-type (WT) mouse and differentiated under the presence or absence of M-CSF and RANKL treatment, the expression of Ctsk, Acp5, c-Fos, and Nfatc1 involved in osteoclast differentiation was confirmed by qPCR.

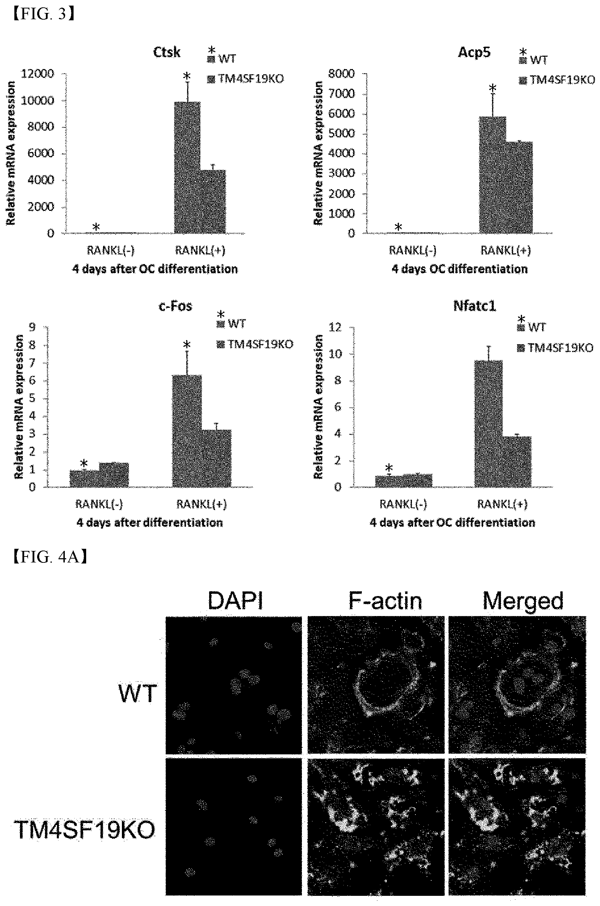

FIG. 3 shows a set of graphs illustrating the mRNA expression of Ctsk, Acp5, c-Fos, and Nfatc1 in osteoclast differentiation under the presence or absence of M-CSF and RANKL treatment.

As shown in FIG. 3, the expression of osteoclast differentiationinvolved genes was significantly inhibited in TM4SF19KO compared to the control (WT) during osteoclastogenesis.

### (3) Confirmation of actin belt formation and inhibition of bone resorption

After differentiation of the bone marrow of the wild-type (WT) and the TM4SF19KO mouse, actin belt formation was confirmed through F-actin staining. While the wild-type (WT) formed a normal actin belt, TM4SF19KO did not form an actin belt (FIG. 4A).

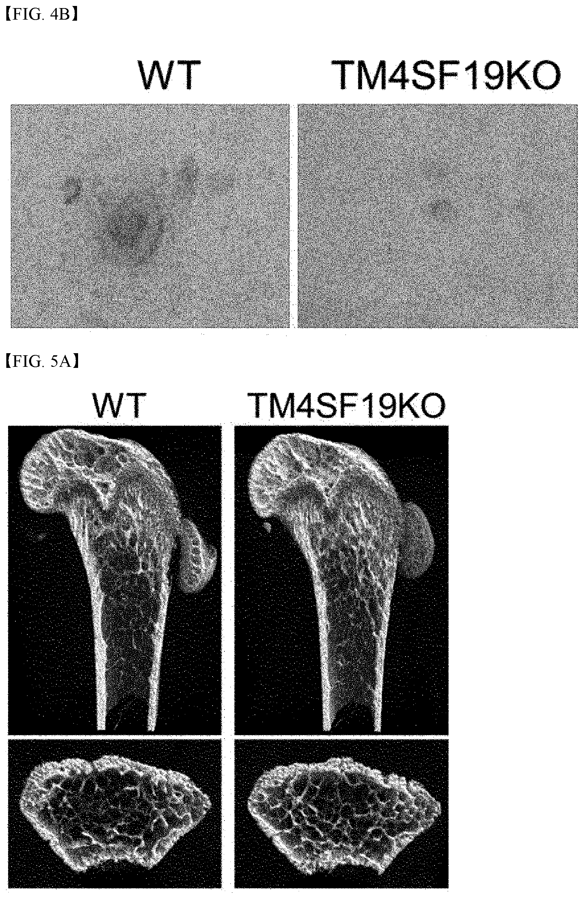

After BMM cells were plated on a Dentin disc and differentiated, pit formation was confirmed. Although bone resorption was confirmed in the wild-type (WT), it was confirmed that bone resorption was suppressed in TM4SF19KO (FIG. 4B).

### (4) Confirmation of bone loss inhibition by OVX

After 8-week-old wild-type (WT) and TM4SF19KO female mice were dissected without ovary removal (sham) or ovariectomized (OVX) and then femurs were fixed on day 31, micro-CT analysis was carried out.

In addition, 3D micro-CT parameters (bone microstructure parameters) including a bone volume (BV), a ratio of bone volume to tissue volume (% BV/TV), an average trabecular number (Tb.N) and a trabecular separation (Tb.Sp) were obtained by analyzing the micro-CT images using a 3D imaging program.

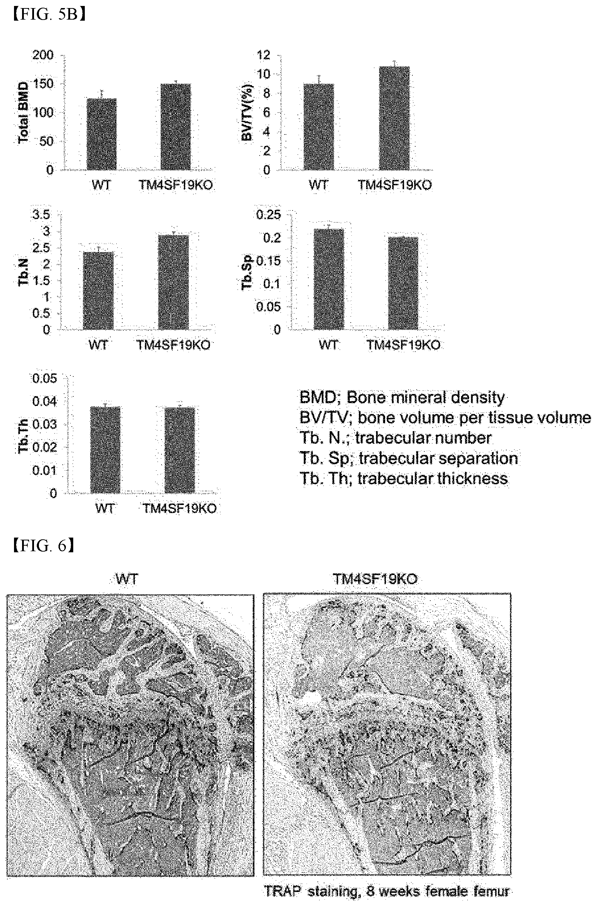

It was confirmed that bone loss caused by the ovariectomy was inhibited in the TM4SF19KO mouse (FIG. 5).

Regardless of the ovariectomy, the bone density of the TM4SF19KO mouse is higher than that of the wild-type (WT) mouse.

### (5) Confirmation of osteoclast formation through femur staining with TRAP

Femurs of the 8-week-old wild-type (WT) and TM4SF19KO mice were fixed and decalcified, and then stained with TRAP.

As a result, as shown in FIG. 6, a lack of multinucleated osteoclast was confirmed through TRAP staining of the femur of the TM4SF 19KO mouse.

### Example 3-1: Confirmation of defect to multinucleated osteoclast formation in TM4SF19 extracellular loop 2 (EC2)-deficient mice

### (1) Confirmation of defect of osteoclast multinucleation

After cells were isolated from the bone marrow of a wild-type (WT) mouse and a TM4SF19EC2 Del (also represented as TM4SF19EC2Δ) mouse in which M4SF19 extracellular domain 2 (116-165) was knocked out using CRISPR and differentiated with or without treatment of M-CSF (25 ng/ml) and RANKL (100 ng/ml), TRAP staining was carried out.

As a result, as shown in FIG. 7A, it was confirmed that, when the BMMs of the TM4SF19EC2Δ mouse was able to be differentiated into mononuclear osteoclasts, but could not differentiate into multinucleated osteoclasts.

FIG. 7B is a TRAP staining result obtained from the BMMs of a wild-type (WT) mouse and a TM4SF19EC2Δ mouse in which M4SF19 extracellular domain 2 (116-165) was knocked out using CRISPR, the RANKL concentration was fixed at 100 ng/ml, and the cells were treated with 25 ng/ml or 100 ng/ml of M-CSF to induce differentiation. TM4SF19EC2Δ osteoclasts could not rescue defect of multinucleated osteoclast formation even by a high concentration of M-CSF treatment.

### Example 3-2: Cytoskeletal rearrangement by TM4SF19EC2Δ

### (1) Confirmation of actin belt formation and inhibition of bone resorption

When multinucleated osteoclasts (mature osteoclasts) are formed during osteoclast differentiation, an actin belt consisting of podosomes is formed and adhered to the bone, resulting in bone resorption.

After cells were isolated from the bone marrow of the wild-type and TM4SF19EC2Δ mice and treated with MCSF (60 ng/ml) and RANKL (100 ng/ml) to induce differentiation, and then the differentiated cells were fixed and stained with phalloidin FITC, an F-actin belt was examined using a confocal microscope (400x).

It was confirmed that TM4SF19EC2Δ inhibits multinucleated osteoclast formation and affects integrin signal-related cytoskeletal rearrangement. As shown in FIG. 7C, an actin belt formation in the wild-type mouse through osteoclast multinucleationbut not formed in TM4SF19EC2Δ.

After differentiation by plating BMM cells on a Dentin disc, pit formation was confirmed by 1% toluidine blue staining. As a result, although bone resorption was confirmed in the wild-type (WT) mouse, bone resorption was blocked in TM4SF19EC2Δ (FIG. 7D).

### (2) Confirmation of inhibition of osteoclast-involved gene expression

To confirm the expression of osteoclast differentiation-associated target genes (Ctsk, Acp5, c-Fos, and Nfatc1) in a wild-type (WT) mouse and a TM4SF19EC2Δ mouse in which TM4SF19 extracellular domain 2 (116-165) was knocked out using CRISPR, qPCR was carried out.

As shown in FIG. 8, the expression of osteoclast differentiationinvolved genes (Ctsk, Acp5, c-Fos and Nfatc1) was significantly inhibited during osteoclast differentiation in the TM4SF 19EC2Δ mouse, compared to the control.

### (3) Confirmation of inhibition of bone loss

After 8-week-old wild-type (WT) and TM4SF19EC2Δ female mice were dissected without ovary removal (sham) or ovariectomized (OVX) and then femurs were fixed on day 31, micro-CT analysis was carried out.

In addition, 3D micro-CT parameters (bone microstructure parameters) including a bone volume (BV), a ratio of bone volume to tissue volume (% BV/TV), an average trabecular number (Tb.N) and a trabecular separation (Tb.Sp) were obtained by analyzing the micro-CT images using a 3D imaging program.

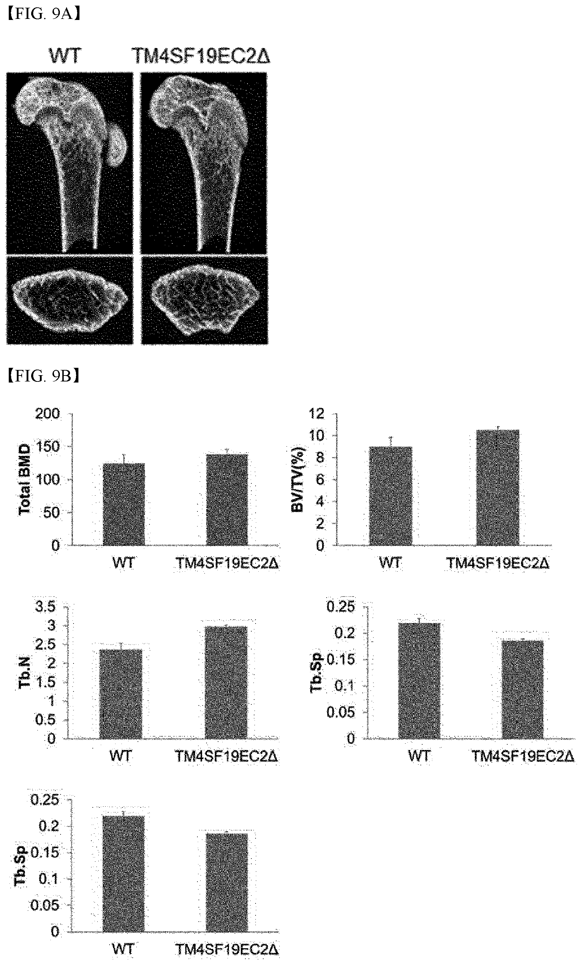

It was confirmed that bone loss caused by the ovariectomy was inhibited in the TM4SF19EC2Δ mouse (FIG. 9).

After 8-week-old wild-type (WT) and TM4SF19KO and TM4SF19EC2Δ female mice were dissected without ovary removal (sham) or ovariectomized (OVX) and then femurs were fixed on day 31, micro-CT (µCT) analysis was carried out.

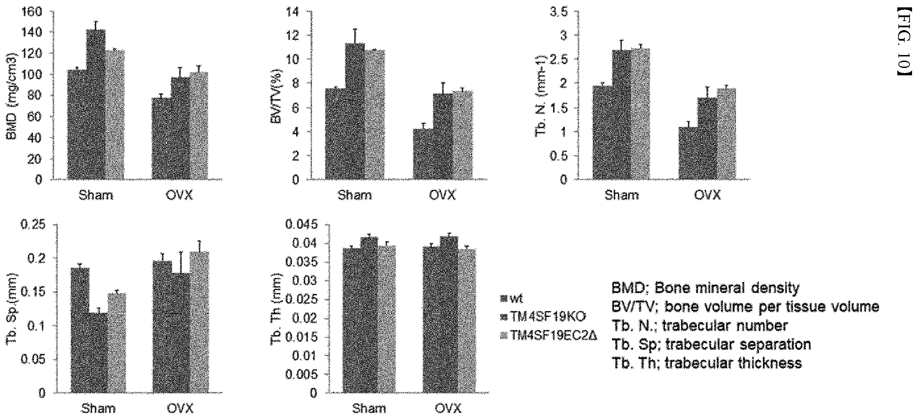

FIG. 10 shows 3D micro-CT parameters (bone microstructure parameters) including a bone volume (BV), a ratio of bone volume to tissue volume (% BV/TV), an average trabecular number (Tb.N), a trabecular separation (Tb.Sp), and a trabecular thickness (Tb.Th) obtained by analyzing the micro-CT images using a 3D imaging program.

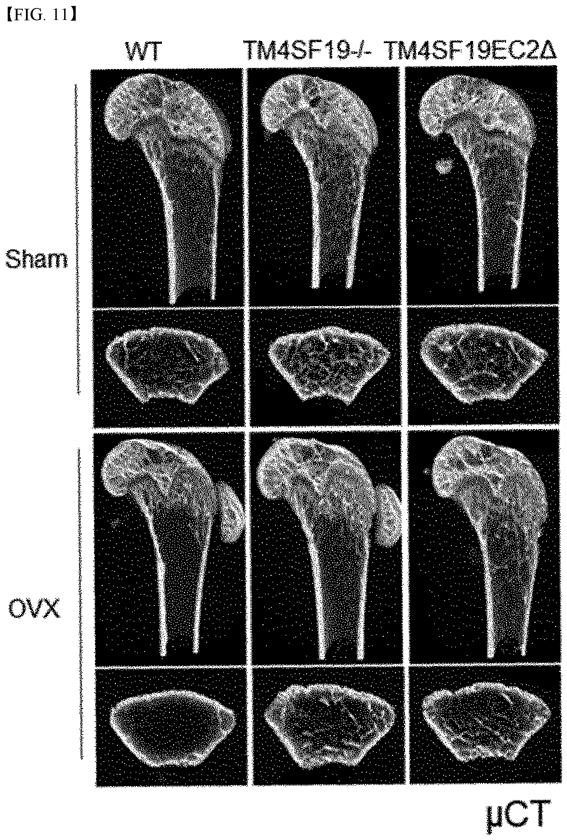

FIG. 11 shows micro-CT images.

As a result, as shown in FIGS. 10 and 11, it was confirmed that bone loss induced by an ovariectomy was inhibited in the TM4SF19KO and TM4SF19EC2Δ mice, and in the sham group in which an ovariectomy (OVX) was not performed, bone densities in the TM4SF19KO and TM4SF19EC2Δ mice was higher than that of the wild-type (WT).

### Example 4: Confirmation of self-binding of TM4SF19 and interaction with integrin

### (1) Confirmation of TM4SF19-to-TM4SF19 self-interaction

To confirm whether TM4SF19 is involved in cell-to-cell interactions, HA- or flag-tagged TM4SF19 was manufactured. TM4SF19 in which 3HA was tagged at the N-terminus, a wild-type in which 3Flag was tagged at the N-terminus, and a TM4SF19-deletion mutant were subjected to transient overexpression in 293T cells to confirm binding through immunoprecipitation. The TM4SF19 deletion mutant was manufactured as shown in FIG. 12A. After 3Flag was tagged to each of hTm4sf19 variants in which a part of the TM4SF19 sequence was deleted (hTM4SF19 115-175Δ, hTm4sf19 105-186Δ, hTm4sf19 105-196Δ, hTm4sf19 94-186Δ and hTm4sf19 94-196Δ) shown in FIG. 12A, cell-to-cell interaction was analyzed by immunoprecipitation.

As a result, it was confirmed that TM4SF19 is involved in cell-to-cell interactions and binds to itself (FIG. 12B). However, it was confirmed that the mutant in which TM4SF19 transmembrane 3 (TM3), extracellular region 2 (EC2) and transmembrane 4 (TM4) are deleted (hTm4sf19 94-196Δ) did not undergo self-binding. From the above result, it was confirmed that the transmembrane 3-extracellular region 2-trasmembrane 4 region of TM4SF19 is important for the self-interaction of TM4SF19, and aa 94 to 196 of TM4SF19 is particularly a region important for the self-interaction between TM4SF19 and TM4SF19.

### (2) Confirmation of region involved in TM4SF19-to-TM4SF19 self-interaction

To verify that aa 94-196 of TM4SF19 is an important region for self-interaction between TM4SF19-TM4SF19, at this time, unlike 3Flag-hTm4sf19 94-196Δ, hTm4sf19 94-196 in which only the sequence of aa 94-196 of hTm4sf19 remains and other sequences are deleted was produced as a TM4SF19 deletion mutant (FIG. 12C, top). In the same manner as described above, by the transient overexpression of TM4SF19 in which 3HA was tagged at the N-terminus, a wild-type in which 3Flag was tagged at the N-terminus, and a TM4SF19 deletion mutant in 293T cells, binding was confirmed through immunoprecipitation. As a result, it was confirmed that the mutant (hTm4sf19 94-196) which expressed transmembrane 3, extracellular region 2 (EC2), and transmembrane 4 of TM4SF19 self-binds similar to the wild-type (WT) (FIG. 12C, bottom). From these results, it was reconfirmed that the aa 94-196 region of TM4SF19 is a very important part for the self-interaction between TM4SF19-TM4SF19.

### (3) Interaction between TM4SF19 and TM4SF19-Fc

The interaction between TM4SF19 tagged with 3Flag at the N-terminus and a hIgG1-Fc fusion protein of a TM4SF19 fragment in 293T cells was confirmed through immunoprecipitation in the same manner as described above. As the TM4SF19 fragment, a hTm4sf19 131-160, hTm4sf19 145-169, hTm4sf19 131-169, hTm4sf19 120-160, hTm4sf19 120-169, hTm4sf19 120-180, hTm4sf19 120-186, hTm4sf19 120-196, hTm4sf19 120-209, hTm4sf19 131-196, or hTm4sf19 145-196 region was used (FIG. 12D, top). As a result, as shown in the bottom of FIG. 12D and FIG. 12E, it was confirmed that the aa 160-169 region of hTm4sf19 plays an important role in binding, and it can also be confirmed that the aa 145-196 region of hTM4SF19-Fc plays an important role in increasing binding to TM4SF19.

### (4) Confirmation of interaction between integrin αv-TM4SF19

The interaction between TM4SF19 and integrin αv, playing an important role in osteoclast differentiation was confirmed. To confirm an important binding region in which TM4SF19-Fc binds to integrin αv-TM4SF19, the interaction between integrin αv tagged with 3HA at the C-terminus and a hIgG1-Fc fusion protein of a TM4SF19 fragment in 293T cells was confirmed through immunoprecipitation (FIG. 12F).

### (5) Confirmation of interaction between integrin β3-TM4SF19

The interaction between TM4SF19 and integrin β3, playing an important role in osteoclast differentiation, was confirmed. To confirm the important binding region in which TM4SF19-Fc binds to integrin β3-TM4SF19, the interaction between the integrin β3 tagged with 3HA at the C-terminus and the hIgG1-Fc fusion protein of the TM4SF19 fragment in 293T cells was confirmed through immunoprecipitation (FIG. 12G).

FIG. 12H is a result of confirming the interaction after overexpressing TM4SF19 and integrin αv or integrin β3, which regulates osteoclast functions and involves in cytoskeletal rearrangement during multinucleated osteoclast formation, in 293T cells or osteoclast precursor Raw 264.7 cells. This is a result confirming that TM4SF19 binds to integrin αv and also binds to integrin β3.

TM4SF19 is a member of the tetraspanin family, it has been known that the extracellular region 2 (large extracellular loop) of this family plays an important role in binding to another binding partner. To confirm whether the extracellular region of TM4SF19 is essential for interacting with a partner, the interaction between TM4SF19 wt or a protein in which extracellular region 2(aa 115-175), is deleted, and integrin αv or integrin β3 was confirmed through immunoprecipitation. A result confirmed that TM4SF19 wt shows an interaction therewith, but the binding to hTM4SF19 115-175Δ is not formed.

FIG. 12I shows the interaction between TM4SF19, and DC-Stamp and siglec-15, which are membrane proteins involved in the regulation of osteoclast functions and cytoskeletal rearrangement during multinucleated osteoclast formation, confirmed by immunoprecipitation. This is an immunoprecipitation result confirming an interaction TM4SF19 tagged with 3HA in N-terminus, DC-Stamp tagged with 3Flag in N-terminus, or Siglec-15 tagged with 3Flag in C-terminus, in 293T cells.

### Example 5: Confirmation of increase in cancer cell proliferation, colony formation, and migration by TM4SF19 overexpression

After the overexpression (hTM4SF19) of TM4SF19 in murine breast cancer cells E0771, a cell proliferation and colony formation assay and a cell migration assay over time were carried out.

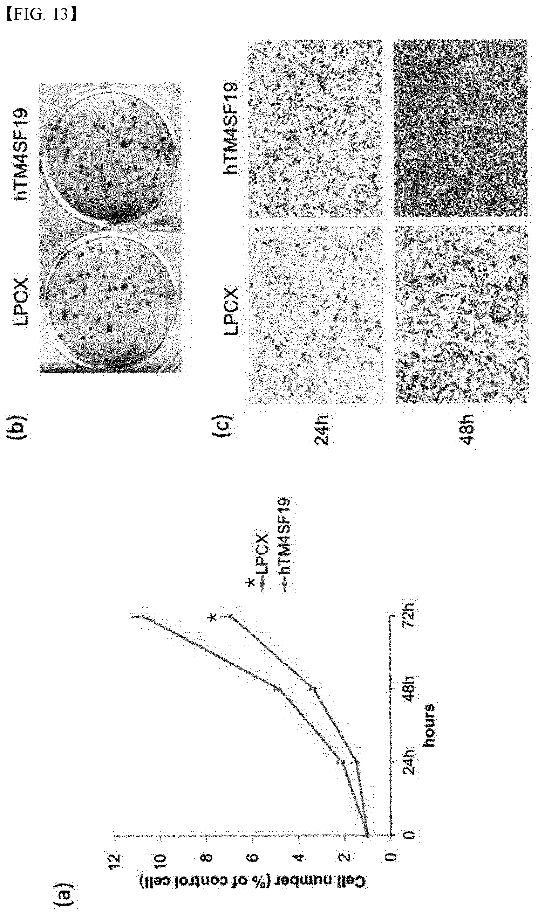

FIG. 13A is a set of graphs showing that the cell proliferation of the breast cancer cells E0771 significantly increases after 24, 48 or 72 hours through TM4SF19 overexpression. FIG. 13B shows that colony formation ability was confirmed after plating 1000 cells each of TM4SF19-overexpressing breast cancer cells E0771 and control cells, and shows that colony formation was increased by TM4SF19 overexpression. FIG. 13C shows cell migration ability over time, evaluated after TM4SF19-overexpressing breast cancer cells E0771 cells and control cells are plated in a migration chamber, indicating that cell migration is increased by TM4SF19 overexpression.

### Example 6: Confirmation of inhibition of cancer cell metastasis by TM4SF19 deficiency

### (1) Confirmation of inhibition of metastasis of breast cancer cells to lungs by TM4SF19 deficiency

After wild-type (WT), TM4SF19KO, and TM4SF19EC2Δ mice were sacrificed 12 days after injecting breast cancer cells E0771 into the tail veins, the lungs were stained with Indian ink to confirm lung metastasis.

Lung metastasis phenotypes of the breast cancer cells of the wild-type (WT), TM4SF19KO, and TM4SF19EC2Δ mice were confirmed, and the number of nodules generated in the lungs was counted

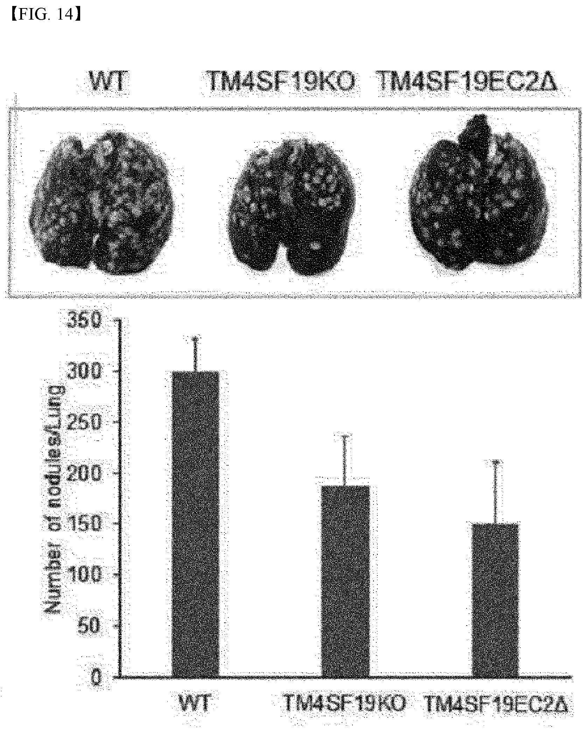

As a result, it was confirmed that the TM4SF19KO and TM4SF19EC2Δ mice significantly inhibit lung metastasis of breast cancer cells, compared to the wild-type (WT) mice (FIG. 14).

### (2) Confirmation of inhibition of expression of breast cancer cell metastasis-associated genes and proteins by TM4SF19 deficiency

Environments around cancer cells act as important factors for cancer metastasis, and among these factors, the degree of metastasis may change due to an anti-tumor factor released from macrophages. For co-culture, bone marrow was obtained from the TM4SF19KO mouse (TMKO) and the wild-type (WT) to differentiate into bone marrow-derived macrophages (BMDM), and put into the lower part of a migration chamber, and breast cancer cells were put into the upper part of the chamber. The expression of target genes (CDH2, SNAI1, and SNAI2) involved in cell migration in the migrated cells was confirmed by qPCR, and western blotting was carried out to confirm the expression of metastasis-associated proteins (vimentin, slug, bnail, E-cadherin, and β-actin).

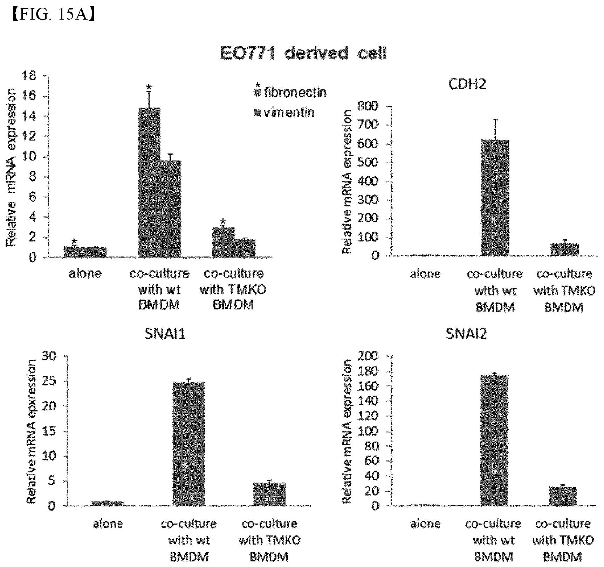

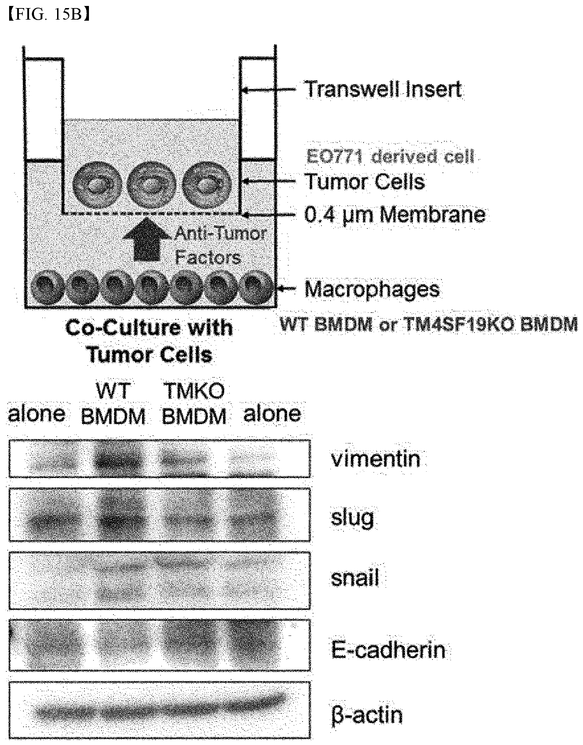

As a result, it was confirmed that, by the co-culture with TM4SF19KO mouse macrophages, the expression of target genes (CDH2, SNAI1, and SNAI2) involved in cell migration was inhibited (FIG. 15A), and the expression of metastasis-related proteins (vimentin, slug, bnail, E-cadherin, and β-actin) was inhibited (FIG. 15B). This shows that the expression of cancer cell metastasis-related genes and proteins was inhibited by TM4SF19 deficiency.

### Example 7: Confirmation of involvement of TM4SF19 in adipogenic differentiation of human ADMSCs

On day 0, day 14 and day 21 after human adipose-derived mesenchymal stem cells (hADMSCs) were grown in DMEM/F12 supplemented with 10 µg/ml insulin, 1 nM 3,3', 5-triiodo-L-thyronine, 1 µM dexamethasone, and 1 µM rosiglitazone, differentiated into adipocytes, the expression of genes (C/EBPα and PPARγ) was confirmed by qPCR, and the expression of proteins (PPARγ and FABP4) was confirmed by western blotting.

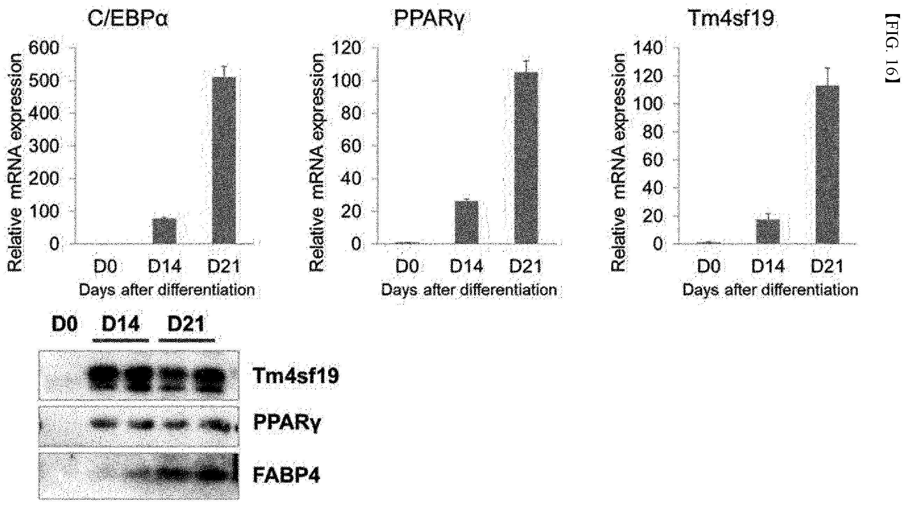

As a result, it was confirmed that the expression level of TM4SF19 is increased by adipogenic differentiation of human ADMSCs (FIG. 16).

### Example 8: Confirmation of the role of TM4SF19 in obesity and metabolic disease-related disease

### (1) Weight gain was confirmed that high-fat fed TM4SF19KO

6-week-old wild-type (WT) and TM4SF19KO mice, which had been fed a normal diet, were fed a high-fat diet (60% fat) for 16 weeks, and weight gain was confirmed.

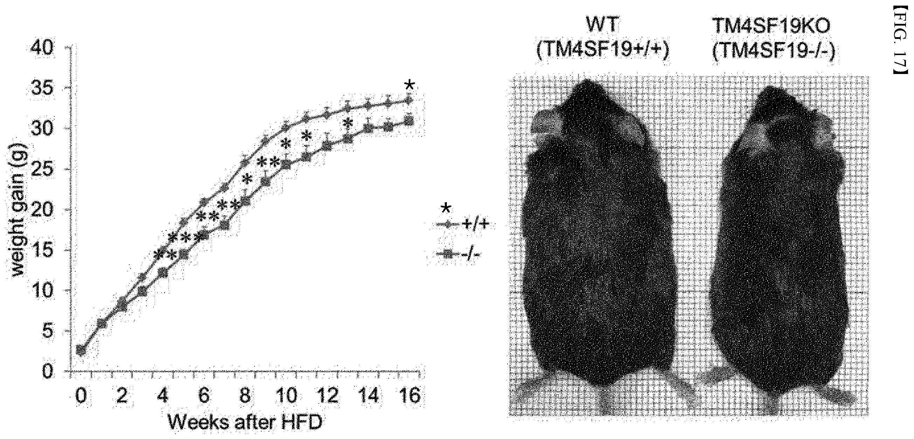

As a result, the TM4SF19KO mouse was resistant to high-fat diet induced obesity (FIG. 17).

### (2) Confirmation of insulin resistance and fatty liver

After wild-type (WT) and TM4SF19KO mice were fed a high-fat diet, insulin resistance was confirmed through HOMA-IR (A), liver phenotypes were confirmed and liver tissue was fixed and stained with H&E (B), and triglyceride levels in the liver were measured (C).

As a result, in the case of TM4SF19KO mice, insulin resistance was lowered and fatty liver formation was inhibited in a high-fat diet-induced obese mouse model. Therefore, it was confirmed that the TM4SF19KO mice (prepared by feeding 6-week-old mice, which had been fed a normal diet, a high-fat diet for 12 weeks) were resistant to high-fat diet-induced insulin resistance and fatty liver (FIG. 18).

### (3) Confirmation of resistance to high fat diet-induced obesity

While 6-week-old wild-type (WT) and TM4SF19KO mice that had been fed a normal diet were fed a high-fat diet for 18 weeks, their weights were increased (FIG. 19A), after 18 weeks, the mice were sacrificed to confirm the weight of each tissue (FIG. 19B), the weights of subcutaneous fat and visceral fat were confirmed (FIG. 19C), a fat phenotype and adipose tissue macrophages around the fat were confirmed by H&E staining of epididymal white adipose tissue (eWAT) (FIG. 19D), and the secretion of adiponectin, which is an anti-obesity cytokine, in serum was also confirmed (FIG. 19E) [rtWAT: retroperitoneal white adipose tissue, sWAT: subcutaneous white adipose tissue, ingWAT: inguinal white adipose tissue, iWAT: interscapular white adipose tissue, iBAT: interscapular brown adipose tissue].

As a result, it was confirmed that, in the case of the TM4SF19KO mice, obesity was inhibited in a high-fat diet-induced obese mouse model, and an adiponectin secretion amount in serum was also increased. Therefore, it was confirmed that the TM4SF19KO mice are resistant to high fat diet-induced obesity (18 weeks) (FIG. 19).

### (4) Confirmation of expression of adipogenic differentiation marker and macrophage /M1-like macrophage marker

After 6-week-old wild-type (WT) and TM4SF19KO mice that had been fed a normal diet were fed a high-fat diet for 12 weeks, the expression of adipogenic differentiation markers (top) and macrophage/M1-like macrophage markers (bottom) in epididymal white adipose tissue (eWAT) and subcutaneous white adipose tissue (sWAT) was confirmed by qPCR.

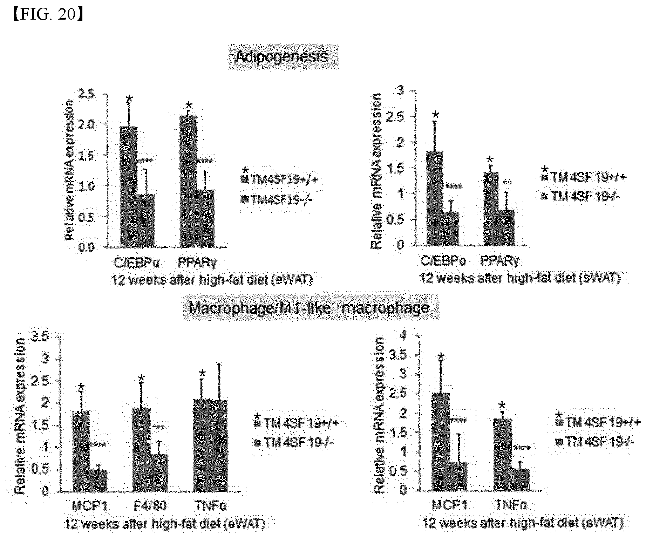

As a result, despite a high-fat diet, the expression of an adipogenic differentiation marker and a macrophage/M1-like macrophage marker was reduced in epididymal and subcutaneous white adipose of the TM4SF19KO mice (FIG. 20).

### (5) Confirmation of population of adipose tissue-related macrophages

After 6-week-old wild-type (WT) and TM4SF19KO mice that had been fed a normal diet were fed a high-fat diet for 12 weeks, % of macrophages (A) and dendritic cells (B) was confirmed through FACS analysis of epididymal white adipose tissue (eWAT).

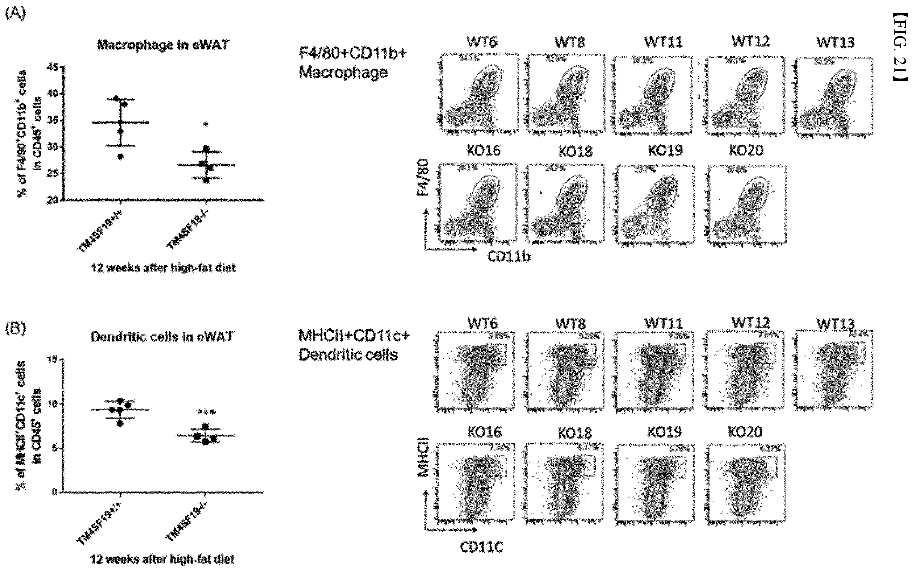

As a result, despite a high-fat diet, % macrophages and dendritic cells in epididymal white fat of the TM4SF19KO mouse were reduced, and the amount of obesity-induced adipose tissue-associated macrophages was reduced in TM4SF19KO (FIG. 21).

### (6) Expression of marker gene in stromal vascular fraction of white adipose tissue

After 6-week-old wild-type (WT) and TM4SF19KO mice that had been fed a normal diet were fed a high-fat diet for 12 weeks, to confirm the expression of a macrophage marker gene in a stromal vascular fraction (SVF), qPCR was performed on epididymal white adipose tissue (eWAT).

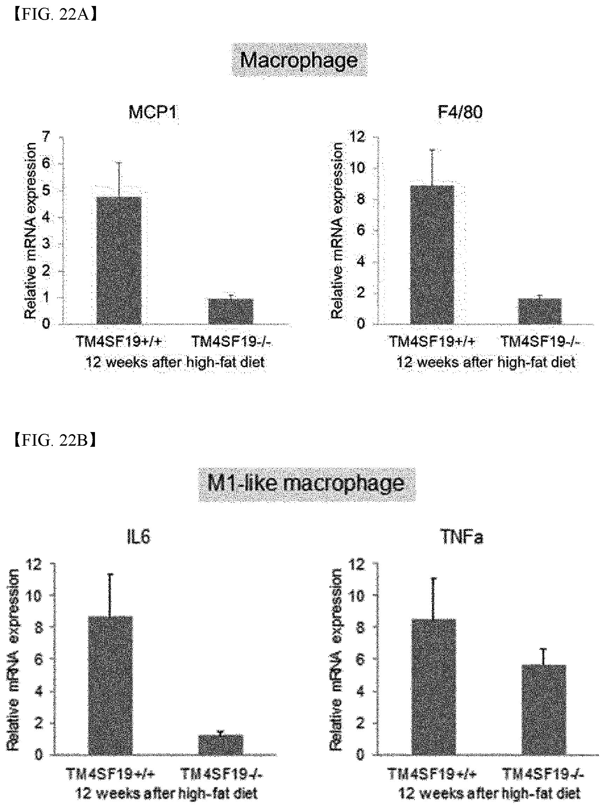

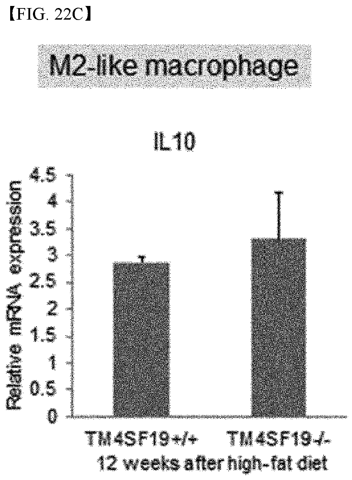

As a result, a stromal vascular fraction (SVF) was isolated from the epididymal white adipose of the TM4SF19KO mice, and a decrease in expression of macrophage markers MCP1 and F4/80 (FIG. 22A) and M1-like macrophage markers IL6 and TNFα (FIG. 22B) and an increase in expression of M2-like macrophage marker IL10 (FIG. 22C) were confirmed.

### (7) Confirmation of the role of TM4SF19 in inflammation, insulin resistance and fatty liver in eWAT

To confirm the role of TM4SF19 in inflammation, insulin resistance and fatty liver in eWAT, an experiment with a high-fat diet-induced obesity mouse model was performed.

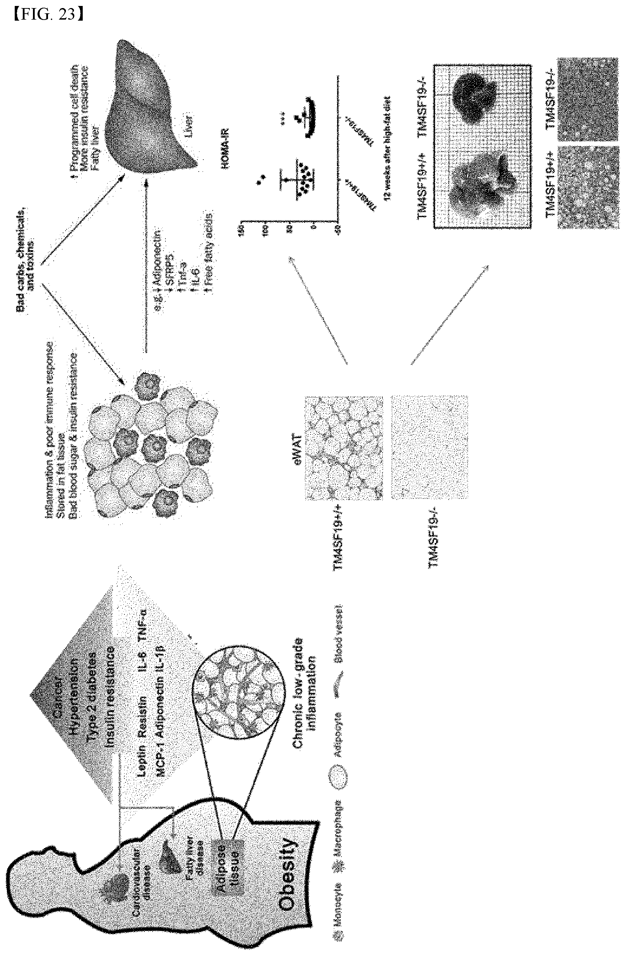

As a result, TM4SF19 regulates adipocyte differentiation and an inflammatory response including macrophages, and therefore, it was confirmed that TM4SF19 deficiency lowers insulin resistance and suppressed fatty liver (FIG. 23).

### (8) Confirmation of white-to beige adipocyte conversion in TM4SF19 KO mouse

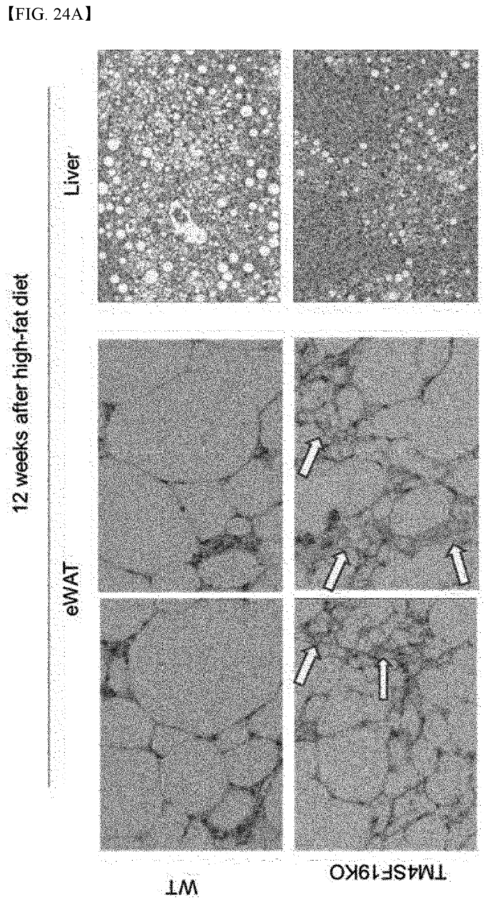

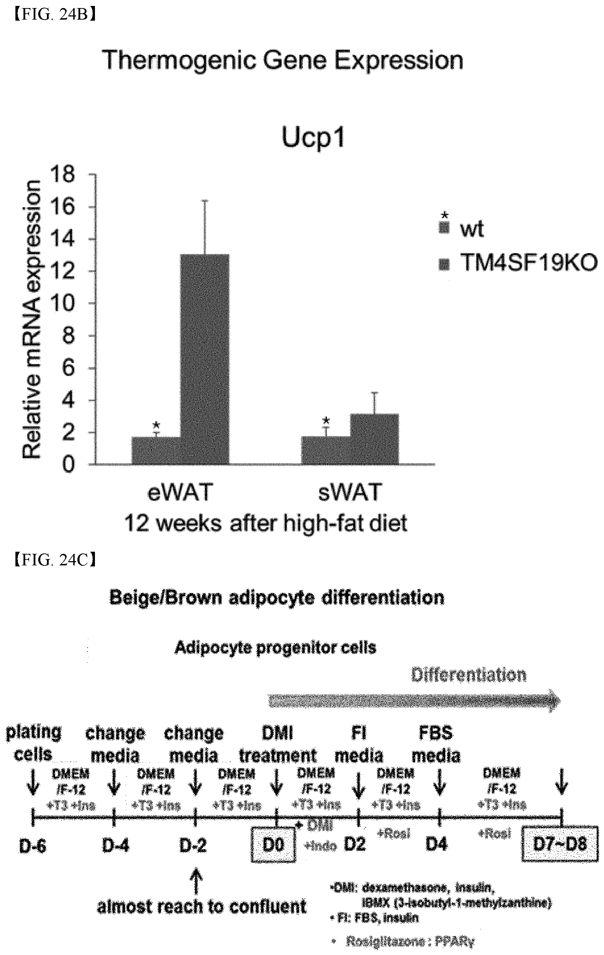

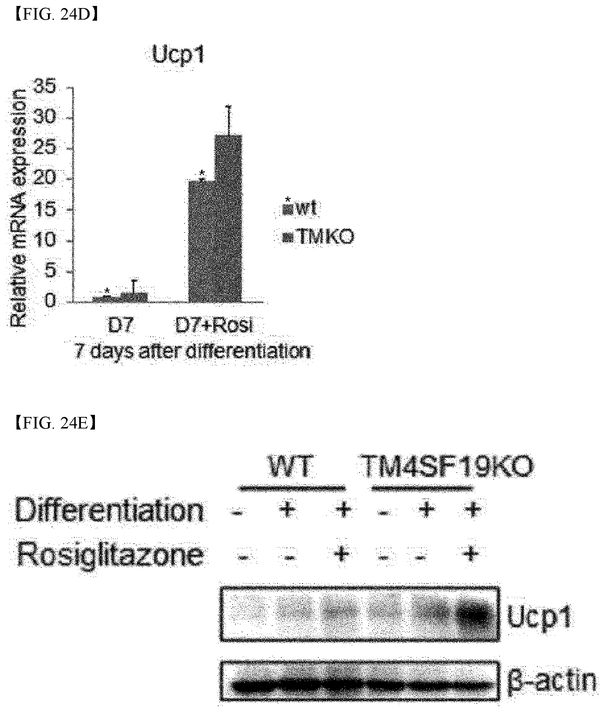

In a high-fat diet-induced obese mouse model (prepared by feeding 6-week-old mouse, which had been fed a normal diet, a high-fat diet for 12 weeks), the conversion from the epididymal white adipose of a TM4SF19KO mouse to a beige or brown fat phenotype was confirmed. In addition, it was confirmed that Ucp1 expression in white fat of the TM4SF19KO mouse was increased in an obese mouse model that was indued by a high-fat diet for 12 weeks, and it was confirmed that Ucp1 was increased in TM4SF19KO in a brown fat differentiation in vitro model, confirming that TM4SF19 is involved in changing white adipocytes into beige adipocytes (FIG. 24).

FIG. 24A shows a beige or brown fat phenotype and fatty liver formation in epididymal white adipose of a high-fat diet-induced obese WT or TM4SF19KO mouse confirmed by H&E staining.

FIG. 24B shows the result of confirming the expression of Ucp1, a brown adipocyte marker, in epididymal white adipose tissue and subcutaneous white adipose tissue of a high-fat diet-induced obese WT or TM4SF19KO mouse. It was confirmed that the Ucp1 expression in the white adipose tissue of TM4SF19KO was increased.

FIG. 24C is a schematic diagram illustrating a method for beige/brown adipocyte differentiation of adipocyte extracted from white adipose tissue. Adipocyte progenitor cells isolated from the white adipose tissue of WT and TM4SF19KO mice were grown in DMEM/F-12 media supplemented with T3 and insulin. Two days post confluency, cells were treated with DMI and indomethacin to begin differentiation, and from day 2, grown until day 7 or 8 while being treated with rosiglitazone to induce beige/brown adipocyte differentiation.

FIGS. 24D and 24E show the results of confirming the expression of the Ucp1 gene and protein after beige/brown adipocyte differentiation.

### (9) Confirmation of TM4SF19 expression in adipocytes and stromal vascular fraction (SVF) of white adipose tissue

TM4SF19 expression was confirmed by dividing adipocytes and SVFs in epididymal white fat and subcutaneous white fat of mice fed a high-fat diet for 24 weeks.

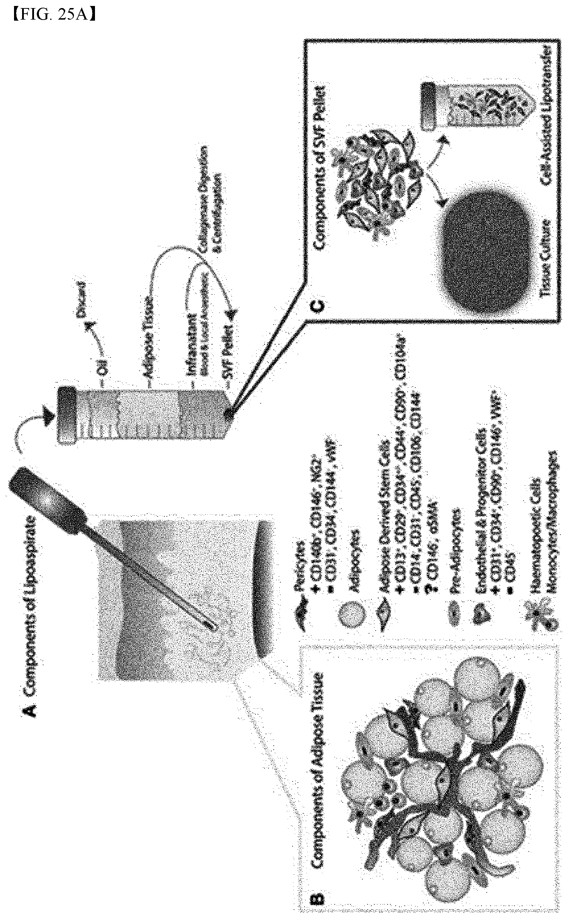

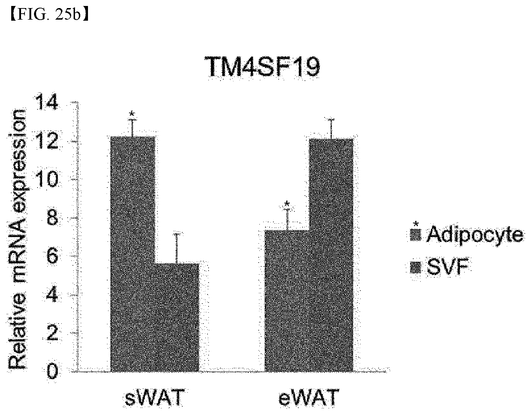

In eWAT and sWAT of a high-fat diet-induced (giving a high-fat diet to 6-week-old mice fed a normal diet for 24 hours) mice, TM4SF19 expression was confirmed in both adipose tissue and SVFs including macrophages, indicating that TM4SF19 functions not only in adipocytes, but also in SVFs including macrophages and monocytes (FIG. 25).

FIG. 25A is a schematic diagram of cell types in each fraction after white fat is digested with collagen type I and divided into adipose tissue, an infranatant and an SVF fraction through centrifugation.

FIG. 25B is a result confirming the expression of TM4SF19 after epididymal and subcutaneous white adipose tissue of mice fed a high-fat diet (60% fat) for 24 weeks are divided into adipocytes and SVF.

### (10) Induction of brown fat differentiation by treatment of stromal vascular fraction (SVF) isolated from sWAT of TM4SF19KO and wild-type mice with isoproterenol

SVFs were isolated from subcutaneous white adipose of wild-type (WT) and TM4SF19KO mice and differentiation into brown fat was induced with isoproterenol, and to confirm thermogenic gene expression and the expression of a beige fat marker gene, qPCR was carried out.

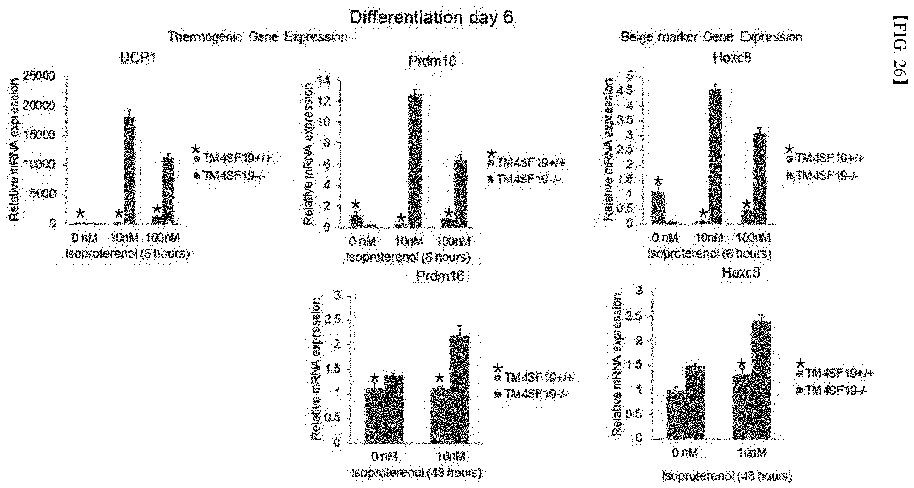

It was seen that thermogenic gene expression and the expression of a beige fat marker gene were increased in the SVF of the TM4SF19KO mouse, and it suggests that the TM4SF19KO mouse may involve in conversion of white fat into brown or beige fat (FIG. 26).

### (11) TM4SF19 expression in co-culture of macrophages and 3T3-L1 adipocytes

In high-fat diet-induced obesity, the interaction between 3T3-L1 adipose tissue and macrophages plays an important role.

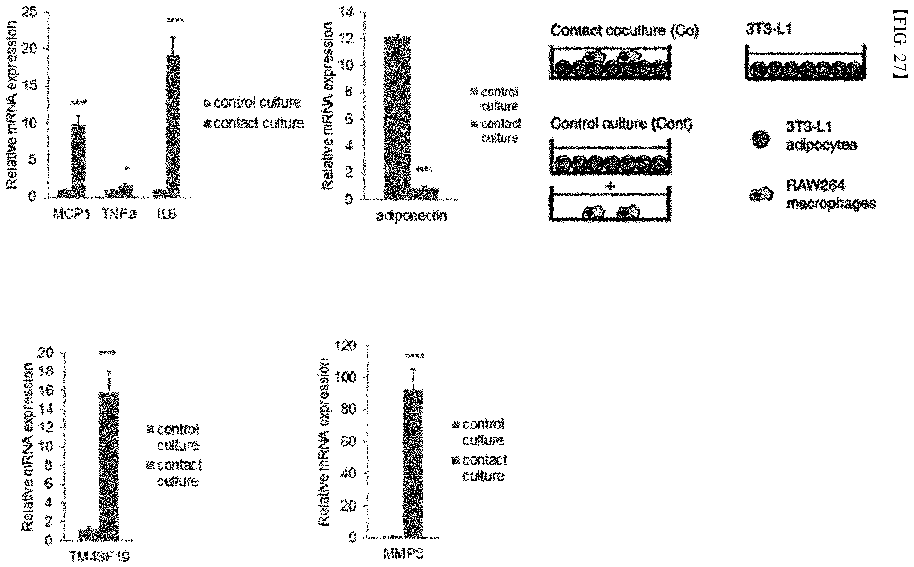

As shown on the right side of FIG. 27, differentiated adipocytes were co-cultured with macrophages in the contact system. The expression of macrophage, inflammation and anti-obesity marker genes [Macrophage marker; MCP-1, inflammation marker; TNFα, IL6, MMP3, anti-obesity marker; adiponectin] was confirmed by qPCR. As a control, macrophages and adipocytes were grown, respectively, and RNA was extracted and combined.

By the co-culture of 3T3-L1 adipocytes and macrophages, the expression of MMP3, which is an inflammation-related gene, and TM4SF19 was increased.

### (12) Induction of inflammatory change by co-culture of adipocytes and macrophages in contact system

After SVFs were extracted from wild-type (WT) mice and subjected to adipogenesis, wild-type (WT) bone marrow was differentiated into macrophages and then an inflammatory change was induced individual culture or contact co-culture. As a control, macrophages and adipocytes were grown, respectively, and RNA was extracted and combined.

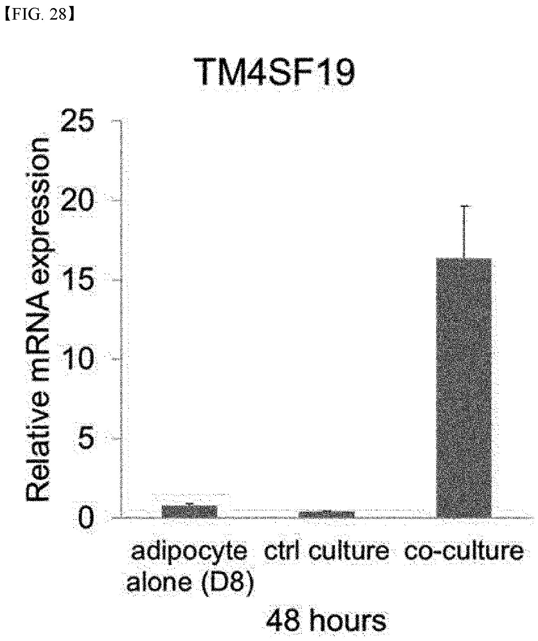

After the adipocyte differentiation of SVFs and the macrophage differentiation, the expression of TM4SF19 was increased by co-culture (FIG. 28).

After SVFs were extracted from wild-type (WT) and TM4SF19KO mice and subjected to adipogenesis and bone marrow was extracted from each mouse and differentiated into macrophages, an inflammatory change was induced by contact co-culture.

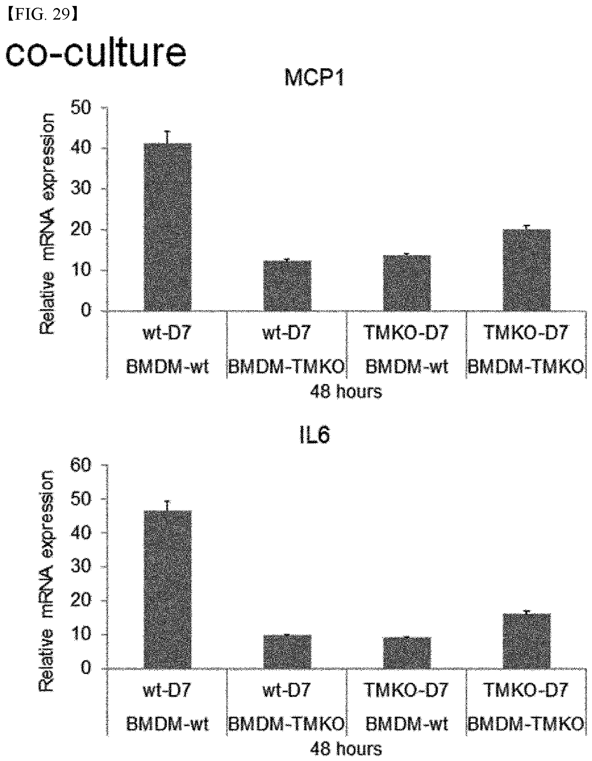

As a result, as shown in FIG. 29, compared to WT differentiated adipocyte + macrophage differentiation in a co-culture system, the expression of MCP1 and IL6 was decreased in TM4SF19KO differentiated adipocyte + macrophage differentiation in a co-culture system.

After SVFs were extracted from wild-type (WT) and TM4SF19KO mice and subjected to adipogenesis and bone marrow was extracted from each mouse and differentiated into macrophages, an inflammatory change was induced by ctrl culture or co-culture. As a control (ctrl culture), macrophages and adipocytes were grown, respectively, and RNA was extracted and combined.

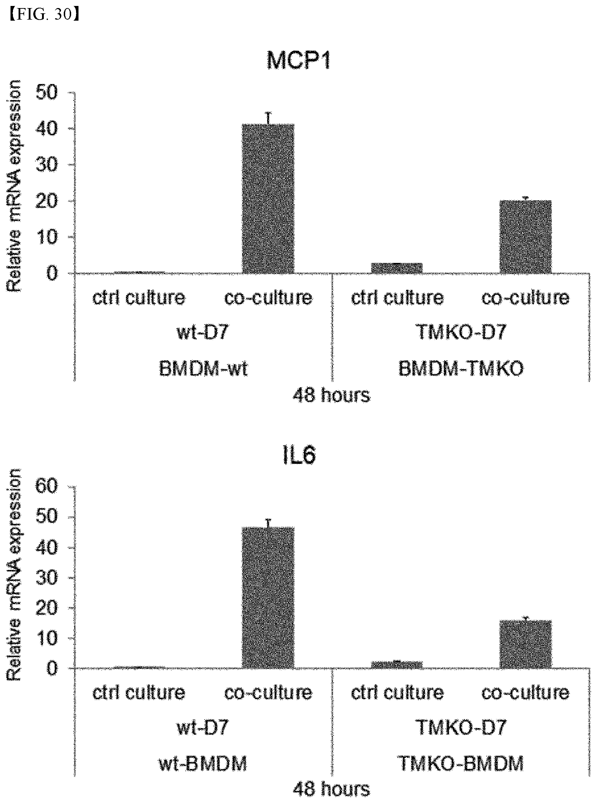

In the co-culture models after adipocyte differentiation of SVFs and macrophage differentiation of the bone marrow, TM4SF19KO BMDM reduced the expression of an inflammatory response marker, and the inflammatory response was reduced in the TM4SF19KO adipocyte-TM4SF19KO macrophage co-culture, compared to the WT adipocyte-WT macrophage (FIG. 30).

In the case of the co-culture of adipocytes and BMDM, an inflammation signal increased.

### (13) Confirmation of the expression of TM4SF19 in in vitro insulin-resistant model

An in vitro insulin-resistant model was manufactured.

3T3-L1 adipocytes were differentiated for 12 days and treated with TNFα for 24 hours, and an adipogenic differentiation marker C/EBPα and insulin-resistant in vitro model markers C/EBPβ and TM4SF19 were confirmed by qPCR.

TNFα induces inflammation, and is a model mimicking an increased inflammation signal in the surroundings when obesity was induced in vivo.

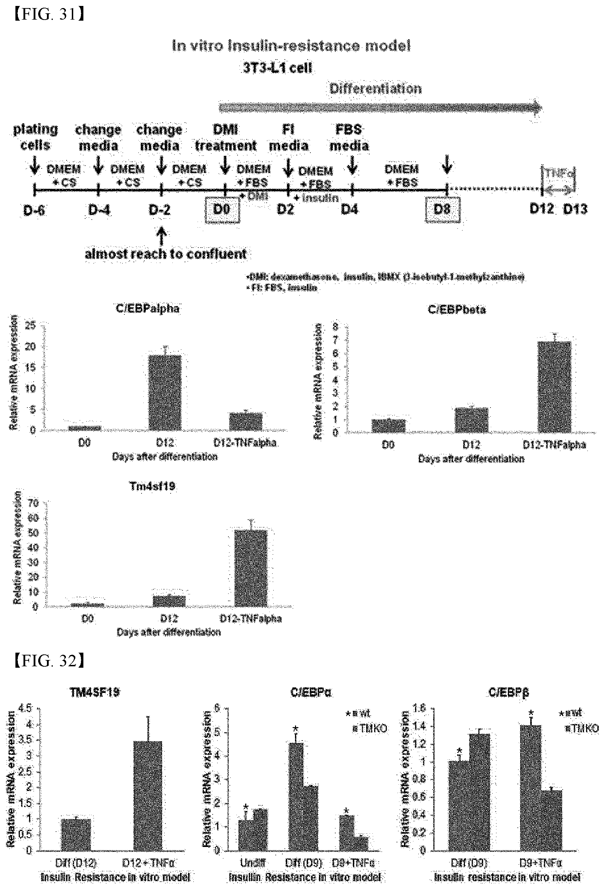

SVFs were extracted from wild-type (WT) and TM4SF19KO mice and subjected to adipogenic differentiation and treated with TNFα for 24 hours, and the adipogenic differentiation marker C/EBPα and the insulin-resistant in vitro model markers C/EBPβ and TM4SF19 were confirmed by qPCR. As a result, it was confirmed that TM4SF19 increased in an in vitro insulin-resistant model (FIGS. 31 and 32). It was confirmed that TM4SF19KO reduces insulin resistance (C/EBPβ is an insulin resistance marker).

### (14) The role of TM4SF19 in macrophage differentiation and polarization

After bone marrow was extracted from wild-type (WT) and TM4SF19KO mice and subjected to differentiation into macrophages, a marker involved in M1 polarization was confirmed. M1 macrophages are involved in preinflammation signaling.

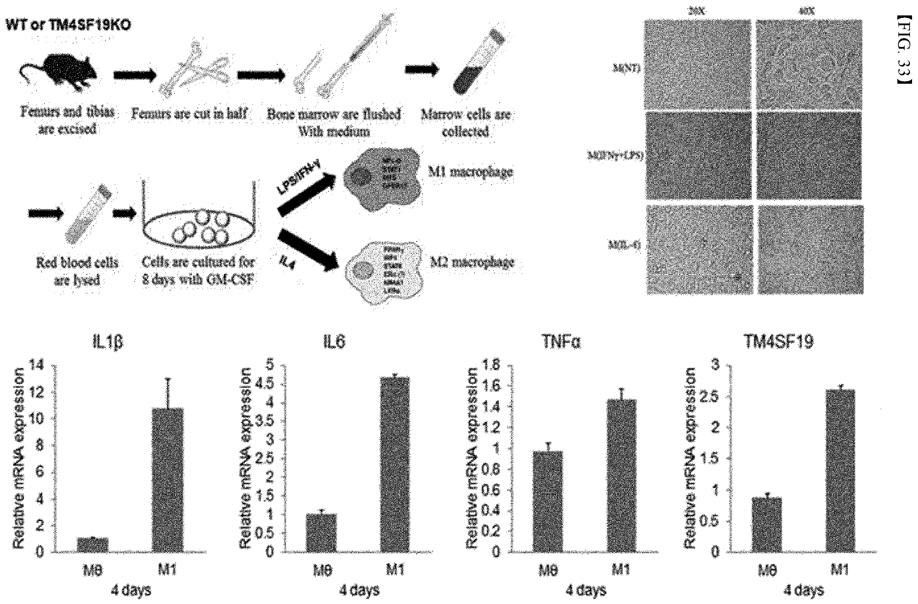

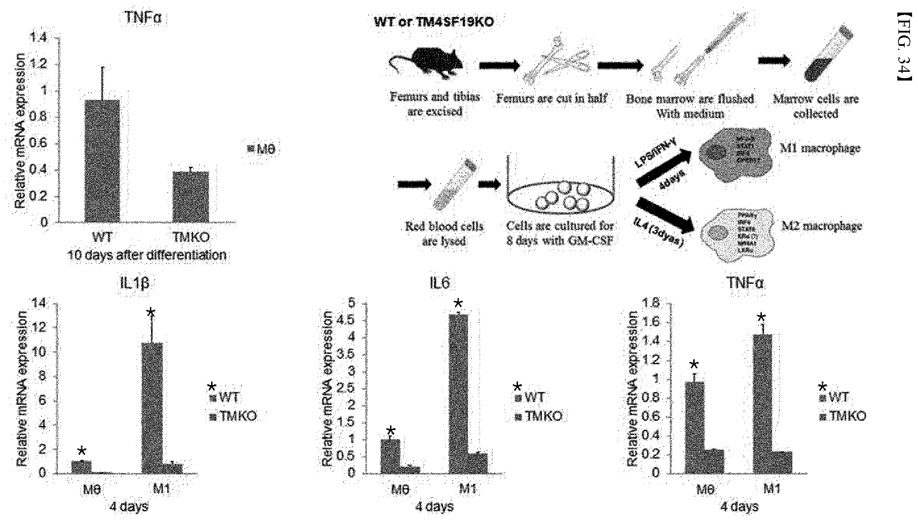

It was confirmed that the inflammation marker and TM4SF19 are increased by the differentiation of wild-type (WT) mouse bone marrow-derived M1 macrophages (FIG. 33), and a bone marrow-derived M1 macrophage differentiation marker is reduced in TM4SF19KO (FIG. 34).

### Preparation Example 1: Manufacture and verification of TM4SF19 antibody

### (1) Manufacture of polyclonal mouse TM4SF19 antibody

After determining an available target by predicting antibody immunogenicity, a polyclonal mouse TM4SF19 antibody targeting the aa 141-159 region in mouse TM4SF19 extracellular loop 2 was manufactured (mouse TM4SF19 protein is represented by SEQ ID NO: 7).

### (2) Verification of TM4SF19 antibody

After human TM4SF19 (hTM4SF19), human EC2Δ (hTM4SF19 115-175Δ), mouse TM4SF19 (mTM4SF19), and mouse EC2Δ (mTM4SF19 116-165Δ) were transiently overexpressed in 293T cells, to confirm expression using the previously-manufactured polyclonal mouse TM4SF19 antibody, western blotting was carried out.

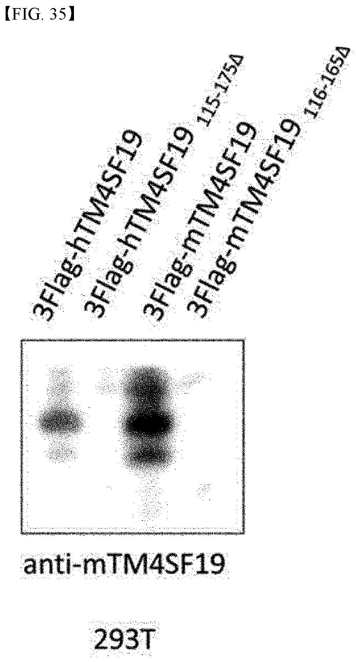

A polyclonal mouse TM4SF19 antibody recognized human TM4SF19 and mouse TM4SF19. However, an extracellular loop 2 deletion mutant was not recognized (FIG. 35).

### Example 9: Verification of TM4SF19 role using TM4SF19 antibody

### (1) Confirmation of osteoclast differentiation inhibition by TM4SF19 antibody

Bone marrow was extracted from a wild-type (WT) mouse, differentiated into osteoclasts, and then treated with a TM4SF19 antibody, followed by TRAP staining.

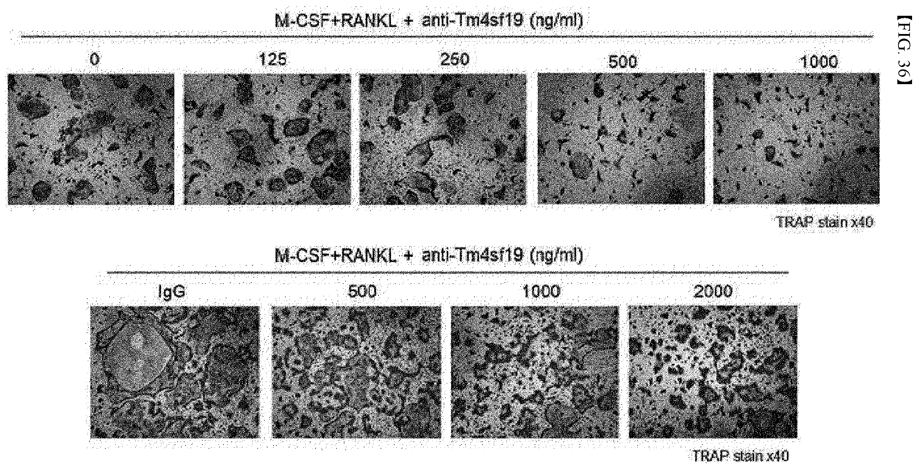

As a result, the TM4SF19 antibody dose-dependently inhibited osteoclast multinucleation (FIG. 36).

### Preparation Example 2: Manufacture of TM4SF19-Fc

TM4SF19-Fc fusion proteins for human TM4SF19 and mouse TM4SF19 extracellular loop 2 were manufactured using information on the sequence and phylogenetic tree of the TM4SF family.

TM4SF19-Fc is a form in which human IgG1-Fc is fused to the C-terminus of human TM4SF19 or mouse TM4SF19 extracellular loop 2.

Since mouse TM4SF19-Fc (116-165) was well secreted, it was purified to perform an in vitro test.

Since human TM4SF19-Fc (115-175) was not well secreted, Fc for a novel region (120-169) was manufactured.

The mouse TM4SF19-Fc was well secreted and thus purified.

The mouse TM4SF19EC2-Fc (116-165) was manufactured, and used in a subsequent experiment.

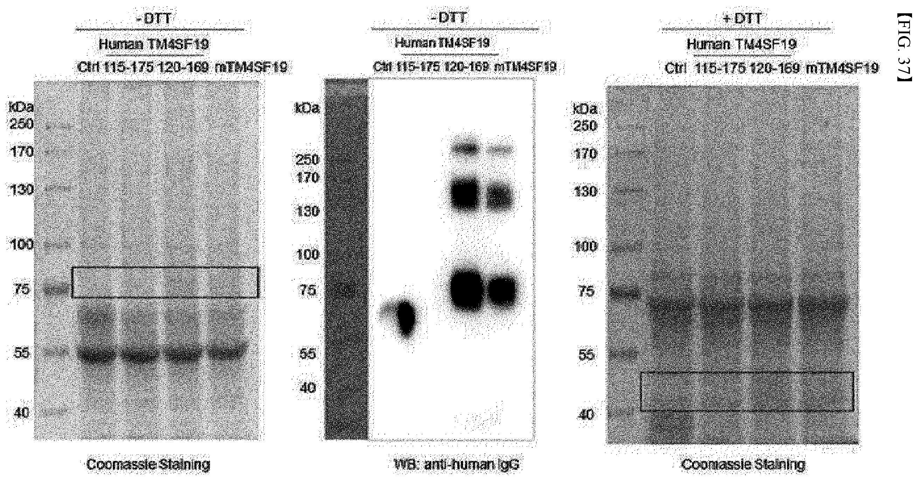

The human TM4SF19-EC2-Fc (115-175) was not well expressed, so the expression was confirmed by re-manufacture with mouse TM4SF19-EC2-Fc and a conserved sequence. Human TM4SF19-Fc (120-169) was secreted into media more than the extracellular loop 2 (115-175), and thus also used in a subsequent experiment (FIG. 37).

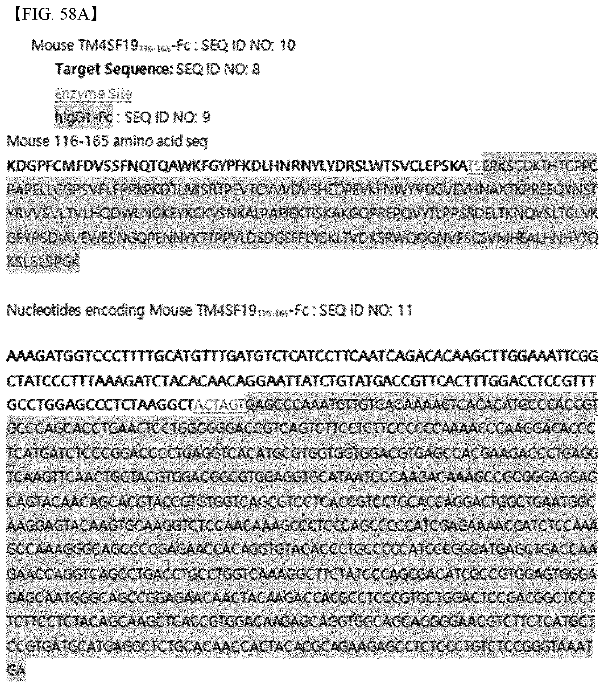

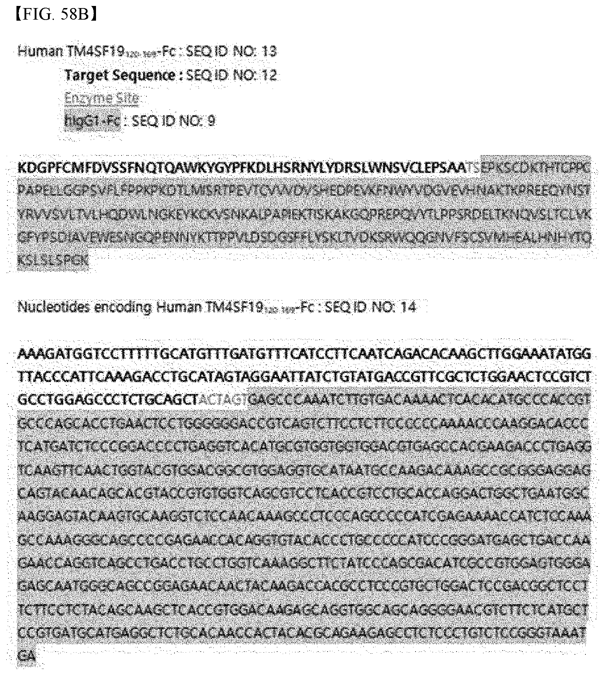

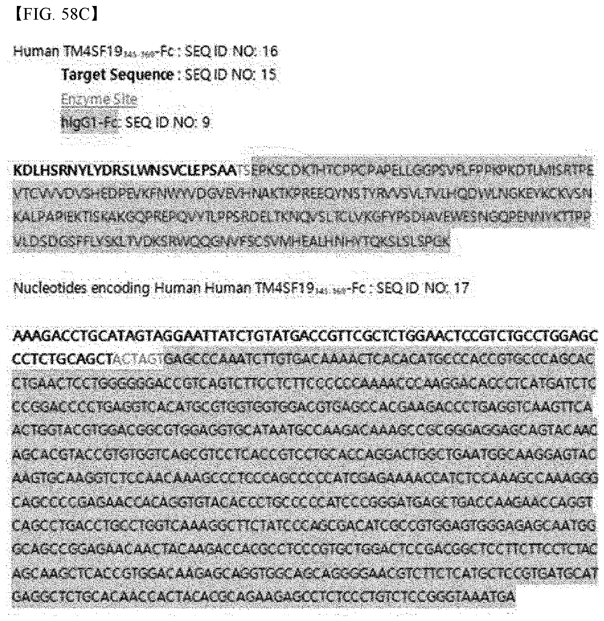

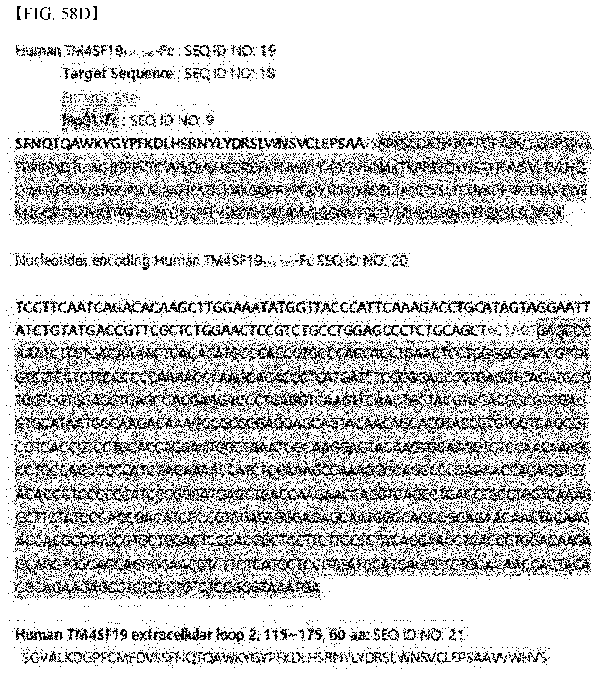

The fusion protein used in the example according to the present invention is shown in FIG. 58.

Hereinafter, the "hTM4SF19-Fc" refers to an hTM4SF19-Fc (aa 120-169) fusion protein. The "mTM4SF19-Fc" refers to a mouse TM4SF19-Fc (aa 116-165) fusion protein.

### Example 10: Verification of TM4SF19 role using TM4SF19-Fc

hIgG1-Fc, hTM4SF19 120-169-Fc and mTM4SF19 116-165-Fc were treated in osteoclast differentiation. For osteoclast differentiation, bone marrow-derived cells were treated with MCSF (60 ng/ml) and RANKL (100 ng/ml), and TRAP staining was carried out.

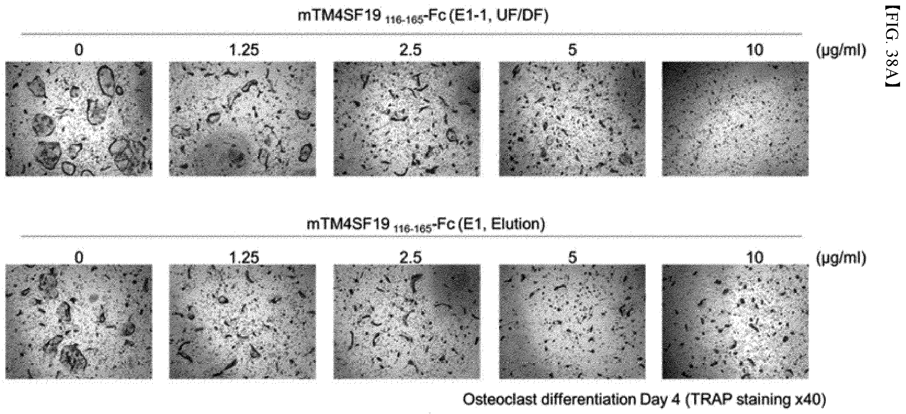

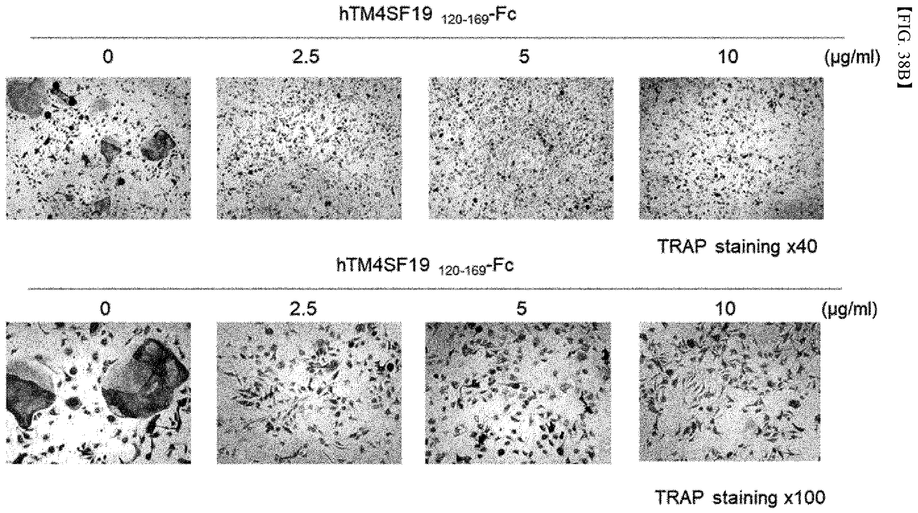

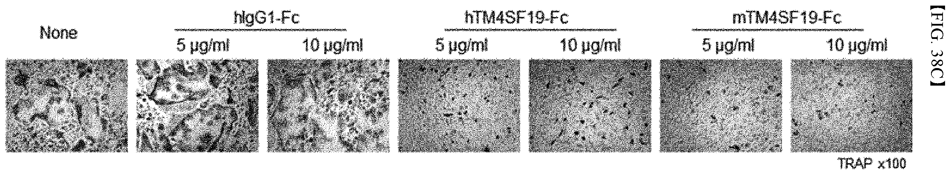

The results are shown in FIGS. 38A to 38C. The inhibition of multinucleated osteoclast formation was confirmed by treating purified mTM4SF19 EC2-Fc during osteoclast differentiation [E1; sample eluted with elution buffer (20 mM glycine). E1-1; sample (Fc may be more stable) subjected to ultrafiltration/diafiltration (UF/DF) with buffer A (50 mM phosphate, 50 mM NaCl, pH 7.0)]. In addition, the inhibition of multinucleated osteoclast formation was confirmed by treating purified hTM4SF19 120-169-Fc.

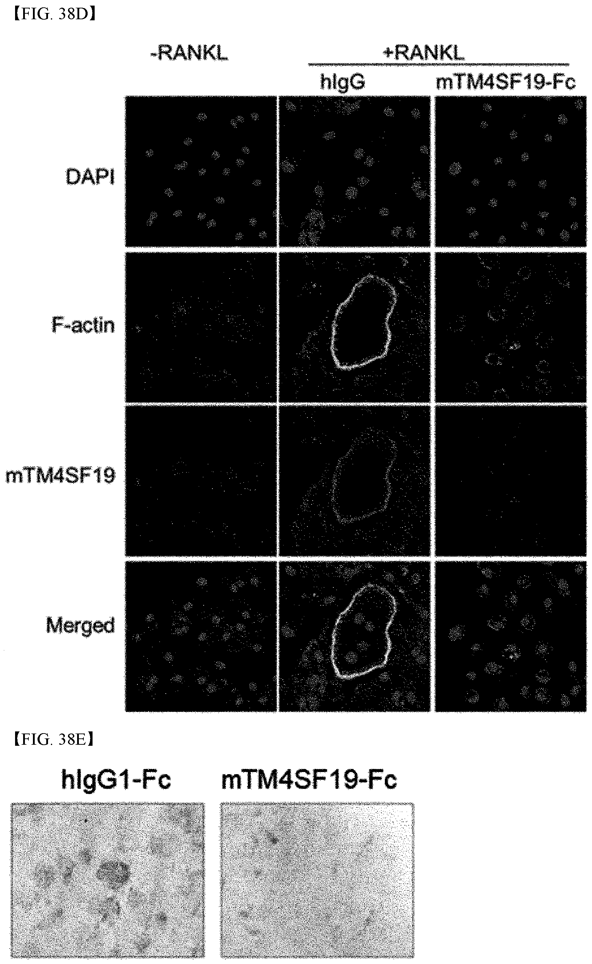

After the wild-type mouse bone marrow was differentiated and treated with 10 µg/ml of IgG-Fc and mTM4SF19-Fc, actin belt formation occurring in multinucleated osteoclast differentiation was confirmed by F-actin staining. It was confirmed that actin belt formation is inhibited by mTM4SF19-Fc, and the localization of TM4SF19 in the actin belt using the mouse TM4SF19 antibody (FIG. 38D).

BMMs were plated on a Dentin disc, differentiated and then treated with 10 µg/ml of IgG-Fc or mTM4SF19-Fc. Pit formation was confirmed by 1% toluidine blue staining. As shown in FIG. 38E, it was confirmed that bone resorption is blocked by mTM4SF19-Fc.

### Example 11: Confirmation of bone loss rescue using TM4SF19-Fc

One week after ovariectomizing 8-week-old female mice, mouse TM4SF19-Fc was injected into the tail vein once a week for 3 weeks, micro-CT was measured after sacrificing and fixing the femur a week after the last injection. The concentrations of the mouse TM4SF19-Fc used herein were 2.5 mg/kg and 5 mg/kg.

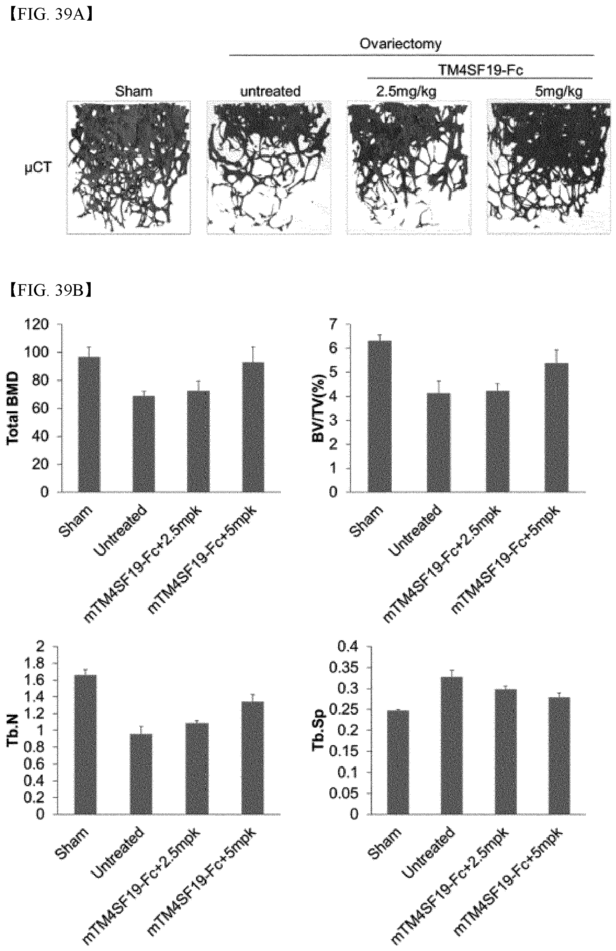

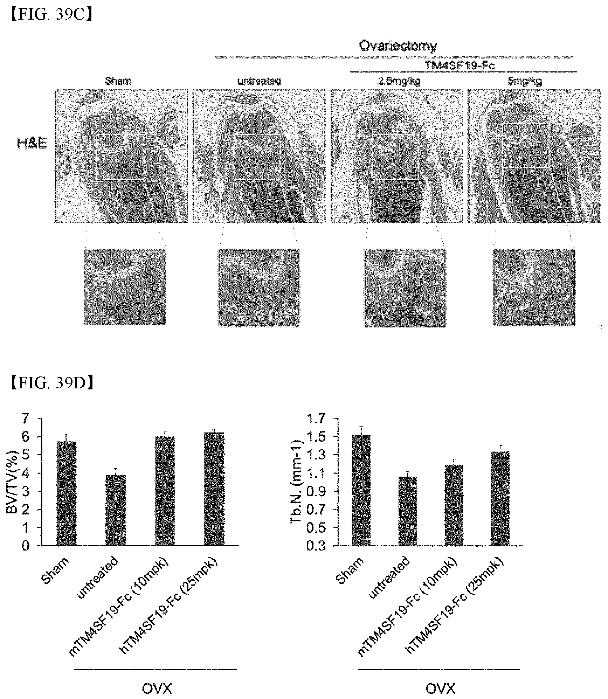

FIG. 39A is a set of micro-CT images showing bone loss rescued by mouse TM4SF19-Fc.

3D micro-CT parameters (bone microstructure parameters) including a bone volume (BV), a ratio of bone volume to tissue volume (% BV/TV), an average trabecular number (Tb.N) and a trabecular separation (Tb.Sp) were obtained by analyzing the micro-CT images using a 3D imaging program of FIG. 39A (FIG. 39B).

As shown in FIG. 39B, it was confirmed that the total BMD (bone mineral density), % BV/TV (bone volume per tissue volume), and Tb.N (trabecular number) are decreased by the ovariectomy and dose-dependently increased by mTM4SF19-Fc. On the other hand, it was confirmed that the Tb. Sp (trabecular separation) was reduced by mTM4SF19-Fc. From the above results, it can be confirmed that mouse TM4SF19-Fc rescued ovariectomy-induced bone loss.

In addition, mouse femur tissues were histopathologically analyzed through H&E staining. As a result, as shown in FGI. 39C, it can be confirmed that Tb.N (trabecular number) was reduced by the ovariectomy and the reduction in Tb.N was rescued by TM4SF 19-Fc treatment in a dose-dependent manner.

In addition, after an ovariectomy on 8-week-old female mice, mouse TM4SF19-Fc (116-165) and human TM4SF19-Fc (131-169) were intravenously injected. Compared to the untreated group, it was confirmed that bone loss is significantly inhibited by human and mouse TM4SF19-Fc (FIG. 39D). The mouse TM4SF19-Fc (116-165) was treated at 10 mg/kg, and the human TM4SF19-Fc (131-169) was treated at 25 mg/kg.

The aa 131-169 region of the human TM4SF19 can rescue ovariectomy-induced bone loss.

The above results suggest the possibility of developing TM4SF19-Fc as a therapeutic agent for osteoporosis.

### Example 12: Verification of TM4SF19 role using TM4SF19-Fc

### (1) Osteoclastogenesis

Cells were isolated from the bone marrow of a wild-type (WT) mouse and differentiated under a condition of treating M-CSF or M-CSF and RANKL, and then after treating 10 µg/ml of mTM4SF19-Fc (control: treated with buffer), the expression of Ctsk and Acp5, which are genes involved in osteoclast differentiation, was confirmed by qPCR.

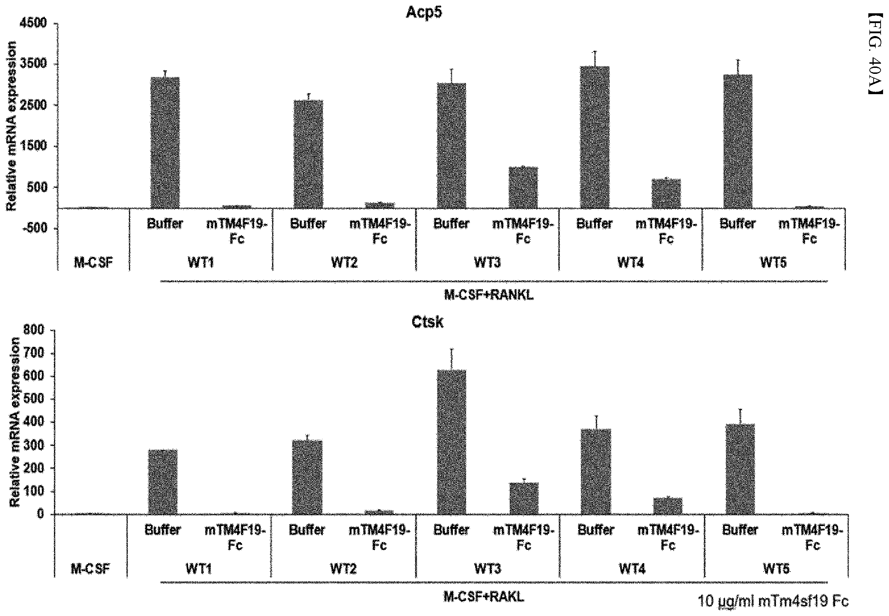

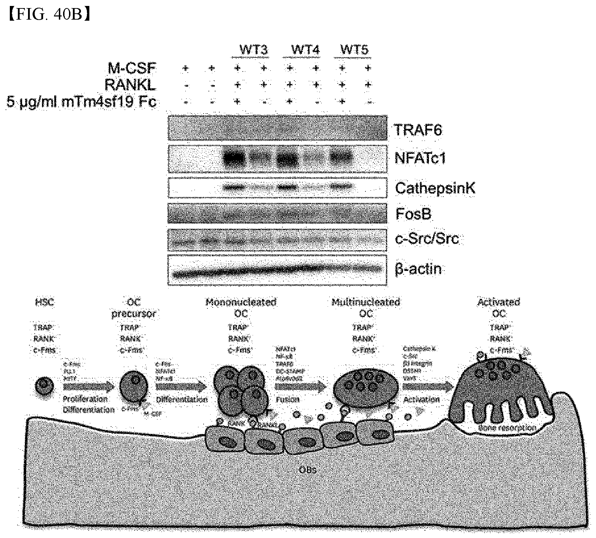

FIG. 40 is a set of graphs showing mRNA expression of Acp5 and Ctsk in osteoclast differentiation under a condition of treating M-CSF or M-CSF and RANKL (WT1, 2, 3, 4 and 5 indicate the results obtained by osteoclast differentiation of bone marrow cells obtained from each of five different wild-type mice; and the group treated with only M-CSF refers to an undifferentiated group).

As shown in FIG. 40A, the expression of genes involved in osteoclast differentiation was inhibited by mTM4SF19-Fc treatment, compared to the control (WT).

Likewise, as a result of confirming the expression of an osteoclast differentiation marker protein after cells isolated from the bone marrow of the wild-type (WT) mouse are treated with 5 µg/ml of mTM4SF19-Fc while being differentiated under a condition of treating M-CSF or M-CSF and RANKL, as shown in FIG. 40B, compared to the control (WT), it was confirmed that the expression of the osteoclast differentiation marker protein is more significantly inhibited by mTM4SF19-Fc.

### (2) Surface binding of mTM4SF19-Fc

The reason why TM4SF19-Fc blocks osteoclast multinucleation is determined to be due to its increased ability of surface binding during differentiation, so the surface binding of mTM4SF19-Fc before and after cell differentiation of Raw264.7 cells was analyzed by FACS. As shown in FIG. 41, while there is almost no difference in surface binding between the control IgG1 (dark gray graph) and mouse TM4SF19-Fc (light gray graph) before differentiation, compared to the control IgG1 (dark gray graph), mTM4SF19-Fc (light gray graph) migrates to the right after differentiation, confirming that, in fact, the surface binding of mTM4SF19-Fc increased after differentiation.

As shown in FIG. 41, it was confirmed that binding to mTM4SF19-Fc increases after differentiation.

### Example 13: Prevention and treatment of rheumatoid arthritis using TM4SF19-Fc

According to the experimental schedule of FIG. 42A, the semi-therapeutic effect of mTM4SF19-Fc was confirmed in a collagen-induced arthritis mouse model.

18 days after acclimation of DBA/1 mice for 14 days, chicken collagen II was mixed with complete Freund's adjuvant (CFA) and injected, chicken collagen II was mixed with incomplete Freund's adjuvant (IFA) for boost injection. mTM4SF19-Fc began to be administered 15 days after the first injection, and intravenously injected at 10 mg/kg or 25 mg/kg twice a week. CIA scores were checked twice a week until day 63 when mice were sacrificed.

The CIA scores were evaluated by the following method: Score 0 (normal paw), Score 1 (one or two toes inflamed and swollen), Score 2 (3+ toes inflamed w/no paw swelling, or mild swelling of entire paw), Score 3 (entire paw inflamed and swollen), Score 4 (severely swollen paw and all toes, or ankylosed paw and toes)

As a result of checking the joint state of the mice 42 days after collagen-induced arthritis (CIA), as shown in FIG. 42B, compared to the Sham group treated with nothing, in the IgG1-treated control, toes and feet were all swollen, but it was confirmed that toe and foot edema was somewhat inhibited in the group treated with 10 mg/kg of mTM4SF19-Fc, and the edema phenomenon was apparently inhibited in the group treated with 25 mg/kg of mTM4SF19-Fc.

Meanwhile, the graphs of FIG. 42C show the results of analyzing the CIA scores and the number of swollen joints investigated during the experiment. It was confirmed that the CIA scores and edema were dose-dependently reduced in the mTM4SF19-Fc-treated group, compared to the IgG1-treated control.

In addition, the feet of the collagen-induced arthritis mouse models were photographed by micro-CT, and the images were analyzed.

FIG. 42D is a set of micro-CT images showing that inflammation and bone damage occurring in the paw of collagen-induced arthritis mouse models are dose-dependently inhibited by mTM4SF19-Fc treatment. The toes were bent by collagen-induced arthritis, and bone damage occurred due to inflammation in toe joints and ankles. However, compared to a control, a hIgG1-Fc-treated mouse, the joint inflammation and bone damage in the mTM4SF19-Fc-treated mouse were dose-dependently inhibited.

Referring to FIG. 42E, inflammation and bone damage occurring in the paw of collagen-induced arthritis mouse models are dose-dependently inhibited by mTM4SF19-Fc treatment, and it can be confirmed that, compared to the control hIgG1-Fc-treated mouse, bone damage was inhibited in the mice having the same RA scores.

In addition, by analyzing the micro-CT images of femurs of the collagen-induced arthritis mouse models using a 3D imaging program, 3D micro-CT parameters (bone microstructure parameters) including a bone volume (BV), a ratio of bone volume to tissue volume (% BV/TV), an average trabecular number (Tb.N), and a trabecular separation (Tb.Sp) were obtained.

FIG. 42F shows that, compared to the Sham group, BMD and Tb.N are reduced by collagen-induced arthritis, but bone loss is inhibited by TM4SF19-Fc.

FIG. 42G is a result of confirming cartilage damage in collagen-induced arthritis mice using toluidine blue. Compared to a hIgG 1-Fc treated group, it is shown that the cartilage damage is inhibited in the mTM4SF19-Fc treated group.

In addition, in collagen antibody-induced arthritis mouse models, the semi-therapeutic effect of mTM4SF19-Fc and hTM4SF19-Fc (120-169) was confirmed by arthritis scores. Three days after the administration of a collagen antibody into the Balbc mouse, arthritis was induced by LPS injection. Compared to the control, an arthritis inhibitory effect was shown at 25 mg/kg of mTM4SF19-Fc and 25 mg/kg of hTM4SF19-Fc (120-169) (FIG. 42H). It was confirmed that the arthritis disease score is lowered and the foot thickness was reduced by TM4SF19-Fc treatment, compared to hIgG1-Fc.

In addition, after arthritis was induced by LPS injection three days after collagen antibody administration into the collagen-induced arthritis mouse models, the therapeutic effect of mTM4SF19-Fc was confirmed by an arthritis score.

To confirm the therapeutic effect of mTM4SF19-Fc after inducing arthritis by LPS injection three days after collagen antibody administration, from day 8 when the arthritis disease score was the highest, 50 mg/kg of mTM4SF19-Fc was treated, and from day 9 to day 19, arthritis disease scores were checked every 2 days.

Compared to the untreated group, it was confirmed that, by mTM4SF19-Fc treatment, the arthritis scores were lowered, the foot thicknesses were reduced, and cartilage damage confirmed by toluidine blue was inhibited (FIG. 42I). That is, the arthritis treatment effect of mTM4SF19-Fc was shown.

### Example 14: Inhibition of bone metastasis of breast cancer using TM4SF19-Fc

According to the experimental schedule of FIG. 43A, MDA-MB231-luc breast cancer cells (a cell line expressing luciferase in MDA-MB231 for imaging) were injected into the tail of a 6-week-old female NOD-SCID mouse via a caudal artery (CA) route to induce bone metastasis. After the induction of bone metastasis, hIgG1 or TM4SF19-Fc was intravenously injected. 10 mpk or 25 mpk of TM4SF19-Fc began to be intravenously injected two days after the cancer cell injection and administered twice a week. A cancer progression state was confirmed through bioluminescence imaging analysis.

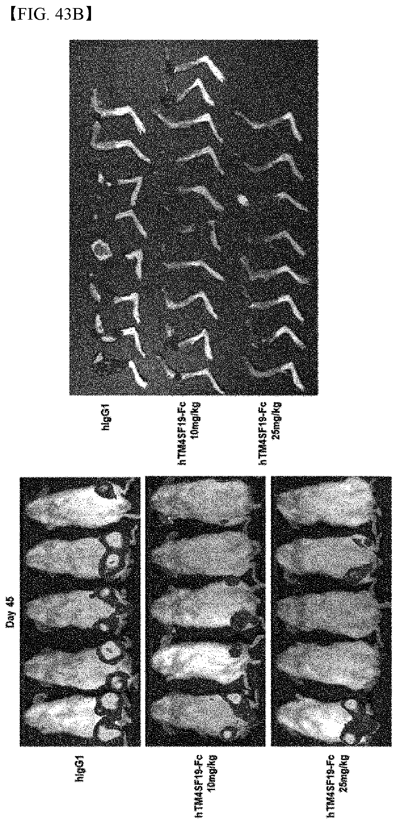

At 45 days after cancer cell injection, bone metastasis was detected through bioluminescence imaging analysis. As shown in FIG. 43B (left), compared to the control, which is the hIgG1-administered group, it was confirmed that bone metastasis is inhibited in the hTM4SF19-Fc-administered group. As a result of luminescence analysis on a leg excised from a mouse sacrificed following luciferin injection on day 45, as shown in FIG. 43B (right), it was confirmed that bone metastasis is inhibited by TM4SF19-Fc treatment.

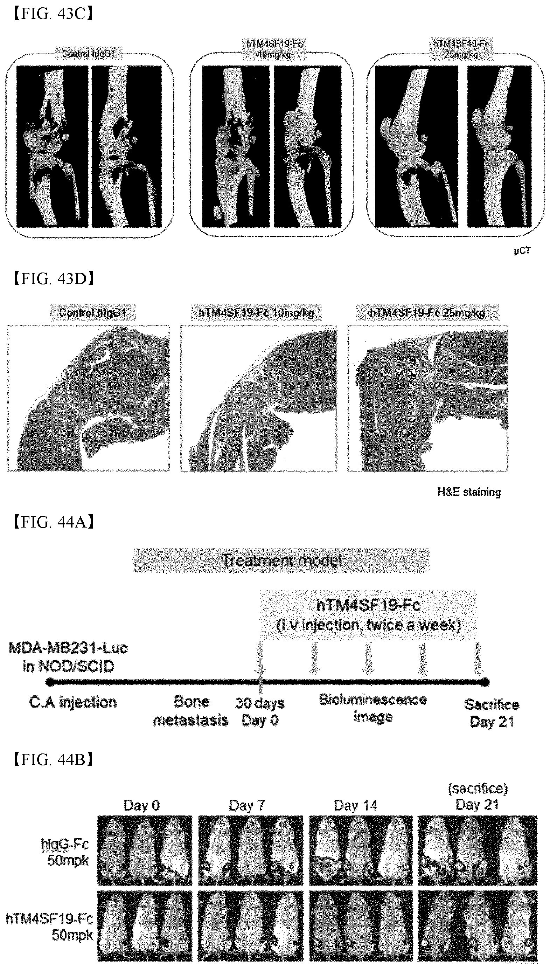

FIG. 43C shows the result of micro-CT scans for joints of a mouse in which bone metastasis of breast cancer occurs. According to the micro-CT analysis result, it was confirmed that bone damage occurred due to the bone metastasis of breast cancer, and bone damage was dose-independently rescued by TM4SF19-Fc treatment.

In addition, histological analysis was performed through H&E staining on joints of the mice in which the bone metastasis of breast cancer occurs. As a result, as shown in FIG. 43D, it can be confirmed that bone metastasis of breast cancer and tumor growth are dose-dependently inhibited by TM4SF19-Fc treatment.

According to the experimental schedule of 44A, MDA-MB231-luc breast cancer cells (a cell line expressing luciferase in MDA-MB231 through imaging) were injected into the caudal artery (CA) of a 6-week-old female NOD-SCID mouse to induce bone metastasis. From 30 days after the induction, 50 mg/kg of the control hIgG1 or TM4SF19-F began to be intravenously administered.

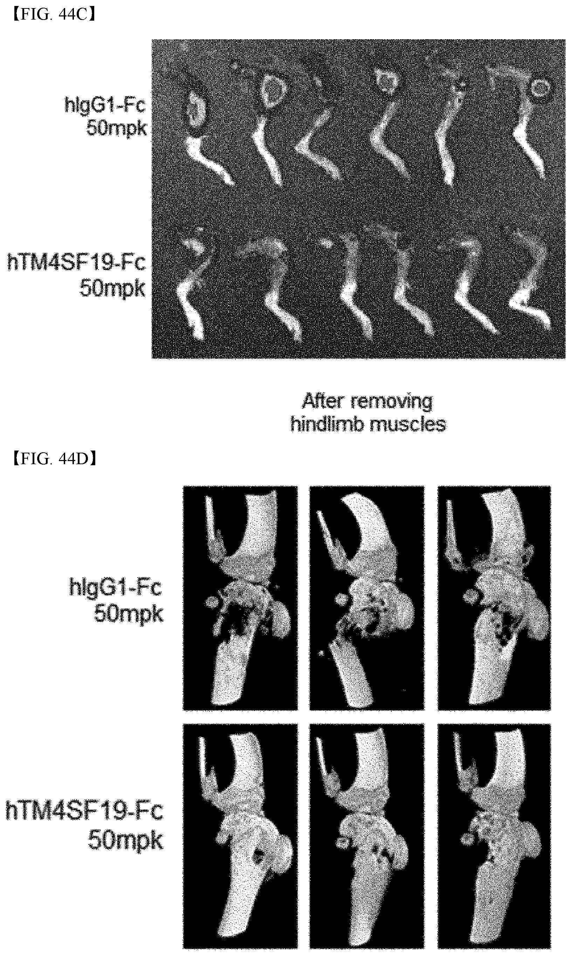

Bioluminescence imaging was analyzed every 7 days until 21 days after injection, and it was confirmed that bone metastasis was inhibited by hTM4SF19-Fc (120-169) (FIG. 44B). After luciferin injection, as a result of luminescence analysis on legs excised from the mouse, which had been sacrificed after luciferin injection, it was confirmed that bone metastasis was inhibited by TM4SF19-Fc treatment (FIG. 44C). The micro-CT images showed that bone destruction occurred due to bone metastasis of breast cancer and was rescued by TM4SF19-Fc treatment (FIG. 44D).

### Example 15: Inhibition of lung metastasis of breast cancer using TM4SF19-Fc

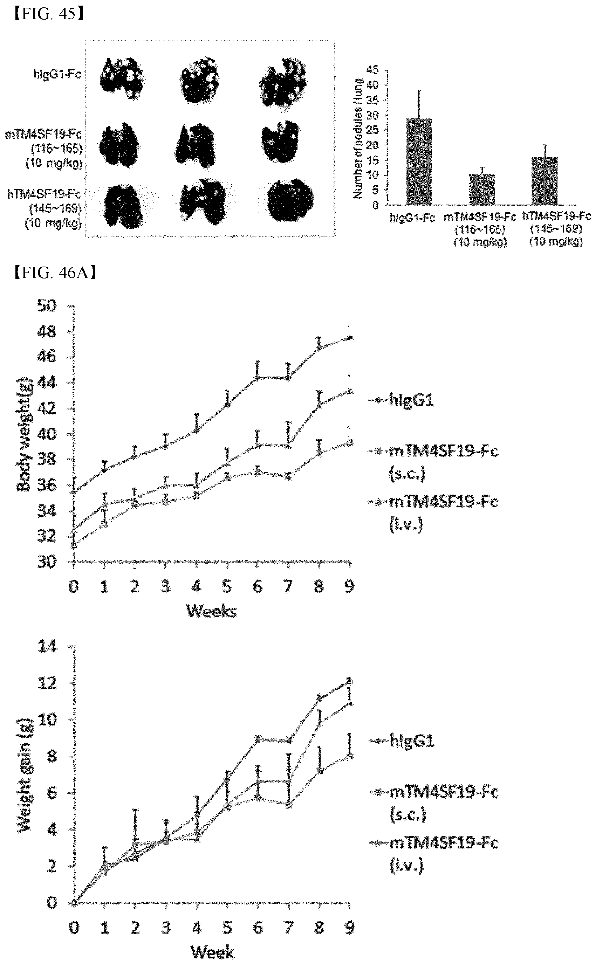

Murine E0771 breast cancer cells were injected into the tail vein of a wild-type mouse to induce lung metastasis. After intravenous injection of 10 mg/kg each of hIgG1-Fc, mouse TM4SF19-Fc (116-165) and human TM4SF19-Fc (145-169), the inhibition of lung metastasis of the breast cancer cells was confirmed by staining the cells with Indian ink and counting the number of nodules in the lungs. As shown in FIG. 45, it was confirmed that lung metastasis was significantly inhibited by mouse TM4SF19-Fc (116-165) and human TM4SF19-Fc (145-169).

### Example 16: Effect of inhibiting high-fat diet-induced obesity and fatty liver by TM4SF19-Fc treatment

After six-week-old mice fed a high-fat diet for 7 weeks were purchased and acclimated by feeding them a high-fat diet for 1 week, they were administered mouse TM4SF19-Fc twice a week through subcutaneous or intravenous injection while the mice were fed a high-fat diet for 9 weeks. As a result, as can be seen in FIG. 46A, it can be confirmed that obesity caused by a high-fat diet was suppressed by the administration of 10 mg/kg of mTM4SF19-Fc.

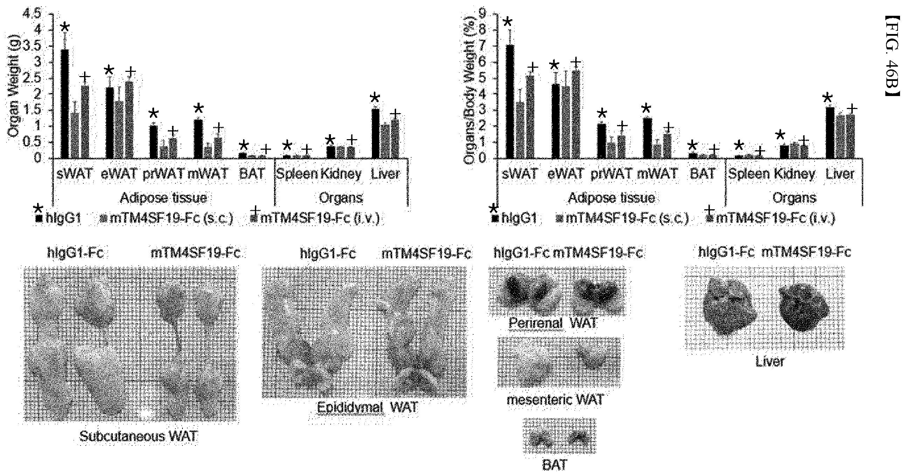

As a result of sacrificing mice and analyzing the distribution of white fat in organs, as can be seen in FIG. 46B, it can be confirmed that the weight of white fat was reduced by TM4SF19-Fc treatment, and an apparent fatty liver inhibitory effect was also confirmed.

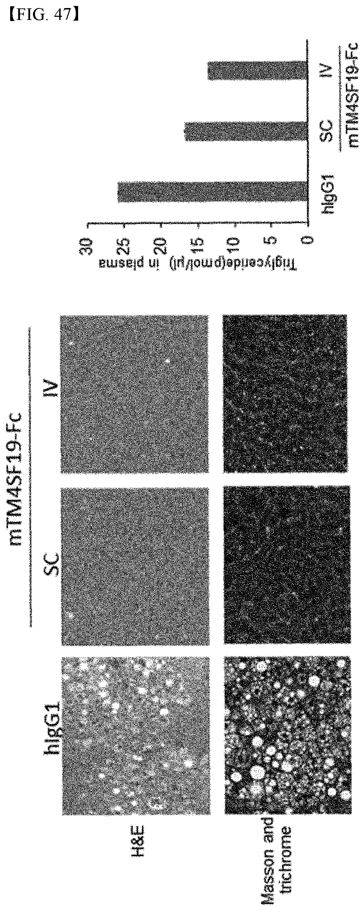

In addition, as a result of observing liver tissues through H&E staining and Masson and trichrome staining, as can be seen from FIG. 47 (left), compared to the hIgG1-Fc-treated control, the effect of decreasing fatty liver was clearly shown. In addition, it was confirmed that triglycerides in plasma decreased (FIG. 47 (right)).

### Example 17: Adipocyte differentiation inhibitory effect by TM4SF19-Fc treatment

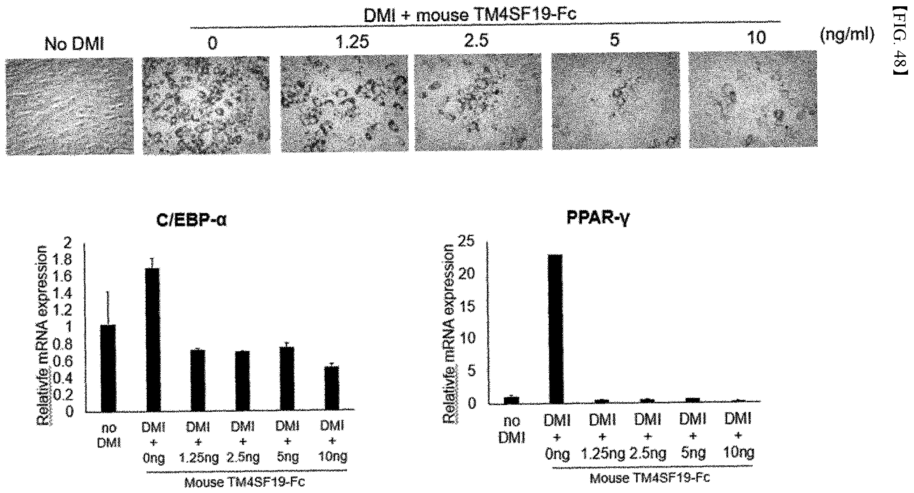

C3H10T1/2 cells were treated with purified mouse TM4SF19-Fc (116-165) and it was confirmed that adipocyte differentiation was dose-dependently inhibited (FIG. 48).

For a control, a differentiation induction factor was not treated, and for an experimental group, mouse TM4SF19 115-165-Fc as well as the differentiation induction factor (treated with dexamethasone (1 µM), IBMX (500 µM), insulin (4 µg/ml, DMI), rosiglitazone (5 µM) in adipocyte differentiation) was treated at 0 ng/ml, 1.25 ng/ml, 2.5 ng/ml, 5 ng/ml, or 10 ng/ml, and adipocyte differentiation was observed.

In addition, as a result of confirming the expression of C/EBP alpha and PPAR-gamma, which are major markers of adipocyte differentiation, it was confirmed that the expression of a DMI-treated group was increased compared to an undifferentiated group (no DMI), and the expression is inhibited by mouse TM4SF19-Fc (116-165) treatment.

### Example 18: Inhibitory effect on cancer cell migration induced by osteoclast differentiation

### (1) TM4SF19-Fc

The inhibition of the cancer cell migration induced by osteoclast differentiation by mTM4SF19-Fc or hTM4SF19-Fc (120-169) was confirmed by staining with a 0.05% crystal violet solution. Bone marrow-derived macrophages were plated on a 12 well and treated with MCSF and RANKL to induce differentiation. At this time, the cells were treated with 10 µg/ml of mTM4SF 19-Fc or hTM4SF19-Fc, or not treated with RANKL to allow no differentiation, and then cancer cells MDA-MB231 or PC3M were put on a migration chamber to allow migration. The migrated cancer cells were stained with crystal violet.

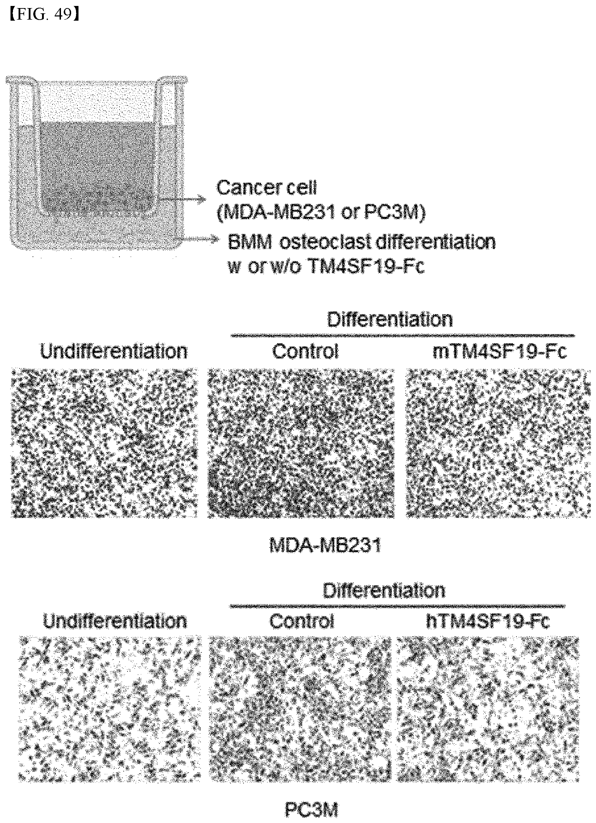

As a result, as shown in FIG. 49, it was confirmed that the cancer cell migration induced by osteoclast differentiation was inhibited by TM4SF19-Fc.

### (2) TM4SF19KO

The inhibition of cancer cell migration by osteoclast differentiation was confirmed in TM4SF19KO, bone marrow-derived macrophages extracted from WT and TM4SF19KO were treated with MCSF and then RANKL to induce differentiation, or not treated to not differentiate, and then cancer cells MDA-MB231 or PC3M were added on a migration chamber to allow migration. The migrated cancer cells were stained with crystal violet.

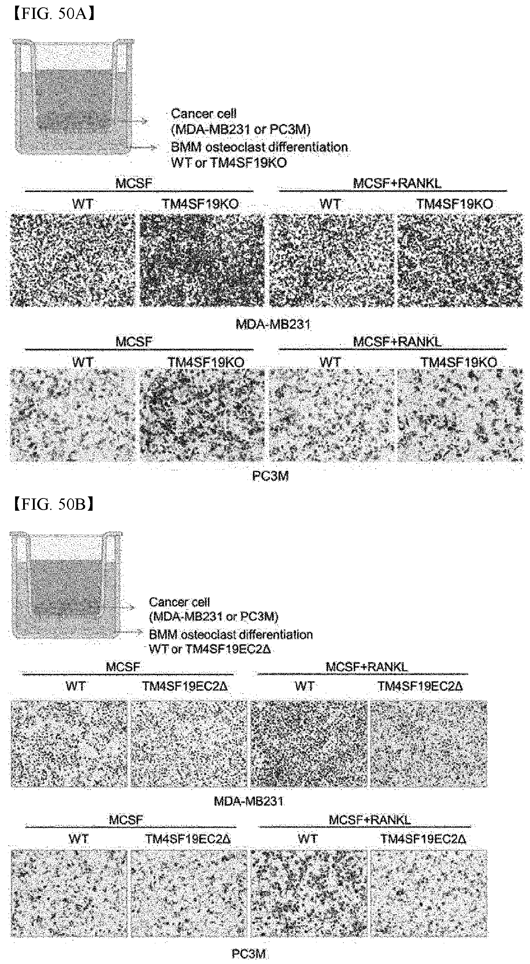

In FIG. 50A, the above result shows the inhibition of cancer cell migration by osteoclast differentiation in TM4SF19KO.

### (3) TM4SF19EC2Δ

To confirm the inhibition of cancer cell migration by osteoclast differentiation in TM4SF19EC2Δ, bone marrow-derived macrophages extracted from WT and TM4SF19EC2Δ were treated with MCSF and then RANKL to induce differentiation, or not treated to not differentiate, and then cancer cells MDA-MB231 or PC3M were put on a migration chamber to allow migration. The migrated cancer cells were stained with crystal violet.

As a result, FIG. 50B shows the inhibition of cancer cell migration by osteoclast differentiation in TM4SF19EC2Δ.

### Example 19: Confirmation of osteosarcoma cell proliferation, migration and colony formation

### (1) TM4SF19 overexpression

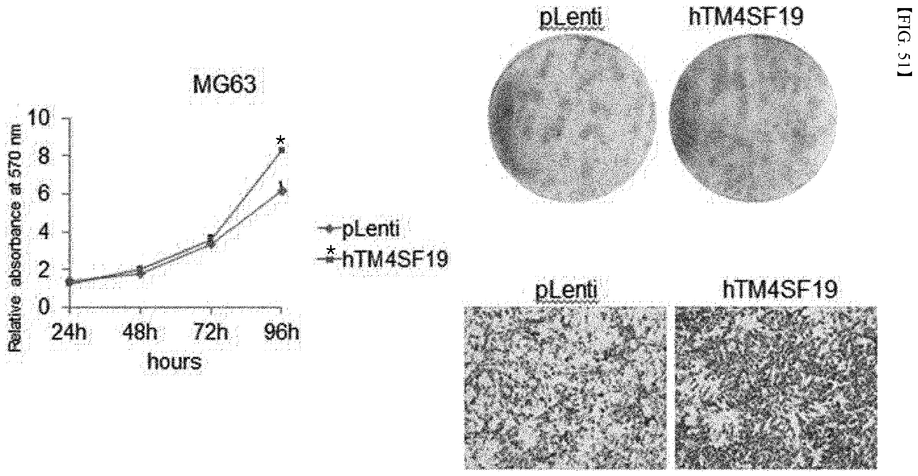

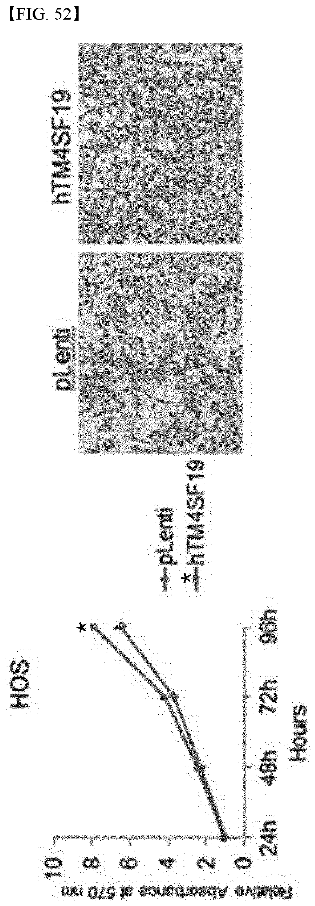

After stably overexpressing 3Flag-hTM4SF19 in MG63 and HOS osteosarcoma cell lines, cell growth was analyzed by MTT, and colony formation and migration were examined. As a result, it was confirmed that osteosarcoma cell proliferation, migration and colony formation were increased by TM4SF19 overexpression (FIG. 51, FIG. 52).

In addition, after knock-out of TM4SF19 in a 143b osteosarcoma cell line through CRISPR, different clones (#4 and #6) were used to examine cell growth through MTT analysis, and examine colony formation.

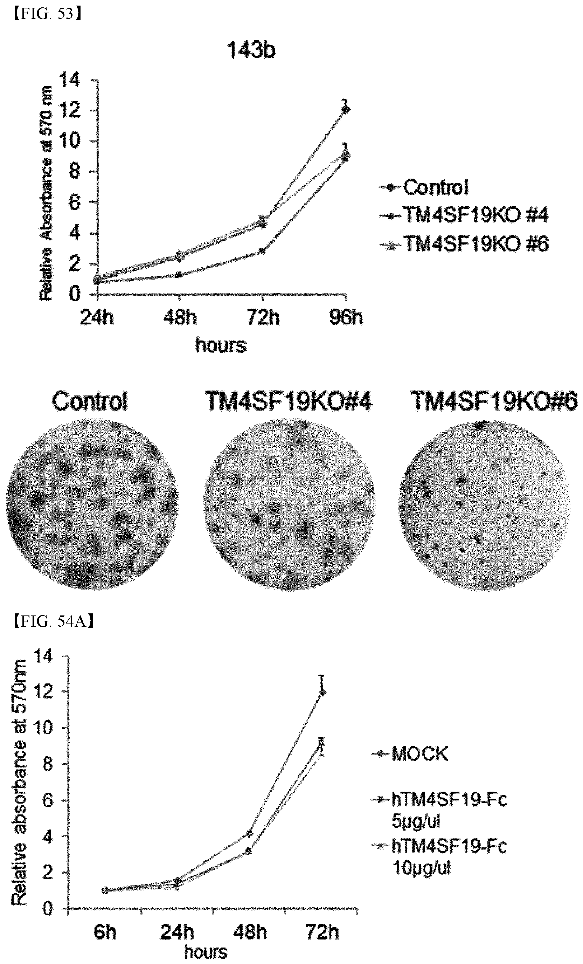

As a result, it was confirmed that the proliferation and colony formation of the osteosarcoma cells (143b) are inhibited by the inhibition of TM4SF19 expression (FIG. 53).

TM4SF19 overexpression increases osteosarcoma cell proliferation and migration. The inhibition of TM4SF19 expression inhibits osteosarcoma cell proliferation and colony formation.

### (2) TM4SF19-Fc treatment

By treating the 143b osteosarcoma cell line with hTM4SF19-Fc (120-169), cell growth was examined by MTT analysis and a cell count, and colonization ability was examined by colony formation, and cell migration was examined.

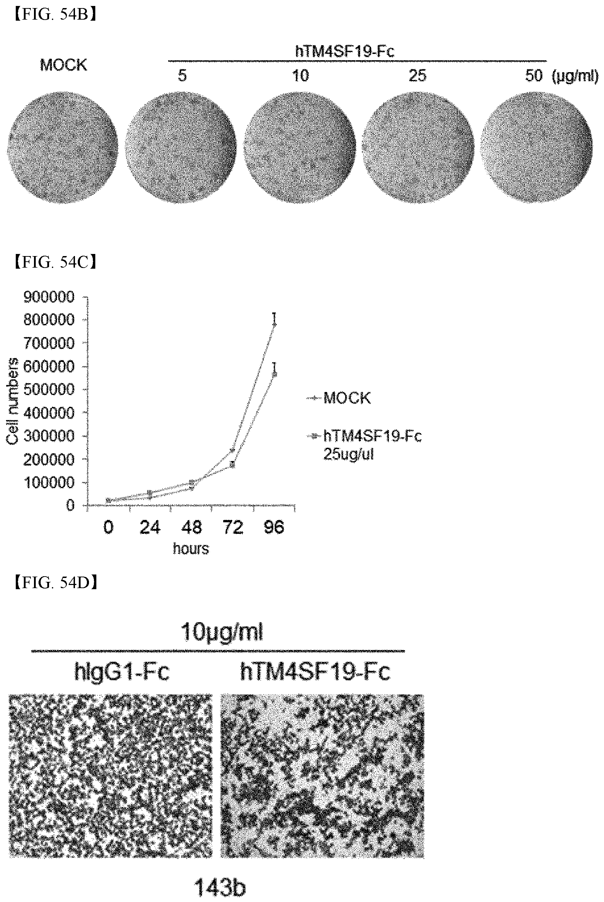

The inhibition of the 143b osteosarcoma cell growth by TM4SF19-Fc treatment was confirmed by MTT analysis (FIG. 54A) and a cell count (FIG. 54C), the colonization ability was confirmed by colony formation (FIG. 54B), and the cell migration was confirmed (FIG. 54D).

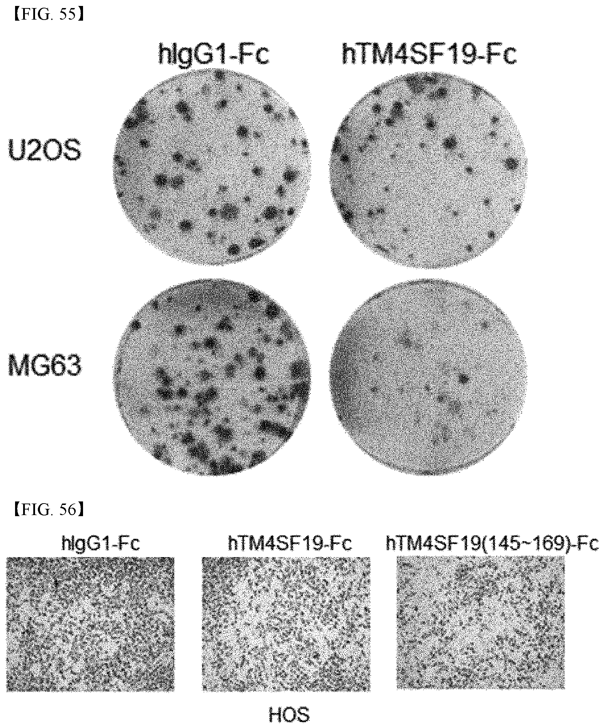

It was confirmed that, by the treatment with human TM4SF19-Fc (aa 120-169) (10 µg/ml), U2OS, MG63 osteosarcoma cell colony formation was inhibited (FIG. 55).

It was confirmed that the cell migration ability of the HOS osteosarcoma cells was inhibited by treatment with 10 µg/ml of hTM4SF19-Fc (aa 120-169) and hTM4SF19-Fc (aa 145-169) (FIG. 56).

It can be determined that hTM4SF19-Fc including at least 145-169 aa has activity.

### Example 20: Confirmation of inhibition of pancreatic cancer cell growth and colony formation

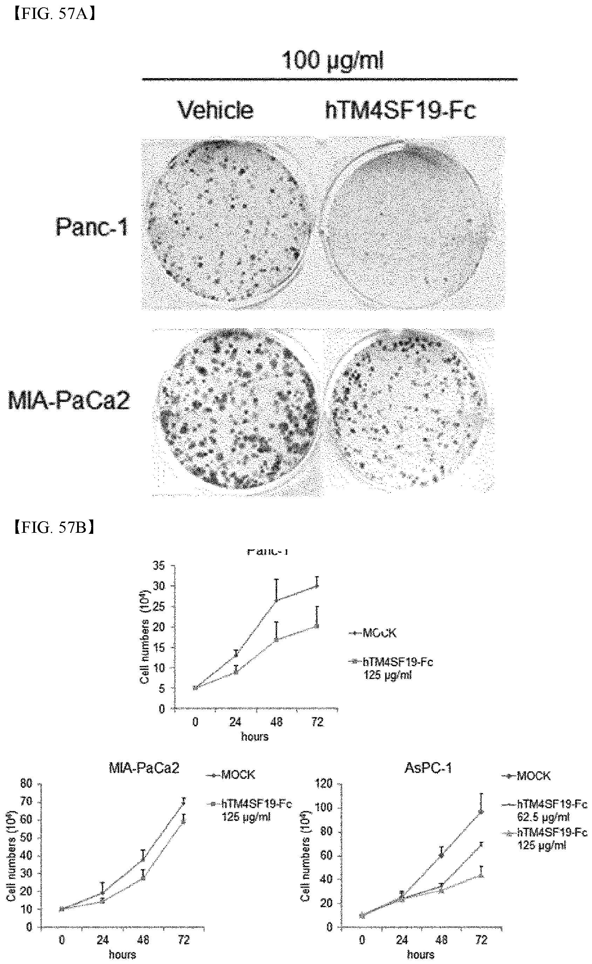

After pancreatic cancer cell lines Panc-1 and MIA-PaCa2 were treated with 100 µg/ml of hTM4SF19-Fc (120-169), the colonization ability of the cells was examined by colony formation assay. As a result, as can be seen from FIG. 57A, it was confirmed that the colonization ability of the pancreatic cancer cell line is inhibited by hTM4SF19-Fc (120-169). In addition, pancreatic cancer cell lines Panc-1, MIA-PaCa2, and AsPC-1 were treated with 62.5 µg/ml or 125 µg/ml of hTM4SF19-Fc (120-169), and the inhibition of the cell growth was examined by a cell count (FIG. 57B). As a result, it was confirmed that the growth and colonization ability of the pancreatic cancer cells were inhibited by TM4SF19-Fc treatment.

## Claims

1. A pharmaceutical composition for use in preventing or treating a bone disease, comprising:
an inhibitor of the expression or activity of transmembrane 4 L six family member 19 (TM4SF19) as an active ingredient.

2. The composition of claim 1, wherein the inhibitor of TM4SF19 expression or activity is one or more selected from the group consisting of an antisense nucleotide, small hairpin RNA (shRNA), small interfering RNA (siRNA), microRNA (miRNA) and a ribozyme, which complementarily bind to mRNA of a TM4SF19 gene.

3. The composition of claim 1, wherein the inhibitor of the expression or activity of TM4SF19 is one or more selected from the group consisting of a compound, a peptide, a peptide mimetic, an aptamer, a fusion protein and an antibody, which specifically bind to a TM4SF19 protein.

4. The composition of claim 3, wherein the fusion protein specifically binding to the TM4SF19 protein comprises a TM4SF19 fragment and the immunoglobulin Fc domain.

5. The composition of claim 4, wherein the TM4SF19 fragment comprises all or a part of the extracellular loop 2 region of TM4SF19.

6. The composition of claim 1, wherein the bone disease is one or more selected from the group consisting of a metabolic bone disease, an orthopedic bone disease, aplastic bone disease, degenerative bone disease, degenerative arthritis, rheumatoid arthritis, psoriatic arthritis, psoriatic spondylitis, age-related bone loss, osteoporosis, osteogenesis imperfecta, osteomalacia, sarcopenia, fractures, bone defect and hip arthrosis, rickets, Paget's bone disease, periodontal disease, and bone damage caused by the bone metastasis of cancer cells.

7. A method of screening a drug for treating a bone disease, comprising:
treating a specimen suspected of a bone disease with a candidate for treating a bone disease; and
comparing the mRNA or protein expression level of a TM4SF19 gene with a control.

8. A pharmaceutical composition for use in preventing or treating obesity or an obesity-mediated metabolic disease, comprising:
an inhibitor of transmembrane 4 L six family member 19 (TM4SF19) expression or activity as an active ingredient.

9. The composition of claim 8, wherein the inhibitor of TM4SF19 expression or activity is one or more selected from the group consisting of an antisense nucleotide, small hairpin RNA (shRNA), small interfering RNA (siRNA), microRNA (miRNA) and a ribozyme, which complementarily bind to mRNA of a TM4SF19 gene.

10. The composition of claim 8, wherein the inhibitor of the expression or activity of TM4SF19 is one or more selected from the group consisting of a compound, a peptide, a peptide mimetic, an aptamer, a fusion protein and an antibody, which specifically bind to a TM4SF19 protein.

11. The composition of claim 10, wherein the fusion protein specifically binding to the TM4SF19 protein comprises a TM4SF19 fragment and the immunoglobulin Fc domain.

12. The composition of claim 11, wherein the TM4SF19 fragment comprises all or a part of the extracellular loop 2 region of TM4SF19.

13. The composition of claim 8, wherein the obesity-mediated metabolic disease is one or more selected from the group consisting of diabetes, high blood pressure, hyperlipidemia, non-alcoholic steatohepatitis and specific cancers, and more broadly, includes high blood pressure, diabetes, insulin-resistant syndrome, metabolic syndrome, obesity-related gastroesophageal reflux disease, arteriosclerosis, hyperlipidemia, hypertriglyceridemia, hypercholesterolemia, lipodystrophy, non-alcoholic steatohepatitis, cardiovascular disease, and polycystic ovary syndrome.

14. A method of screening a drug for treating obesity or an obesity-mediated metabolic disease, comprising:
treating a specimen suspected of obesity or an obesity-mediated metabolic disease with a candidate material for treating obesity or an obesity-mediated metabolic disease; and
comparing the mRNA or protein expression level of a TM4SF19 gene with a control.

15. A pharmaceutical composition for use in preventing or treating cancer, or inhibiting cancer metastasis, comprising an inhibitor of transmembrane 4 L six family member 19 (TM4SF19) expression or activity as an active ingredient.

16. The composition of claim 15, wherein the inhibitor of TM4SF19 expression or activity is one or more selected from the group consisting of an antisense nucleotide, small hairpin RNA (shRNA), small interfering RNA (siRNA), microRNA (miRNA) and a ribozyme, which complementarily bind to mRNA of a TM4SF19 gene.

17. The composition of claim 15, wherein the inhibitor of the expression or activity of TM4SF19 is one or more selected from the group consisting of a compound, a peptide, a peptide mimetic, an aptamer, a fusion protein and an antibody, which specifically bind to a TM4SF19 protein.

18. The composition of claim 17, wherein the fusion protein specifically binding to the TM4SF19 protein comprises a TM4SF19 fragment and the immunoglobulin Fc domain.

19. The composition of claim 18, wherein the TM4SF19 fragment comprises all or a part of the extracellular loop 2 region of TM4SF19.

20. The composition of claim 15, wherein the cancer is one or more selected from the group consisting of colorectal cancer, stomach cancer, colon cancer, breast cancer, lung cancer, non-small cell lung cancer, bone cancer, pancreatic cancer, skin cancer, head or neck cancer, melanoma, uterine cancer, ovarian cancer, small intestine cancer, rectal cancer, perianal cancer, fallopian tube carcinoma, endometrial carcinoma, cervical carcinoma, vaginal carcinoma, vulvar carcinoma, esophageal cancer, lymphatic adenocarcinoma, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumors, spinal cord tumors, brainstem glioma, and pituitary adenomas.

21. A method of screening a drug for treating cancer or cancer metastasis, comprising:
treating a specimen suspected of cancer or cancer metastasis with a candidate for preventing or treating cancer or inhibiting cancer metastasis; and
comparing the mRNA or protein expression level of a TM4SF19 gene with a control.

22. A fusion protein for inhibiting transmembrane 4 L six family member 19 (TM4SF19) expression or activity, comprising an extracellular loop 2-derived fragment of a TM4SF19 protein and the immunoglobulin Fc domain.

23. The fusion protein of claim 22, wherein the extracellular loop 2 (EC2)-derived fragment of the TM4SF19 protein corresponds all or a part of EC2.

24. The fusion protein of claim 22, wherein the amino acid sequence region corresponding to all of EC2 is the region of amino acids 120-169 of a human TM4SF19 protein or the region of amino acids 116-165 of a mouse TM4SF19 protein.

25. The fusion protein of claim 22, wherein the amino acid sequence region corresponding to a part of EC2 is the region of amino acids 145-169 of a human TM4SF19 protein.

26. The fusion protein of claim 22, which comprises an amino acid sequence represented by SEQ ID NO: 10, SEQ ID NO: 13, SEQ ID NO: 16, or SEQ ID NO: 19.

27. A method of preventing or treating a bone disease, comprising:
administering a therapeutically effective amount of composition for preventing or treating a bone disease including an inhibitor of transmembrane 4 L six family member 19 (TM4SF19) expression or activity into a subject.

28. A method of preventing or treating obesity or an obesity-mediated metabolic disease, comprising:
administering a therapeutically effective amount of composition for preventing or treating obesity or an obesity-mediated metabolic disease including an inhibitor of transmembrane 4 L six family member 19 (TM4SF19) expression or activity into a subject.

29. A method of preventing or treating cancer or cancer metastasis, comprising:
administering a therapeutically effective amount of composition for preventing or treating cancer or inhibiting cancer metastasis including an inhibitor of transmembrane 4 L six family member 19 (TM4SF19) expression or activity into a subj ect.
